# EUROPEAN PATENT SPECIFICATION

(11) **EP 3 083 679 B1**
(45) Date of publication and mention of the grant of the patent: **30.09.2020**
(21) Application number: 14835554.8
(22) Date of filing: 19.12.2014
(51) Int. Cl.: C07K 16/12, C07K 14/315

(54) **ANTIBODIES DIRECTED AGAINST THE LUKGH (LUKAB) TOXIN OF STAPHYLOCOCCUS AUREUS AND ANTIBODY SEQUENCES**
GEGEN DAS LUKGH (LUKAB)-TOXIN VON STAPHYLOCOCCUS AUREUS GERICHTETE ANTIKÖRPER UND ANTIKÖRPERSEQUENZEN
ANTICORPS DIRIGÉS CONTRE LA TOXINE LUKGH (LUKAB) DU STAPHYLOCOQUE DORÉ ET SÉQUENCES D'ANTICORPS

(30) Priority: 19.12.2013 EP 13198484
(43) Date of publication of application: 26.10.2016
(73) Proprietor: X4 Pharmaceuticals (Austria) GmbH, 1030 Vienna (AT)
(72) Inventor: NAGY, Eszter, A-1070 Vienna (AT); BADARAU, Adriana, A-1030 Vienna (AT); ROUHA, Harald, A-1220 Wien (AT); MIRKINA, Irina, A-1020 Vienna (AT); BATTLES, Michael Benjamin, Lebanon, New Hampshire 03766 (US); WALKER, Laura M, Lebanon, New Hampshire 03766 (US); NIELSON, Nels, Lebanon, New Hampshire 03766 (US); JAIN, Tushar S., Lebanon, New Hampshire 03766 (US)
(74) Representative: Redl, Gerda
(86) International application number: PCT/EP2014/078708
(87) International publication number: WO 2015/091935

(56) References cited:
- WO-A2-2014/187746
- US-A1- 2011 274 693
- N. MALACHOWA ET AL: "Staphylococcus aureus Leukotoxin GH Promotes Formation of Neutrophil Extracellular Traps", THE JOURNAL OF IMMUNOLOGY, vol. 191, no. 12, 4 November 2013 (2013-11-04), pages 6022-6029, XP055178705, ISSN: 0022-1767, DOI: 10.4049/jimmunol.1301821
- GORDON YC CHEUNG ET AL: "The potential use of toxin antibodies as a strategy for controlling acute Staphylococcus aureus infections", EXPERT OPINION ON THERAPEUTIC TARGETS, vol. 16, no. 6, 1 June 2012 (2012-06-01), pages 601-612, XP055178709, ISSN: 1472-8222, DOI: 10.1517/14728222.2012.682573
- RUDIKOFF S ET AL: "Single amino acid substitution altering antigen-binding specificity", PROCEEDINGS OF THE NATIONAL ACADEMY OF SCIENCES, NATIONAL ACADEMY OF SCIENCES, US, vol. 79, 1 March 1982 (1982-03-01), pages 1979-1983, XP007901436, ISSN: 0027-8424, DOI: 10.1073/PNAS.79.6.1979
- ADRIANA BADARAU ET AL: "Context matters: The importance of dimerization-induced conformation of the LukGH leukocidin of Staphylococcus aureus for the generation of neutralizing antibodies", MABS, vol. 8, no. 7, 28 July 2016 (2016-07-28), pages 1347-1360, XP055358253, US ISSN: 1942-0862, DOI: 10.1080/19420862.2016.1215791

## Description

The invention relates to antibodies directed against the *Staphylococcus aureus* cytotoxin LukGH (also called LukAB) which are characterized by specific amino acid sequences.

### BACKGROUND OF THE INVENTION

*Staphylococcus aureus* (*S. aureus*) is one of most important human pathogens that can cause a broad spectrum of infections in humans ranging from asymptomatic colonization and mild skin infections to severe deep tissue infections, pneumonia, blood stream infections and sepsis. This pathogen uses multiple virulence mechanisms to cause disease and interfere with host defense. One of its most potent virulence factors are the leukocidins specialized in killing white blood cells, especially phagocytic cells. LukGH (also called LukAB) is the most recently identified leukocidin that is able to lyse polymorphonuclear cells (PMNs), monocytes and dendritic cells (Dumont et al. Mol Microbiol. 2011 Feb;79(3):814-25; Ventura et al. PLoS One. 2010 Jul 16;5(7):e11634) and also to activate them to produce pro-inflammatory cytokines.

LukGH is a bi-component cytolysin, similarly to HlgAB, HlgCB, LukED and LukSF (PVL). LukH (S-component) and LukG (F-component) display approximately 30 and 40% amino acid homology with the S and F components of the above mentioned bi-component leukocidins, respectively.

LukGH is the most variable bi-component *S. aureus* toxin. While LukSF, LukED and HlgABC are highly conserved in different *S. aureus* strains, LukGH exhibits up to 14% amino acid changes. This level of amino acid differences almost reaches the one observed between two different toxins, eg HlgC vs LukS or LukS vs HlgAC or LukE (-16% difference). There are very few data about the function of LukGH and those are generated with two sequences from the Newman and LAC (USA300) strains that are almost identical to each other. It is not known whether the other variants are active with human cells or not, especially two sequences derived from the two *S. aureus* genomes, MRSA252 (EMRSA16) and MSHR1132 ("silver" *S*. *aureus*) that are considered to be the most different from USA300 and other *S. aureus* clonal complexes.

Based on the PMN lysis activity of *S. aureus* cultures supernatants, LukGH seems to be one of the most potent leukotoxin for human innate cells (Dumont et al. Infect Immun. 2013 May;81(5):1830-41; Malachowa et al. J Infect Dis. 2012 Oct;206(8):1185-93). Therefore, it is plausible that inhibiting LukGH's toxin function by neutralizing antibodies has a positive effect during *S. aureus* disease and supports host defense by saving the phagocytic cells migrating to the site of infections. It is the aim to develop human therapeutics to prevent and treat human *S. aureus* infections with monoclonal antibodies neutralizing the LukGH toxins. US2011/274693 discloses isolated and purified S. aureus bi-component leukotoxin LukGH (LukAB) and its components LukH (LukA) and LukG (LukB), as well as antibodies specific to LukH, and antibodies specific to LukG.

Based on literature, it is the S component of bi-component leukocidins that recognizes a cell surface receptor, and this interaction induces a conformational change leading to the binding of the F-component and formation of the octameric membrane spanning pore structure described for LukSF and HlgAB (Colin, Infect Immun, 1994:3184; Meunier, Biochim Biophys Acta, 1997:275).

### SUMMARY OF THE INVENTION

It is the objective of the present invention to provide for an antibody directed against the *S. aureus* cytotoxin LukGH (also called LukAB) that is cross-reactive among the different variants of this toxin and provide cross neutralizing potency. Specifically, the objective refers to an antibody with high neutralizing potency against LukGH *in vitro* and improved protection compared to antibodies that are specifically binding the individual LukG or LukH antigens, but binding the LukGH heterodimer to a less extent.

The object is solved by the subject of the present invention.

According to the invention there is provided an antibody comprising at least one binding site that specifically binds to a LukGH heterodimer, which antibody comprises at least an antibody heavy chain variable region (VH), which comprises CDR1 to CDR3 sequences of said binding site, and an antibody light chain variable region (VL), which comprises CDR4 to CDR6 sequences of said binding site, which is selected from the group consisting of
a) an antibody comprising
   a. the HC amino acid sequence of SEQ ID 161; and
   b. the LC amino acid sequence of SEQ ID 162;
b) an antibody comprising
   a. the HC amino acid sequence of SEQ ID 147; and
   b. the LC amino acid sequence of SEQ ID 148;
c) an antibody comprising
   a. the HC amino acid sequence of SEQ ID 149; and
   b. the LC amino acid sequence of SEQ ID 150;
d) an antibody comprising
   a. the HC amino acid sequence of SEQ ID 151; and
   b. the LC amino acid sequence of SEQ ID 152;
e) an antibody comprising
   a. the HC amino acid sequence of SEQ ID 153; and
   b. the LC amino acid sequence of SEQ ID 154;
f) an antibody comprising
   a. the HC amino acid sequence of SEQ ID 155; and
   b. the LC amino acid sequence of SEQ ID 156;
g) an antibody comprising
   a. the HC amino acid sequence of SEQ ID 157; and
   b. the LC amino acid sequence of SEQ ID 158;
h) an antibody comprising
   a. the HC amino acid sequence of SEQ ID 159; and
   b. the LC amino acid sequence of SEQ ID 160;
i) an antibody comprising
   a. the HC amino acid sequence of SEQ ID 163; and
   b. the LC amino acid sequence of SEQ ID 164;
j) an antibody comprising
   a. the HC amino acid sequence of SEQ ID 165; and
   b. the LC amino acid sequence of SEQ ID 166;
k) an antibody comprising
   a. the HC amino acid sequence of SEQ ID 167; and
   b. the LC amino acid sequence of SEQ ID 168;
l) an antibody comprising
   a. the HC amino acid sequence of SEQ ID 169; and
   b. the LC amino acid sequence of SEQ ID 170;
   and
m) an antibody comprising
   a. the HC amino acid sequence of SEQ ID 171; and
      the LC amino acid sequence of SEQ ID 172.

Specifically, the antibody described herein is a full-length monoclonal antibody, an antibody fragment thereof comprising the binding site, or a fusion protein comprising the binding site.

Further provided herein is an antibody comprising at least one binding site that specifically binds to a LukGH complex, which antibody comprises at least an antibody heavy chain variable region (VH), which comprises any of the CDR1 to CDR3 sequences as listed in Table 1, or functionally active CDR variants thereof.

Specifically, the antibody is a monoclonal antibody.

Specifically, the antibody is selected from the group consisting of group members i) to viii), wherein
i)
   A) is an antibody which comprises
      a) a CDR1 comprising or consisting of the amino acid sequence of SEQ ID 2 or SEQ ID 15; and
      b) a CDR2 comprising or consisting of the amino acid sequence of SEQ ID 4; and
      c) a CDR3 comprising or consisting of the amino acid sequence of SEQ ID 6;
      or
   B) is an antibody which is a functionally active variant of the antibody A, which comprises at least one functionally active CDR variant of
      a) the parent CDR1 consisting of the amino acid sequence of SEQ ID 2 or SEQ ID 15; and/or
      b) the parent CDR2 consisting of the amino acid sequence of SEQ ID 4; and/or
      c) the parent CDR3 consisting of the amino acid sequence of SEQ ID 6;
ii)
   A) is an antibody which comprises
      a) a CDR1 comprising or consisting of any of the amino acid sequences of SEQ ID 26, SEQ ID 36, or SEQ ID 38; and
      b) a CDR2 comprising or consisting of any of the amino acid sequences of SEQ ID 28, SEQ ID 37, SEQ ID 39, or SEQ ID 40; and
      c) a CDR3 comprising or consisting of the amino acid sequence SEQ ID 30;
      or
   B) is an antibody which is a functionally active variant of the antibody A, which comprises at least one functionally active CDR variant of
      a) the parent CDR1 consisting of any of the amino acid sequence of SEQ ID 26, SEQ ID 36, or SEQ ID 38; and/or
      b) the parent CDR2 consisting of any of the amino acid sequences of SEQ ID 28, SEQ ID 37, SEQ ID 39, or SEQ ID 40; and/or
      c) the parent CDR3 consisting of the amino acid sequence of SEQ ID 30;
iii)
   A) is an antibody which comprises
      a) a CDR1 comprising or consisting any of the amino acid sequences of SEQ ID 47, SEQ ID 55, SEQ ID 57, SEQ ID 59, SEQ ID 61, SEQ ID 63, or SEQ ID 64; and
      b) a CDR2 comprising or consisting of any of the amino acid sequences of SEQ ID 49, SEQ ID 56, SEQ ID 58, SEQ ID 60, SEQ ID 62, SEQ ID 65, or SEQ ID 66; and
      c) a CDR3 comprising or consisting of the amino acid sequence of SEQ ID 51;
      or
   B) is an antibody which is a functionally active variant of the antibody A, which comprises at least one functionally active CDR variant of
      a) the parent CDR1 consisting of any of the amino acid sequences of SEQ ID 47, SEQ ID 55, SEQ ID 57, SEQ ID 59, SEQ ID 61, SEQ ID 63, or SEQ ID 64; and/or
      b) the parent CDR2 consisting of any of the amino acid sequences SEQ ID 49, SEQ ID 56, SEQ ID 58, SEQ ID 60, SEQ ID 62, SEQ ID 65, or SEQ ID 66; and/or
      c) the parent CDR3 consisting of the amino acid sequence of SEQ ID 51;
iv)
   A) is an antibody which comprises
      a) a CDR1 comprising or consisting of any of the amino acid sequences of SEQ ID 71, SEQ ID 77, SEQ ID 79, SEQ ID 81, SEQ ID 83, or SEQ ID 85; and
      b) a CDR2 comprising or consisting of any of the amino acid sequences of SEQ ID 72, SEQ ID 78, SEQ ID 84, or SEQ ID 4; and
      c) a CDR3 comprising or consisting of the amino acid sequence of SEQ ID 73;
      or
   B) is an antibody which is a functionally active variant of the antibody A, which comprises at least one functionally active CDR variant of
      a) the parent CDR1 consisting of any of the amino acid sequences of SEQ ID 71, SEQ ID 77, SEQ ID 79, SEQ ID 81, SEQ ID 83, or SEQ ID 85; and/or
      b) the parent CDR2 consisting of any of the amino acid sequences of SEQ ID 72, SEQ ID 78, SEQ ID 84, or SEQ ID 4; and/or
      c) the parent CDR3 consisting of the amino acid sequence of SEQ ID 73;
v)
   A) is an antibody which comprises
      a) a CDR1 comprising or consisting of any of the amino acid sequences of SEQ ID 87, SEQ ID 97, SEQ ID 99, or SEQ ID 101; and
      b) a CDR2 comprising or consisting of any of the amino acid sequences of SEQ ID 88, SEQ ID 98, SEQ ID 100, or SEQ ID 102; and
      c) a CDR3 comprising or consisting of the amino acid sequence of SEQ ID 89;
      or
   B) is an antibody which is a functionally active variant of the antibody A, which comprises at least one functionally active CDR variant of
      a) the parent CDR1 consisting of any of the amino acid sequences of SEQ ID 87, SEQ ID 97, SEQ ID 99, or SEQ ID 101; and/or
      b) the parent CDR2 consisting of any of the amino acid sequences of SEQ ID 88, SEQ ID 98, SEQ ID 100, or SEQ ID 102; and/or
      c) the parent CDR3 consisting of the amino acid sequence of SEQ ID 89;
vi)
   A) is an antibody which comprises
      a) a CDR1 comprising or consisting of any of the amino acid sequences of SEQ ID 104, SEQ ID 110, SEQ ID 112, SEQ ID 38, SEQ ID 114, SEQ ID 119, or SEQ ID 120; and
      b) a CDR2 comprising or consisting of any of the amino acid sequences of SEQ ID 105, SEQ ID 109, SEQ ID 111, SEQ ID 113, SEQ ID 102, SEQ ID 115, or SEQ ID 121; and
      c) a CDR3 comprising or consisting of the amino acid sequence of SEQ ID 106;
      or
   B) is an antibody which is a functionally active variant of the antibody A, which comprises at least one functionally active CDR variant of
      a) the parent CDR1 consisting of the amino acid sequence of SEQ ID 104, SEQ ID 110, SEQ ID 112, SEQ ID 38, SEQ ID 114, SEQ ID 119, or SEQ ID 120; and/or
      b) the parent CDR2 consisting of the amino acid sequence of SEQ ID 105, SEQ ID 109, SEQ ID 111, SEQ ID 113, SEQ ID 102, SEQ ID 115, or SEQ ID 121; and/or
      c) the parent CDR3 consisting of the amino acid sequence of SEQ ID 106;
vii)
   A) is an antibody which comprises
      a) a CDR1 comprising or consisting of the amino acid sequence of SEQ ID 125; and
      b) a CDR2 comprising or consisting of the amino acid sequence of SEQ ID 126; and
      c) a CDR3 comprising or consisting of the amino acid sequence of SEQ ID 127;
      or
   B) is an antibody which is a functionally active variant of the antibody A, which comprises at least one functionally active CDR variant of
      a) the parent CDR1 consisting of the amino acid sequence of SEQ ID 125; and/or
      b) the parent CDR2 consisting of the amino acid sequence of SEQ ID 126; and/or
      c) the parent CDR3 consisting of the amino acid sequence of SEQ ID 127;
and viii)
   A) is an antibody which comprises
      a) a CDR1 comprising or consisting of the amino acid sequence of SEQ ID 134; and
      b) a CDR2 comprising or consisting of the amino acid sequence of SEQ ID 135; and
      c) a CDR3 comprising or consisting of the amino acid sequence of SEQ ID 137;
      or
   B) is an antibody which is a functionally active variant of the antibody A, which comprises at least one functionally active CDR variant of
      a) the parent CDR1 consisting of the amino acid sequence of SEQ ID 134; and/or
      b) the parent CDR2 consisting of the amino acid sequence of SEQ ID 135; and/or
      c) the parent CDR3 consisting of the amino acid sequence of SEQ ID 137;
wherein any of the functionally active CDR variants (in particular as specified in one of the group members i) to viii) above) comprises at least one point mutation in the parent CDR sequence, and comprises or consists of the amino acid sequence that has at least 60% sequence identity with the parent CDR sequence, preferably at least 70%, at least 80%, at least 90% sequence identity.

Specifically, the antibody is an antibody of group member iv) (see above) or a functionally active variant thereof, wherein
a) in VH CDR1 at position 7, the amino acid residue is selected from S, A, D, E, F, G, H, I, K, L, M, N, Q, R, T, V, W, and Y, preferentially any of E, F, H, I, K, L, M, R, V, W or Y, and more preferentially is any of E, F, M, W or Y; and/or
b) in VH CDR2 at position 1, the amino acid residue is selected from N, A, D, E, F, H, L, S, T, V and Y, preferentially any of F, H or Y; and/or
c) in VH CDR2 at position 3, the amino acid residue is selected from Y, H, T and W; and/or
d) in VH CDR2 at position 5, the amino acid residue is selected from S, A, E, F, H, I, K, L, M, N, Q, R, T, V, W and Y, preferentially any of N, R or W, and more preferentially is N or W; and/or
e) in VH CDR2 at position 7, the amino acid residue is selected from S, D, F, H, K, L, M, N, R and W; and/or
f) in VH CDR2 at position 9, the amino acid residue is selected from Y, D, E, F, N, S and W, preferentially D or H, and more preferentially is H; and/or
g) in VH CDR3 at position 4, the amino acid residue is selected from R, A, D, E, F, G, H, I, K, L, M, N, Q, S, T, V and W, preferentially D or H; and/or
h) in VH CDR3 at position 5, the amino acid residue is selected from G, A, F and Y; and/or
i) in VH CDR3 at position 6, the amino acid residue is selected from M, E, F, H and Q, preferentially F or H; and/or
j) in VH CDR3 at position 7, the amino acid residue is selected from H, A, D, E, F, G, I, K, L, M, N, Q, R, S, T, W and Y, preferentially any of E, K, Q, R, W or Y, and more preferentially is W or Y.

Specifically, the functionally active CDR variant comprises at least one of
a) 1, 2, or 3 point mutations in the parent CDR sequence; or
b) 1 or 2 point mutations in any of the four C-terminal or four N-terminal, or four centric amino acid positions of the parent CDR sequence.

Specifically, the functionally active variant antibody comprises at least one of the functionally active CDR variants of the invention.

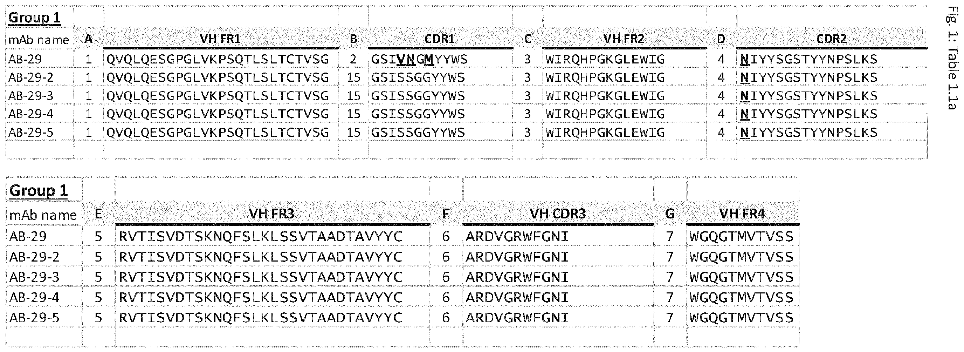

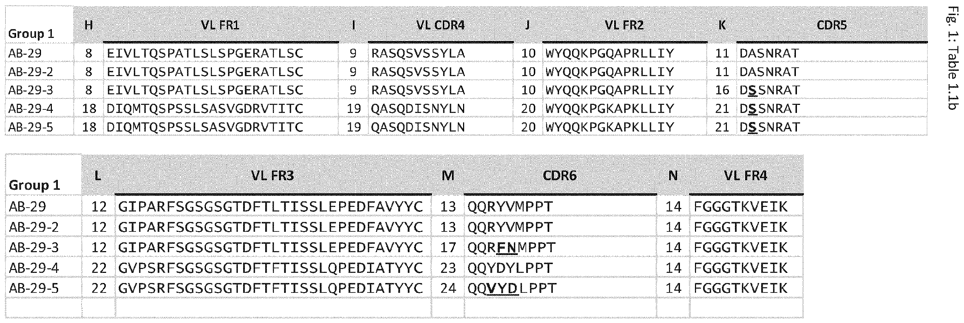

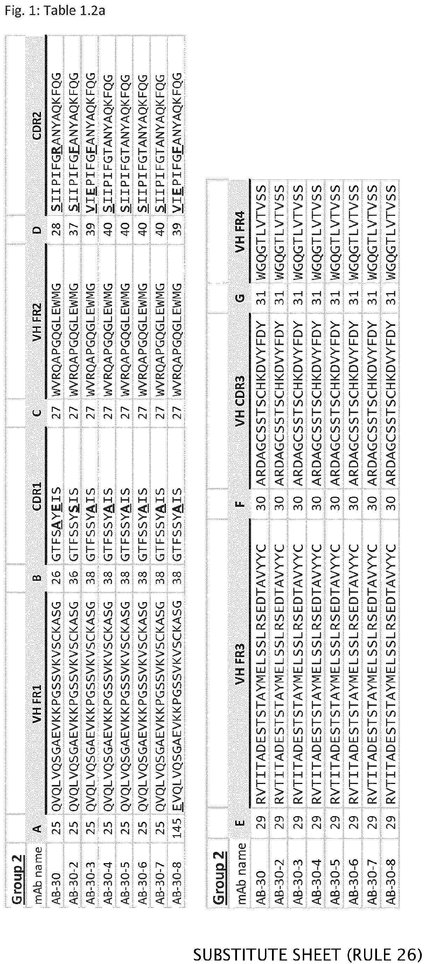

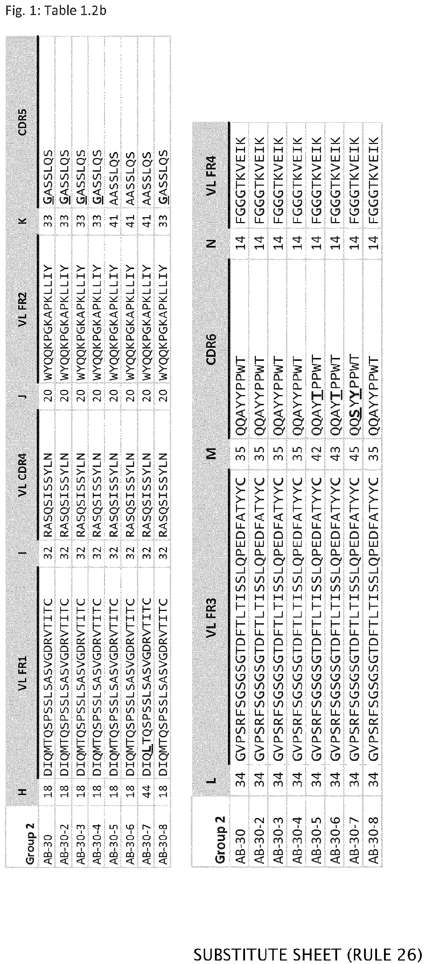

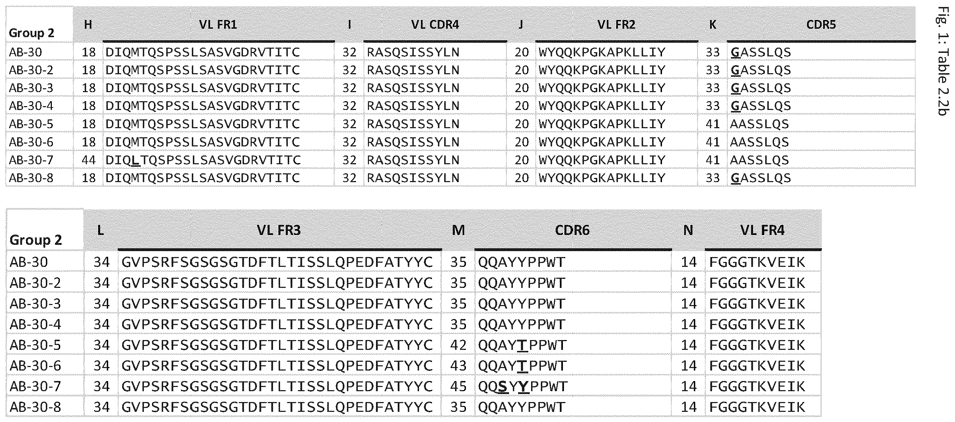

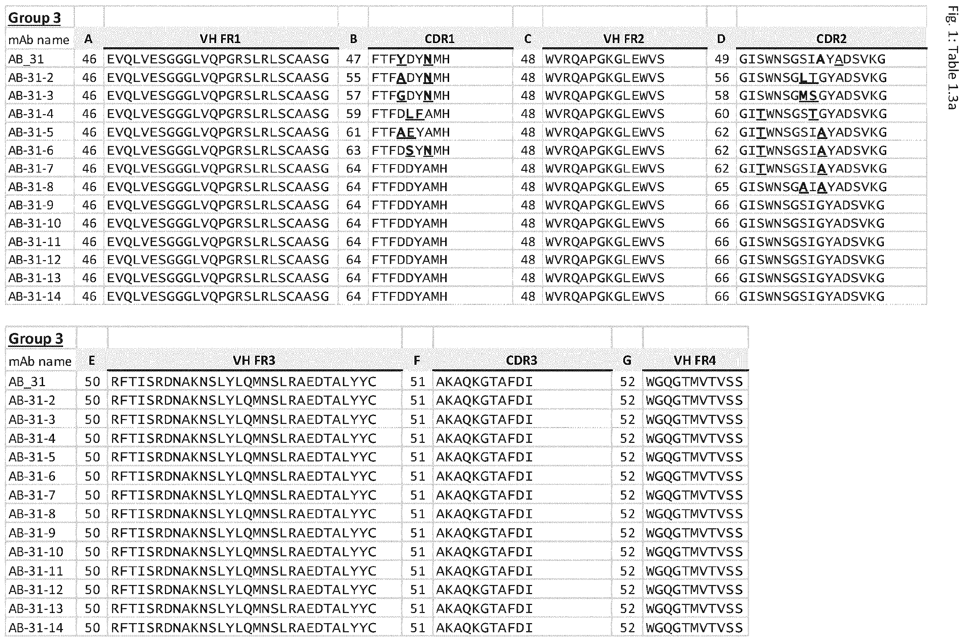

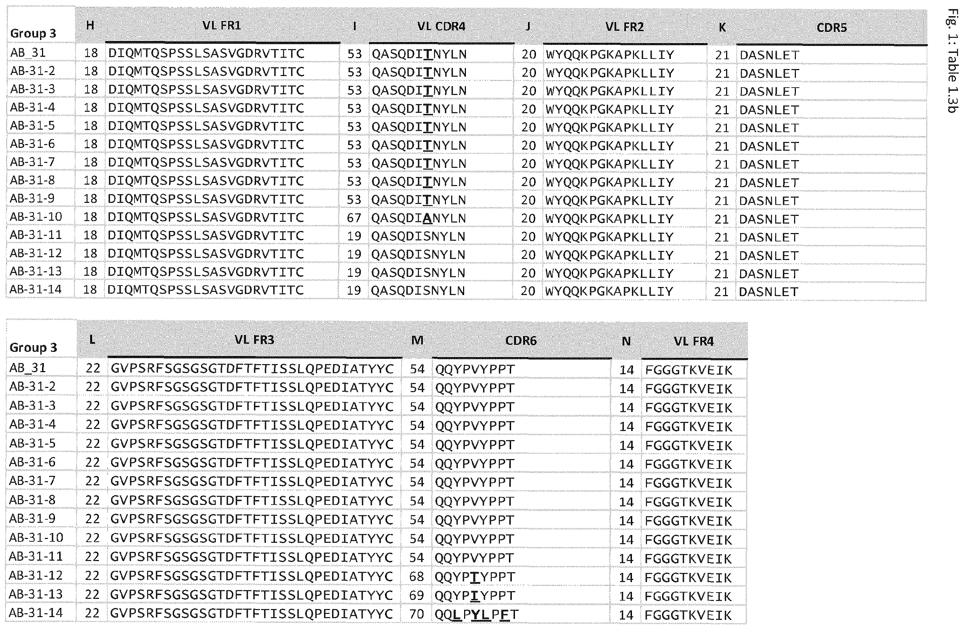

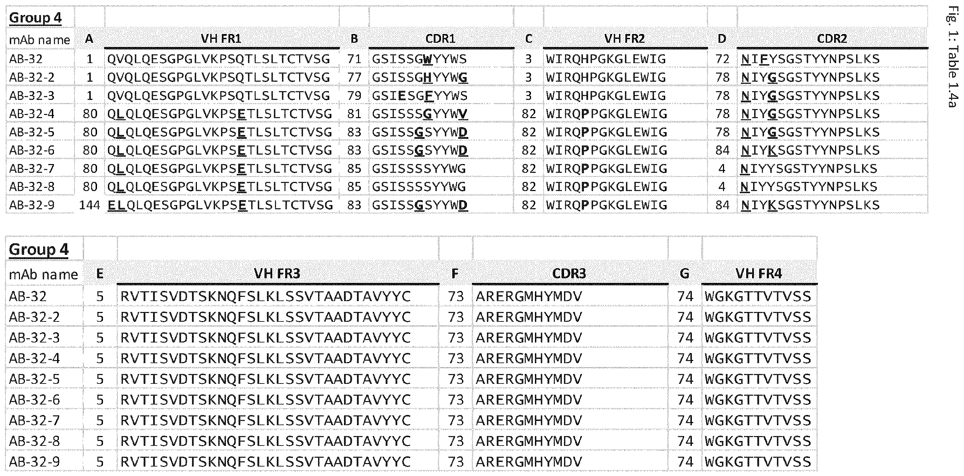

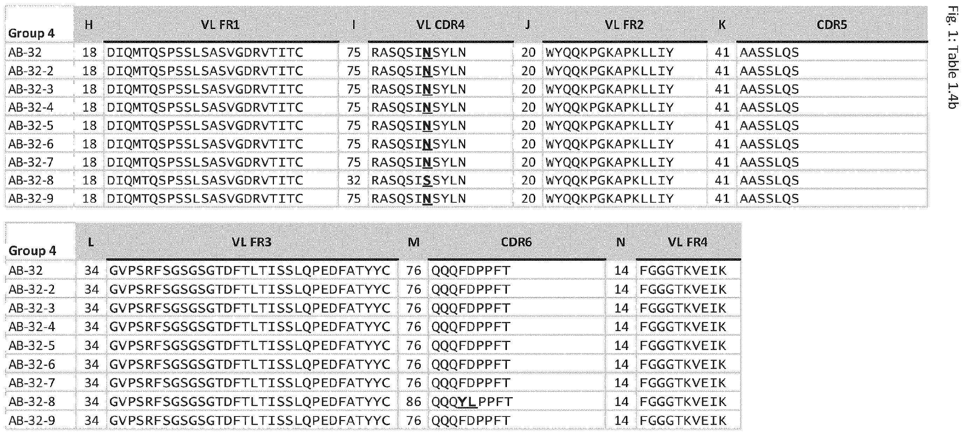

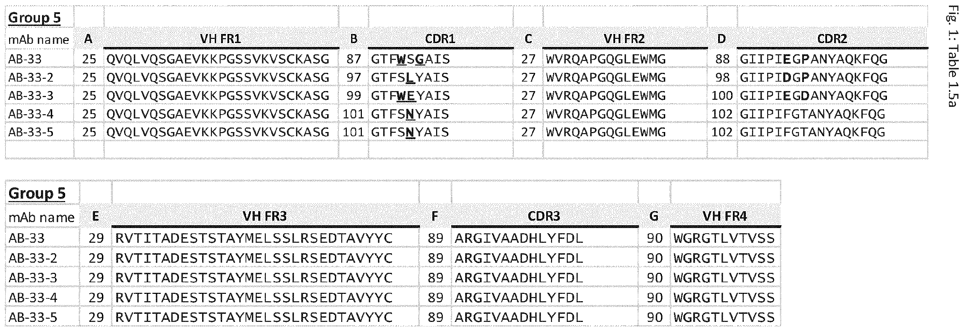

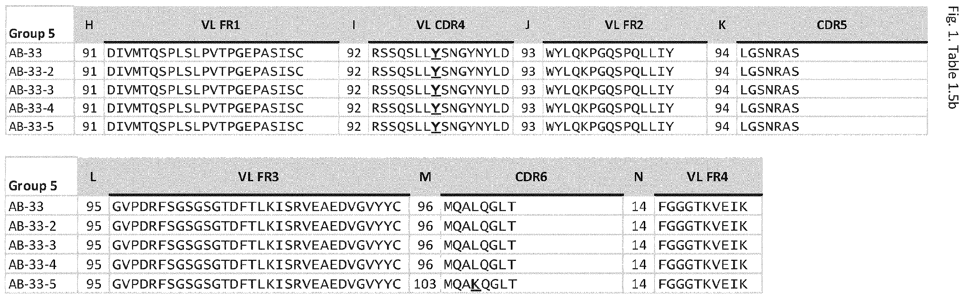

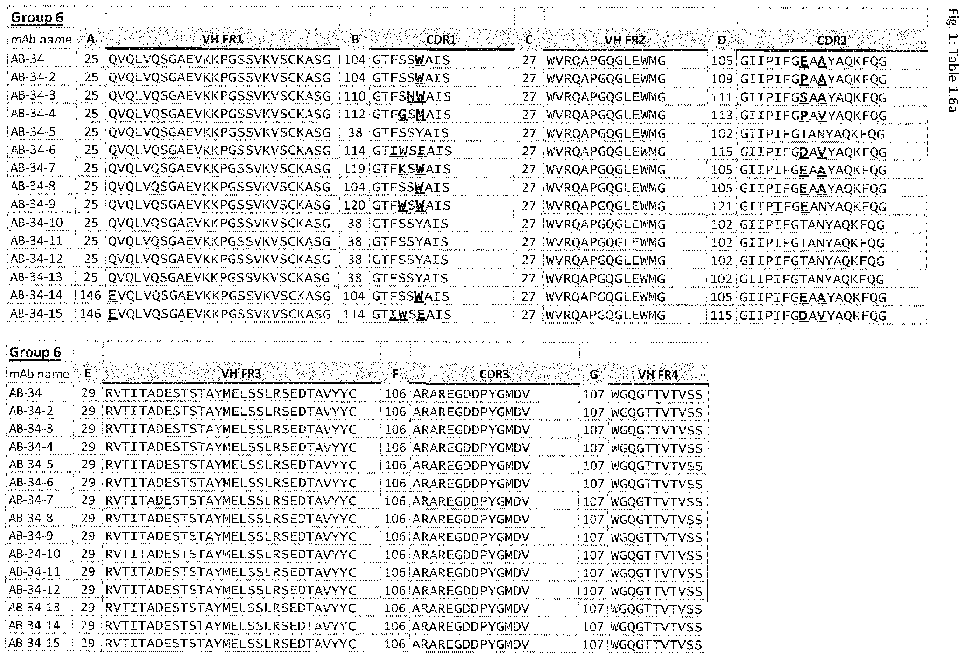

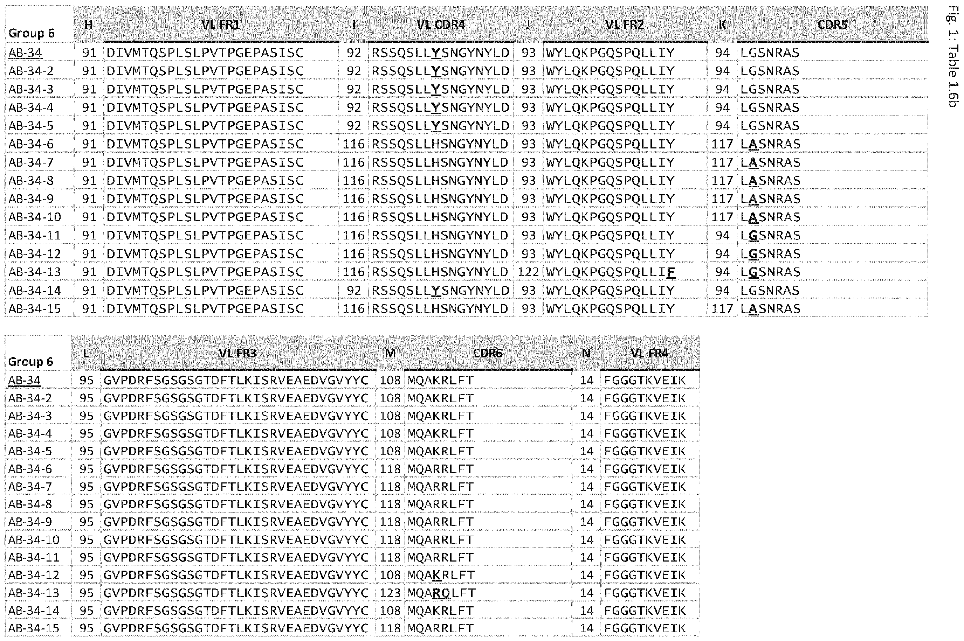

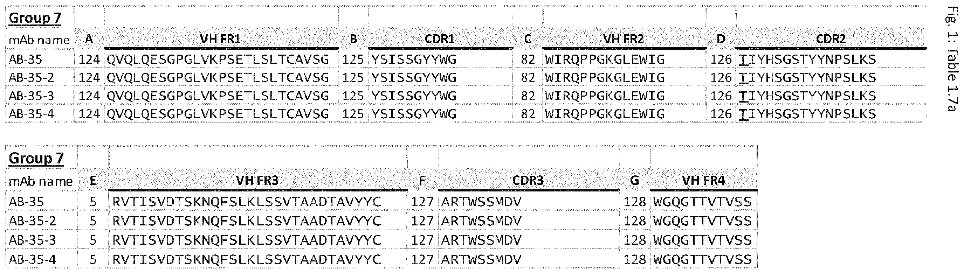

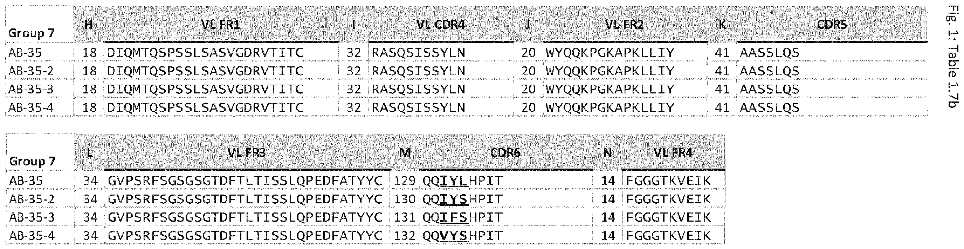

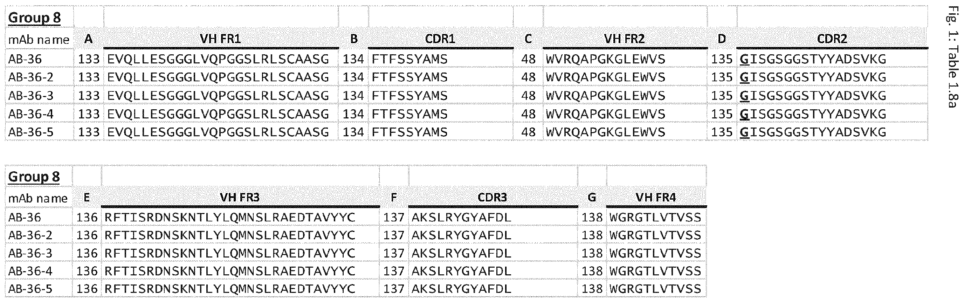

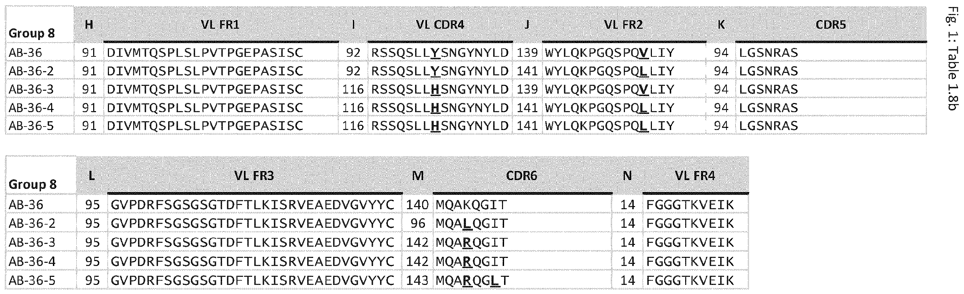

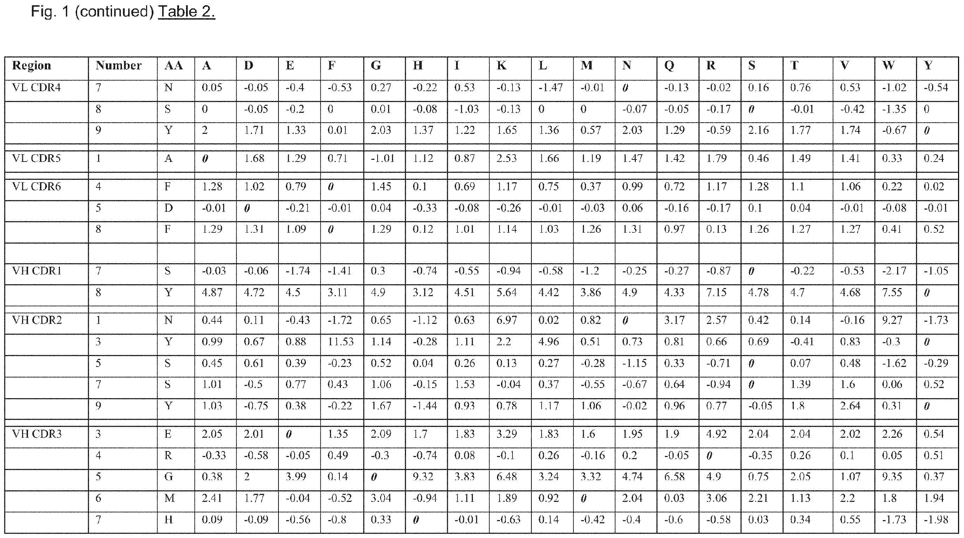

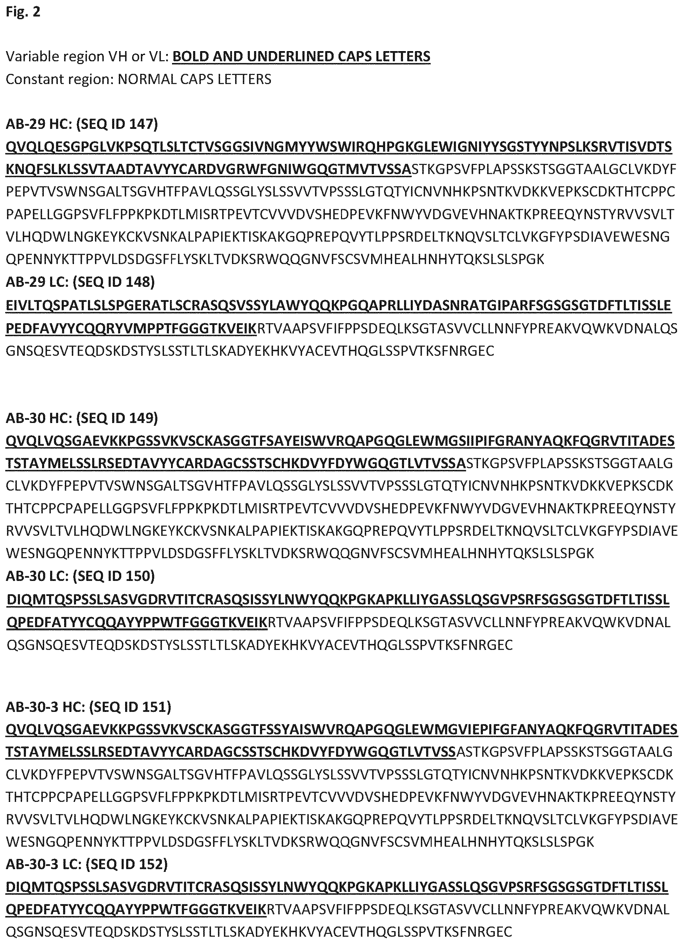

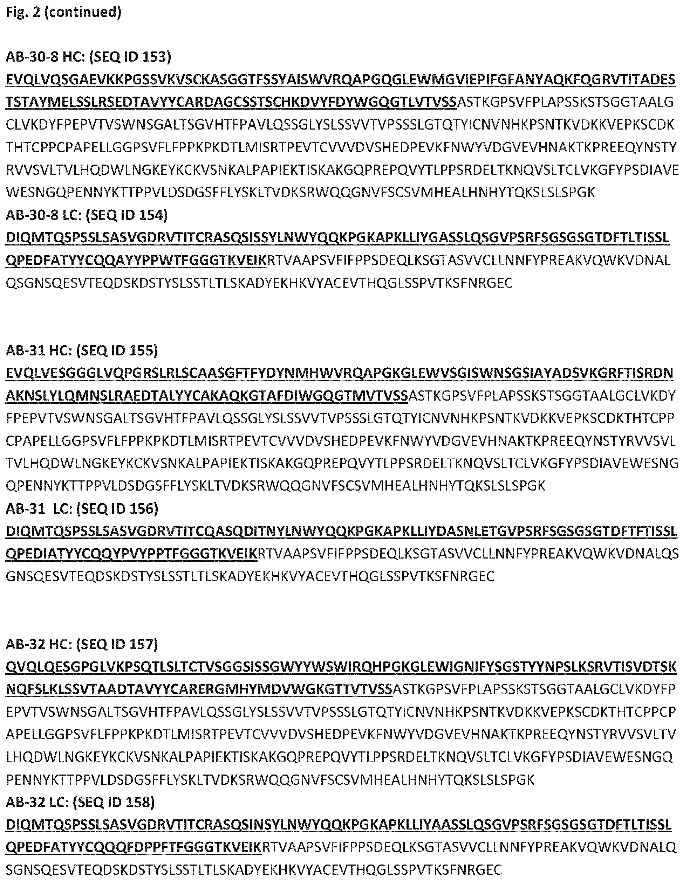

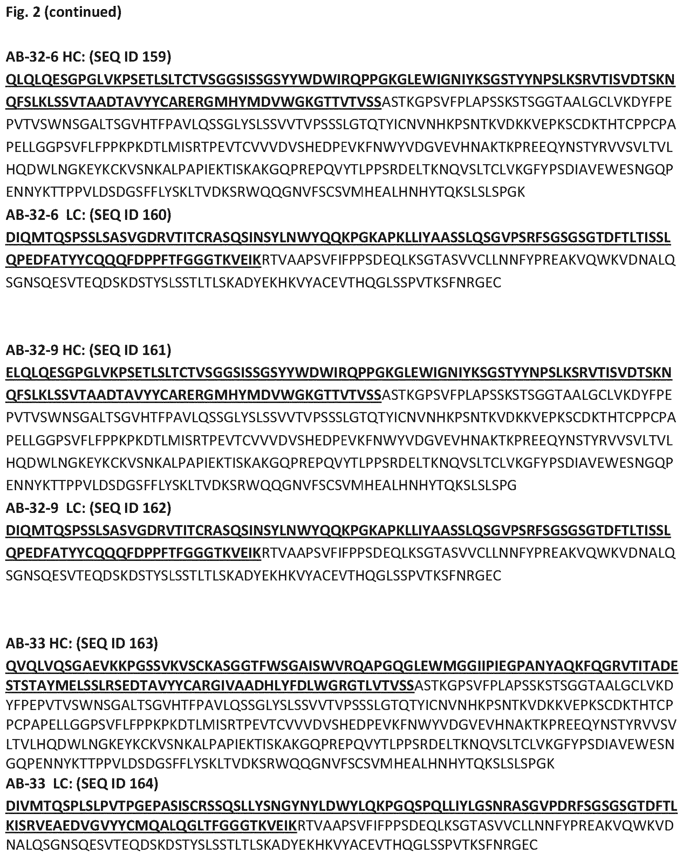

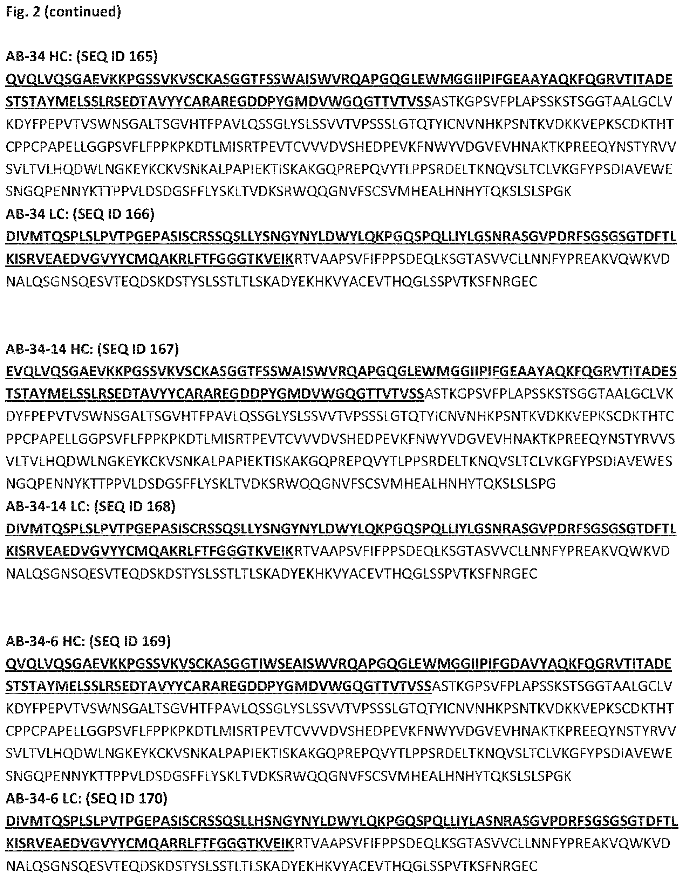

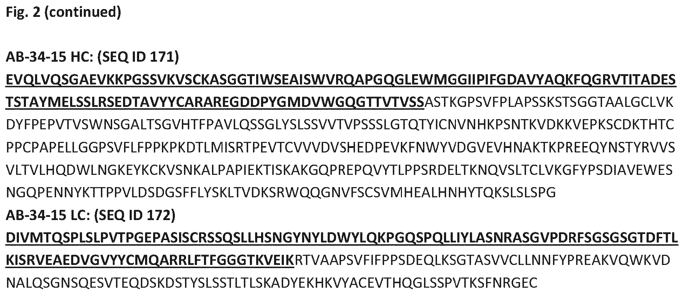

Specifically, the functionally active variant differs from a parent antibody, e.g. any of the antibodies as listed in Figure 1 (Table 1) or Figure 2, in at least one point mutation in the amino acid sequence, preferably in the CDR, e.g. to obtain a CDR variant derived from a parent CDR, wherein the number of point mutations in each of the CDR amino acid sequences is either 0, 1, 2 or 3.

Specifically, the antibody is derived from such antibodies, employing the respective CDR sequences, or CDR mutants, including functionally active CDR variants, e.g. with 1, 2 or 3 point mutations within one CDR loop, e.g. within a CDR length of 5-18 amino acids, e.g. within a CDR region of 5-15 amino acids or 5-10 amino acids. Alternatively, there may be 1 to 2 point mutations within one CDR loop, e.g. within a CDR length of less than 5 amino acids, to provide for an antibody comprising a functionally active CDR variant. Specific CDR sequences might be short, e.g. the CDR2 or CDR5 sequences. According to a specific embodiment, the functionally active CDR variant comprises 1 or 2 point mutations in any CDR sequence consisting of less than 4 or 5 amino acids.

According to a specific aspect, the antibody described herein comprises CDR and framework sequences, wherein at least one of the CDR and framework sequences includes human, humanized, chimeric, murine or affinity matured sequences, preferably wherein the framework sequences are of an IgG antibody, e.g. of an IgG1, IgG2, IgG3, or IgG4 subtype, or of an IgA1, IgA2, IgD, IgE, or IgM antibody.

Specific antibodies are provided as framework mutated antibodies, e.g. to improve manufacturability or tolerability of a parent antibody, e.g. to provide an improved (mutated) antibody which has a low immunogenic potential, such as humanized antibodies with mutations in any of the CDR sequences and/or framework sequences as compared to a parent antibody.

Further specific antibodies are provided as CDR mutated antibodies, e.g. to improve the affinity of an antibody and/or to target the same epitope or epitopes near the epitope that is targeted by a parent antibody (epitope shift).

Accordingly, any of the antibodies as listed in Figure 1 or Figure 2 may be used as parent antibodies to engineer improved versions.

Specifically, the functionally active variant antibody has a specificity to bind the same epitope as the parent antibody.

According to a specific aspect, at least one point mutation is any of an amino acid substitution, deletion and/or insertion of one or more amino acids.

Specifically, the functionally active variant antibody has a specificity to bind the same epitope as the parent antibody.

According to a specific aspect, at least one point mutation is any of an amino acid substitution, deletion and/or insertion of one or more amino acids.

Specifically, the antibody is selected from the group consisting of
a) an antibody comprising
   a. the CDR1 sequence of SEQ ID 38; and
   b. the CDR2 sequence of SEQ ID 39; and
   c. the CDR3 sequence of SEQ ID 30;
b) an antibody comprising
   a. the CDR1 sequence of SEQ ID 47; and
   b. the CDR2 sequence of SEQ ID 49; and
   c. the CDR3 sequence of SEQ ID 51;
c) an antibody comprising
   a. the CDR1 sequence of SEQ ID 83; and
   b. the CDR2 sequence of SEQ ID 84; and
   c. the CDR3 sequence of SEQ ID 73;
d) an antibody comprising
   a. the CDR1 sequence of SEQ ID 104; and
   b. the CDR2 sequence of SEQ ID 105; and
   c. the CDR3 sequence of SEQ ID 106;
   and
e) an antibody comprising
   a. the CDR1 sequence of SEQ ID 114; and
   b. the CDR2 sequence of SEQ ID 115; and
   c. the CDR3 sequence of SEQ ID 106.

Specifically, the antibody comprises
a) a VH amino acid sequence selected from any of the VH sequences as depicted in Figure 2;
b) an antibody heavy chain (HC) amino acid sequence selected from the group consisting of SEQ ID 147, SEQ ID 149, SEQ ID 151, SEQ ID 153, SEQ ID 155, SEQ ID 157, SEQ ID 159, SEQ ID 161, SEQ ID 163, SEQ ID 165, SEQ ID 167, SEQ ID 169, and SEQ ID 171;
c) an antibody heavy chain (HC) amino acid sequence selected from the group consisting of SEQ ID 147, SEQ ID 149, SEQ ID 151, SEQ ID 153, SEQ ID 155, SEQ ID 157, SEQ ID 159, SEQ ID 161, SEQ ID 163, SEQ ID 165, SEQ ID 167, SEQ ID 169, and SEQ ID 171, which is further comprising a deletion of the C-terminal amino acid and/or a Q1E point mutation, if the first amino acid of the VH sequence is a Q.

It is understood that the specific antibody comprises such HC amino acid sequence with or without the respective signal sequence, or with alternative signal or leader sequences.

According to a specific aspect, each of the HC sequences may be terminally extended or deleted in the constant region, e.g. a deletion of one or more or the C-terminal amino acids.

Specifically, each of the HC sequences that comprises an C-terminal Lysine residue is preferably employed with a deletion of such C-terminal Lysine residue.

According to a specific embodiment, the antibody further comprises at least three complementarity determining regions (CDR4 to CDR6) of the antibody light chain variable region (VL).

Specifically, the antibody described herein further comprises an antibody light chain variable region (VL), which comprises any of the CDR4 to CDR6 sequences as listed in Table 1, or functionally active CDR variants thereof.

Specifically, the antibody comprises at least a CDR4 sequence, or at least a CDR4 and a CDR5 sequence, or at least a CDR4, CDR5, and a CDR6 sequence as listed in Table 1.

According to a specific aspect, the antibody described herein comprises CDR combinations as listed in Table 1. According to a specific aspect, the antibody described herein comprises CDR combinations as listed in Table 1, in particular provided, that the antibody is still functionally active.

Specifically, the antibody described herein comprises the CDR1-6 of any of the antibodies as listed in Table 1. However, according to an alternative embodiment, the antibody may comprise different CDR combinations, e.g. wherein an antibody as listed in Table 1 comprises at least one CDR sequence, such as 1, 2, 3, 4, 5, or 6 CDR sequences of one antibody and at least one further CDR sequence of a different antibody of any of the antibodies as listed in Table 1. According to a specific example, the antibody comprises 1, 2, 3, 4, 5, or 6 CDR sequences, wherein the CDR sequences are CDR combinations of more than 1 antibody, e.g. 2, 3, 4, 5, or 6 different antibodies. For example, the CDR sequences may be combined to preferably comprise 1, 2, or all 3 of CDR1-3 of any of the antibodies as listed in Table 1, and 1, 2, or all 3 of CDR4-6 of the same or any other antibody listed in Table 1.

It is herein specifically understood that the CDRs numbered CDR1, 2, and 3 represent the binding region of the VH domain, and CDR4, 5, and 6 represent the binding region of the VL domain.

For example, the CDR sequences may be combined to preferably comprise at least CDR1-3 of any of the antibodies as listed in Table 1, e.g. any of the antibodies designated #AB-29, #AB-30, #AB-31, #AB-32, #AB-33, #AB-34, #AB-35, or #AB-36, or any of the antibodies designated #AB-30-3, #AB-31, #AB-32-6, #AB-34, #AB-34-6, #AB-30-8, #AB-32-9, #AB-34-14, or #AB-34-15, and/or at least CDR4-6 of any of the antibodies as listed in Table 1, e.g. any of the antibodies designated #AB-29, #AB-30, #AB-31, #AB-32, #AB-33, #AB-34, #AB-35, or #AB-36, or any of the antibodies designated #AB-30-3, #AB-31, #AB-32-6, #AB-34, #AB-34-6, #AB-30-8, #AB-32-9, #AB-34-14, or #AB-34-15. According to a specific embodiment, the antibody of the invention comprises the CDR1-6 of any of the antibodies as listed in Table 1, e.g. any of the antibodies designated #AB-29, #AB-30, #AB-31, #AB-32, #AB-33, #AB-34, #AB-35, or #AB-36, or any of the antibodies designated #AB-30-3, #AB-31, #AB-32-6, #AB-34, #AB-34-6, #AB-30-8, #AB-32-9, #AB-34-14, or #AB-34-15. However, according to a further specific aspect, the antibody may comprise different CDR combinations, e.g. wherein an antibody as listed in Table 1, e.g. any of the antibodies designated #AB-29, #AB-30, #AB-31, #AB-32, #AB-33, #AB-34, #AB-35, or #AB-36, or any of the antibodies designated #AB-30-3, #AB-31, #AB-32-6, #AB-34, #AB-34-6, #AB-30-8, #AB-32-9, #AB-34-14, or #AB-34-15, comprises at least one CDR sequence, such as 1, 2, 3, 4, 5, or 6 CDR sequences, of a different antibody, e.g. a CDR sequence of any different antibody of any of the antibodies as listed in Table 1, e.g. any different antibody of any of the antibodies designated #AB-29, #AB-30, #AB-31, #AB-32, #AB-33, #AB-34, #AB-35, or #AB-36, or any different antibody of any of the antibodies designated #AB-30-3, #AB-31, #AB-32-6, #AB-34, #AB-34-6, #AB-30-8, #AB-32-9, #AB-34-14, or #AB-34-15. For example, the antibody comprises 1, 2, 3, 4, 5, or 6 CDR sequences, wherein the CDR sequences are CDR combinations of more than 1 antibody, e.g. 2, 3, 4, 5, or 6 different antibodies.

According to a specific aspect, the antibody described herein comprises any of the HC and LC amino acid sequence combinations as depicted in Figure 1 or Figure 2, or the binding site formed by such combination of HC and LC amino acid sequences. Alternatively, combinations of the immunoglobulin chains of two different antibodies may be used, provided, that the antibody is still functionally active. For example, the HC sequence of one antibody may be combined with an LC sequence of another antibody. According to further specific embodiments, any of the framework regions as provided in Figure 1 or Figure 2 may be employed as a framework to any of the CDR sequences and/or VH/VL combinations as described herein.

Figure 1 shows 8 groups of antibodies characterized by different HC and/or LC sequences with similarities in any of the CDR in each of the groups, and supports any HC/LC combination, in particular a combination of a HC and a LC of the same group. According to a specific aspect one of the CDR1-3 of one HC, e.g. CDR1 is combined with any other CDR sequence of a second and optionally a third HC, e.g. CDR2 and CDR3 of a second and a third HC, respectively, in particular where the combination of CDR1-3 sequences is of the same group. According to a specific aspect one of the CDR4-6 of one LC, e.g. CDR4 is combined with any other CDR sequence of a second and optionally a third LC, e.g. CDR5 and CDR6 of a second and a third LC, respectively, in particular where the combination of CDR4-6 sequences is of the same group.

Specifically, the antibody is selected from the group consisting of group members i) to viii), wherein
i)
   A) is an antibody which comprises
      a) a CDR4 comprising or consisting of the amino acid sequence of SEQ ID 9 or SEQ ID 19; and
      b) a CDR5 comprising or consisting of any of the amino acid sequences of SEQ ID 11, SEQ ID 16, or SEQ ID 21; and
      c) a CDR6 comprising or consisting of any of the amino acid sequences of SEQ ID 13, SEQ ID 17, SEQ ID 23, or SEQ ID 24;
      or
   B) is an antibody which is a functionally active variant of the antibody A, which comprises at least one functionally active CDR variant of
      a) the parent CDR4 consisting of the amino acid sequence of SEQ ID 9 or SEQ ID 19; and/or
      b) the parent CDR5 consisting of any of the amino acid sequences of SEQ ID 11, SEQ ID 16, or SEQ ID 21; and/or
      c) the parent CDR6 consisting of any of the amino acid sequences of SEQ ID 13, SEQ ID 17, SEQ ID 23, or SEQ ID 24;
ii)
   A) is an antibody which comprises
      a) a CDR4 comprising or consisting of the amino acid sequence of SEQ ID 32; and
      b) a CDR5 comprising or consisting of the amino acid sequence of SEQ ID 33 or SEQ ID 41; and
      c) a CDR6 comprising or consisting of any of the amino acid sequences of SEQ ID 35, SEQ ID 42, SEQ ID 43, or SEQ ID 45;
      or
   B) is an antibody which is a functionally active variant of the antibody A, which comprises at least one functionally active CDR variant of
      a) the parent CDR4 consisting of the amino acid sequence of SEQ ID 32; and/or
      b) the parent CDR5 consisting of the amino acid sequence of SEQ ID 33 or SEQ ID 41; and/or
      c) the parent CDR6 consisting of any of the amino acid sequences of SEQ ID 35, SEQ ID 42, SEQ ID 43, and/or SEQ ID 45;
iii)
   A) is an antibody which comprises
      a) a CDR4 comprising or consisting of any of the amino acid sequences of SEQ ID 53, SEQ ID 67, or SEQ ID 19; and
      b) a CDR5 comprising or consisting of the amino acid sequence of SEQ ID 21; and
      c) a CDR6 comprising or consisting of any of the amino acid sequences of SEQ ID 54, SEQ ID 68, SEQ ID 69, or SEQ ID 70;
      or
   B) is an antibody which is a functionally active variant of the antibody A, which comprises at least one functionally active CDR variant of
      a) the parent CDR4 consisting of any of the amino acid sequences of SEQ ID 53, SEQ ID 67, or SEQ ID 19; and/or
      b) the parent CDR5 consisting of the amino acid sequence of SEQ ID 21; and/or
      c) the parent CDR6 consisting of any of the amino acid sequences of SEQ ID 54, SEQ ID 68, SEQ ID 69, or SEQ ID 70;
iv)
   A) is an antibody which comprises
      a) a CDR4 comprising or consisting of the amino acid sequence of SEQ ID 75 or SEQ ID 32; and
      b) a CDR5 comprising or consisting of the amino acid sequence of SEQ ID 41; and
      c) a CDR6 comprising or consisting of the amino acid sequence of SEQ ID 76 or SEQ ID 86;
      or
   B) is an antibody which is a functionally active variant of the antibody A, which comprises at least one functionally active CDR variant of
      a) the parent CDR4 consisting of the amino acid sequence of SEQ ID 75 or SEQ ID 32; and/or
      b) the parent CDR5 consisting of the amino acid sequence of SEQ ID 41; and/or
      c) the parent CDR6 consisting of the amino acid sequence of SEQ ID 76 or SEQ ID 86;
v)
   A) is an antibody which comprises
      a) a CDR4 comprising or consisting of the amino acid sequence of SEQ ID 92; and
      b) a CDR5 comprising or consisting of any of the amino acid sequence of SEQ ID 94; and
      c) a CDR6 comprising or consisting of the amino acid sequence of SEQ ID 96 or SEQ ID 103;
      or
   B) is an antibody which is a functionally active variant of the antibody A, which comprises at least one functionally active CDR variant of
      a) the parent CDR4 consisting of the amino acid sequence of SEQ ID 92; and/or
      b) the parent CDR5 consisting of the amino acid sequence of SEQ ID 94; and/or
      c) the parent CDR6 consisting of the amino acid sequence of SEQ ID 96 or SEQ ID 103;
vi)
   A) is an antibody which comprises
      a) a CDR4 comprising or consisting of the amino acid sequence of SEQ ID 92 or SEQ ID 116; and
      b) a CDR5 comprising or consisting of the amino acid sequence of SEQ ID 94 or SEQ ID 117; and
      c) a CDR6 comprising or consisting of any of the amino acid sequences of SEQ ID 108, SEQ ID 118, or SEQ ID 123;
      or
   B) is an antibody which is a functionally active variant of the antibody A, which comprises at least one functionally active CDR variant of
      a) the parent CDR4 consisting of the amino acid sequence of SEQ ID 92 or SEQ ID 116; and/or
      b) the parent CDR5 consisting of the amino acid sequence of SEQ ID 94 or SEQ ID 117; and/or
      c) the parent CDR6 consisting of any of the amino acid sequences of SEQ ID 108, SEQ ID 118, or SEQ ID 123;
vii)
   A) is an antibody which comprises
      a) a CDR4 comprising or consisting of the amino acid sequence of SEQ ID 32; and
      b) a CDR5 comprising or consisting of the amino acid sequence of SEQ ID 41; and
      c) a CDR6 comprising or consisting of any of the amino acid sequences of SEQ ID 129, SEQ ID 130, SEQ ID 131, or SEQ ID 132;
      or
   B) is an antibody which is a functionally active variant of the antibody A, which comprises at least one functionally active CDR variant of
      a) the parent CDR4 consisting of the amino acid sequence of SEQ ID 32; and/or
      b) the parent CDR5 consisting of the amino acid sequence of SEQ ID 41; and/or
      c) the parent CDR6 consisting of any of the amino acid sequences of SEQ ID 129, SEQ ID 130, SEQ ID 131, or SEQ ID 132;
      and
viii)
   A) is an antibody which comprises
      a) a CDR4 comprising or consisting of the amino acid sequence of SEQ ID 92 or SEQ ID 116; and
      b) a CDR5 comprising or consisting of the amino acid sequence of SEQ ID 94; and
      c) a CDR6 comprising or consisting of any of the amino acid sequences of SEQ ID 140, SEQ ID 96, SEQ ID 142, or SEQ ID 143;
      or
   B) is an antibody which is a functionally active variant of the antibody A, which comprises at least one functionally active CDR variant of
      a) the parent CDR4 consisting of the amino acid sequence of SEQ ID 92 or SEQ ID 116; and/or
      b) the parent CDR5 consisting of the amino acid sequence of SEQ ID 94; and/or
      c) the parent CDR6 consisting of any of the amino acid sequences of SEQ ID 140, SEQ ID 96, SEQ ID 142, or SEQ ID 143;
wherein any of the functionally active CDR variants (in particular as specified in one of the group members i) to viii) above) comprises at least one point mutation in the parent CDR sequence, and comprises or consists of the amino acid sequence that has at least 60% sequence identity with the parent CDR sequence, preferably at least 70%, at least 80%, at least 90% sequence identity.

Specifically, the antibody is an antibody of group member iv) (see above) or a functionally active variant thereof, wherein
a) in VL CDR4 at position 7, the amino acid residue is selected from the group consisting of N, A, D, E, F, G, H, K, L, M, Q, R, S, W and Y, preferentially any of F, L, W, or Y, and more preferentially is L or W; and/or
b) in VL CDR4 at position 8, the amino acid residue is selected from S, A, D, E, F, G, H, I, K, L, M, N, Q, R, T, V, W, and Y, preferentially I or W; and/or
c) in VL CDR4 at position 9, the amino acid residue is selected from Y, F, R and W, and preferentially R or W; and/or
d) in VL CDR5 at position 1, the amino acid residue is selected from A, G, S, W and Y, and preferentially is G; and/or
e) in VL CDR6 at position 4, the amino acid residue is selected from F, H, M, W and Y; and/or
f) in VL CDR6 at position 5, the amino acid residue is selected from D, A, D, E, F, G, H, I, K, L, M, N, Q, R, S, T, V, W, and Y; and/or
g) in VL CDR6 at position 8, the amino acid residue is selected from F, H, R and W.

Specifically, the antibody comprises a VL amino acid sequence selected from any of the VL sequences as depicted in Figure 2, or an antibody light chain (LC) amino acid sequence selected from the group consisting of SEQ ID 148, SEQ ID 150, SEQ ID 152, SEQ ID 154, SEQ ID 156, SEQ ID 158, SEQ ID 160, SEQ ID 162, SEQ ID 164, SEQ ID 166, SEQ ID 168, SEQ ID 170, and SEQ ID 172, or a functionally active CDR variant of any of the foregoing, which has an affinity to bind the LukGH heterodimer with a Kd of less than 10⁻⁸M, preferably less than 10⁻⁹M, preferably less than 10⁻¹⁰M, preferably less than 10⁻¹¹M, e.g. with an affinity in the picomolar range.

Specifically, the antibody or functionally active variant thereof comprises a VL amino acid sequence selected from any of the VL sequences as depicted in Figure 2, Group 4, or an antibody light chain (LC) amino acid sequence selected from the group consisting of SEQ ID 158, SEQ ID 160, SEQ ID 162, wherein
a) in VL CDR4 at position 7, the amino acid residue is selected from the group consisting of N, A, D, E, F, G, H, K, L, M, Q, R, S, W and Y, preferentially any of F, L, W, or Y, and more preferentially is L or W; and/or
b) in VL CDR4 at position 8, the amino acid residue is selected from S, A, D, E, F, G, H, I, K, L, M, N, Q, R, T, V, W, and Y, preferentially I or W; and/or
c) in VL CDR4 at position 9, the amino acid residue is selected from Y, F, R and W, and preferentially R or W; and/or
d) in VL CDR5 at position 1, the amino acid residue is selected from A, G, S, W and Y, and preferentially is G; and/or
e) in VL CDR6 at position 4, the amino acid residue is selected from F, H, M, W and Y; and/or
f) in VL CDR6 at position 5, the amino acid residue is selected from D, A, D, E, F, G, H, I, K, L, M, N, Q, R, S, T, V, W, and Y; and/or
g) in VL CDR6 at position 8, the amino acid residue is selected from F, H, R and W.

Specifically, the antibody is selected from the group consisting of
a) an antibody comprising
   a. the CDR1 sequence of SEQ ID 38; and
   b. the CDR2 sequence of SEQ ID 39; and
   c. the CDR3 sequence of SEQ ID 30; and
   d. the CDR4 sequence of SEQ ID 32; and
   e. the CDR5 sequence of SEQ ID 33; and
   f. the CDR6 sequence of SEQ ID 35;
b) an antibody comprising
   a. the CDR1 sequence of SEQ ID 47; and
   b. the CDR2 sequence of SEQ ID 49; and
   c. the CDR3 sequence of SEQ ID 51; and
   d. the CDR4 sequence of SEQ ID 53; and
   e. the CDR5 sequence of SEQ ID 21; and
   f. the CDR6 sequence of SEQ ID 54;
c) an antibody comprising
   a. the CDR1 sequence of SEQ ID 83; and
   b. the CDR2 sequence of SEQ ID 84; and
   c. the CDR3 sequence of SEQ ID 73; and
   d. the CDR4 sequence of SEQ ID 75; and
   e. the CDR5 sequence of SEQ ID 41; and
   f. the CDR6 sequence of SEQ ID 76;
d) an antibody comprising
   a. the CDR1 sequence of SEQ ID 104; and
   b. the CDR2 sequence of SEQ ID 105; and
   c. the CDR3 sequence of SEQ ID 106; and
   d. the CDR4 sequence of SEQ ID 92; and
   e. the CDR5 sequence of SEQ ID 94; and
   f. the CDR6 sequence of SEQ ID 108;
e) and an antibody comprising
   a. the CDR1 sequence of SEQ ID 114; and
   b. the CDR2 sequence of SEQ ID 115; and
   c. the CDR3 sequence of SEQ ID 106; and
   d. the CDR4 sequence of SEQ ID 116; and
   e. the CDR5 sequence of SEQ ID 117; and
   f. the CDR6 sequence of SEQ ID 118
or a functionally active CDR variant of any of the foregoing (in particular of an antibody as specified in one of the group members a) to e) above), which has an affinity to bind the LukGH heterodimer with a Kd of less than 10⁻⁸M, preferably less than 10⁻⁹M, preferably less than 10⁻¹⁰M, preferably less than 10⁻¹¹M, e.g. with an affinity in the picomolar range.

Specifically, the antibody is an antibody of group member c) (see above, characterized by the CDR1 sequence of SEQ ID 83; the CDR2 sequence of SEQ ID 84; the CDR3 sequence of SEQ ID 73; the CDR4 sequence of SEQ ID 75; the CDR5 sequence of SEQ ID 41; and the CDR6 sequence of SEQ ID 76) or a functionally active variant thereof, wherein:
a) in VH CDR1 at position 7, the amino acid residue is selected from S, A, D, E, F, G, H, I, K, L, M, N, Q, R, T, V, W, and Y, preferentially any of E, F, H, I, K, L, M, R, V, W or Y, and more preferentially is any of E, F, M, W or Y; and/or
b) in VH CDR2 at position 1, the amino acid residue is selected from N, A, D, E, F, H, L, S, T, V and Y, preferentially any of F, H or Y; and/or
c) in VH CDR2 at position 3, the amino acid residue is selected from Y, H, T and W; and/or
d) in VH CDR2 at position 5, the amino acid residue is selected from S, A, E, F, H, I, K, L, M, N, Q, R, T, V, W and Y, preferentially any of N, R or W, and more preferentially is N or W; and/or
e) in VH CDR2 at position 7, the amino acid residue is selected from S, D, F, H, K, L, M, N, R and W; and/or
f) in VH CDR2 at position 9, the amino acid residue is selected from Y, D, E, F, N, S and W, preferentially D or H, and more preferentially is H; and/or
g) in VH CDR3 at position 4, the amino acid residue is selected from R, A, D, E, F, G, H, I, K, L, M, N, Q, S, T, V and W, preferentially D or H; and/or
h) in VH CDR3 at position 5, the amino acid residue is selected from G, A, F and Y; and/or
i) in VH CDR3 at position 6, the amino acid residue is selected from M, E, F, H and Q, preferentially F or H; and/or
j) in VH CDR3 at position 7, the amino acid residue is selected from H, A, D, E, F, G, I, K, L, M, N, Q, R, S, T, W and Y, preferentially any of E, K, Q, R, W or Y, and more preferentially is W or Y; and/or
k) in VL CDR4 at position 7, the amino acid residue is selected from the group consisting of N, A, D, E, F, G, H, K, L, M, Q, R, S, W and Y, preferentially any of F, L, W, or Y, and more preferentially is L or W; and/or
l) in VL CDR4 at position 8, the amino acid residue is selected from S, A, D, E, F, G, H, I, K, L, M, N, Q, R, T, V, W, and Y, preferentially I or W; and/or
m) in VL CDR4 at position 9, the amino acid residue is selected from Y, F, R and W, and preferentially R or W; and/or
n) in VL CDR5 at position 1, the amino acid residue is selected from A, G, S, W and Y, and preferentially is G; and/or
o) in VL CDR6 at position 4, the amino acid residue is selected from F, H, M, W and Y; and/or
p) in VL CDR6 at position 5, the amino acid residue is selected from D, A, D, E, F, G, H, I, K, L, M, N, Q, R, S, T, V, W, and Y; and/or
q) in VL CDR6 at position 8, the amino acid residue is selected from F, H, R and W.

Specifically, the antibody comprises a framework, such as an immunoglobulin framework consisting of immunoglobulin constant regions or constant domains, including any of the framework regions of the VH and/or VL as listed in Table 1, optionally comprising a Q1E point mutation, if the first amino acid of the VH framework region (VH FR1) is a Q.

Specifically, the antibody comprises a HC amino acid sequence as shown in Figure 2.

Specifically, the antibody is selected from the group consisting of
a) an antibody comprising
   a. the HC amino acid sequence of SEQ ID 147; and
   b. the LC amino acid sequence of SEQ ID 148;
b) an antibody comprising
   a. the HC amino acid sequence of SEQ ID 149; and
   b. the LC amino acid sequence of SEQ ID 150;
c) an antibody comprising
   a. the HC amino acid sequence of SEQ ID 151; and
   b. the LC amino acid sequence of SEQ ID 152;
d) an antibody comprising
   a. the HC amino acid sequence of SEQ ID 153; and
   b. the LC amino acid sequence of SEQ ID 154;
e) an antibody comprising
   a. the HC amino acid sequence of SEQ ID 155; and
   b. the LC amino acid sequence of SEQ ID 156;
f) an antibody comprising
   a. the HC amino acid sequence of SEQ ID 157; and
   b. the LC amino acid sequence of SEQ ID 158;
g) an antibody comprising
   a. the HC amino acid sequence of SEQ ID 159; and
   b. the LC amino acid sequence of SEQ ID 160;
h) an antibody comprising
   a. the HC amino acid sequence of SEQ ID 161; and
   b. the LC amino acid sequence of SEQ ID 162;
i) an antibody comprising
   a. the HC amino acid sequence of SEQ ID 163; and
   b. the LC amino acid sequence of SEQ ID 164;
j) an antibody comprising
   a. the HC amino acid sequence of SEQ ID 165; and
   b. the LC amino acid sequence of SEQ ID 166;
k) an antibody comprising
   a. the HC amino acid sequence of SEQ ID 167; and
   b. the LC amino acid sequence of SEQ ID 168;
l) an antibody comprising
   a. the HC amino acid sequence of SEQ ID 169; and
   b. the LC amino acid sequence of SEQ ID 170;
   and
m) an antibody comprising
   a. the HC amino acid sequence of SEQ ID 171; and
   b. the LC amino acid sequence of SEQ ID 172
or a functionally active CDR variant of any of the foregoing (in particular of an antibody as specified in one of the group members a) to m) above), which has an affinity to bind the LukGH heterodimer with a Kd of less than 10⁻⁸M, preferably less than 10⁻⁹M, preferably less than 10⁻¹⁰M, preferably less than 10⁻¹¹M, e.g. with an affinity in the picomolar range.

Specifically, the antibody is an antibody of any of group member f) (see above, characterized by the HC amino acid sequence of SEQ ID 157; and the LC amino acid sequence of SEQ ID 158), g) (characterized by the HC amino acid sequence of SEQ ID 159; and the LC amino acid sequence of SEQ ID 160), and h) (characterized by the HC amino acid sequence of SEQ ID 161; and the LC amino acid sequence of SEQ ID 162), or a functionally active variant of any of the foregoing, wherein
a) in VH CDR1 at position 7, the amino acid residue is selected from S, A, D, E, F, G, H, I, K, L, M, N, Q, R, T, V, W, and Y, preferentially any of E, F, H, I, K, L, M, R, V, W or Y, and more preferentially is any of E, F, M, W or Y; and/or
b) in VH CDR2 at position 1, the amino acid residue is selected from N, A, D, E, F, H, L, S, T, V and Y, preferentially any of F, H or Y; and/or
c) in VH CDR2 at position 3, the amino acid residue is selected from Y, H, T and W; and/or
d) in VH CDR2 at position 5, the amino acid residue is selected from S, A, E, F, H, I, K, L, M, N, Q, R, T, V, W and Y, preferentially any of N, R or W, and more preferentially is N or W; and/or
e) in VH CDR2 at position 7, the amino acid residue is selected from S, D, F, H, K, L, M, N, R and W; and/or
f) in VH CDR2 at position 9, the amino acid residue is selected from Y, D, E, F, N, S and W, preferentially D or H, and more preferentially is H; and/or
g) in VH CDR3 at position 4, the amino acid residue is selected from R, A, D, E, F, G, H, I, K, L, M, N, Q, S, T, V and W, preferentially D or H; and/or
h) in VH CDR3 at position 5, the amino acid residue is selected from G, A, F and Y; and/or
i) in VH CDR3 at position 6, the amino acid residue is selected from M, E, F, H and Q, preferentially F or H; and/or
j) in VH CDR3 at position 7, the amino acid residue is selected from H, A, D, E, F, G, I, K, L, M, N, Q, R, S, T, W and Y, preferentially any of E, K, Q, R, W or Y, and more preferentially is W or Y; and/or
k) in VL CDR4 at position 7, the amino acid residue is selected from the group consisting of N, A, D, E, F, G, H, K, L, M, Q, R, S, W and Y, preferentially any of F, L, W, or Y, and more preferentially is L or W; and/or
l) in VL CDR4 at position 8, the amino acid residue is selected from S, A, D, E, F, G, H, I, K, L, M, N, Q, R, T, V, W, and Y, preferentially I or W; and/or
m) in VL CDR4 at position 9, the amino acid residue is selected from Y, F, R and W, and preferentially R or W; and/or
n) in VL CDR5 at position 1, the amino acid residue is selected from A, G, S, W and Y, and preferentially is G; and/or
o) in VL CDR6 at position 4, the amino acid residue is selected from F, H, M, W and Y; and/or
p) in VL CDR6 at position 5, the amino acid residue is selected from D, A, D, E, F, G, H, I, K, L, M, N, Q, R, S, T, V, W, and Y; and/or
q) in VL CDR6 at position 8, the amino acid residue is selected from F, H, R and W.

According to a specific aspect, the antibody described herein comprises any of the HC and LC amino acid sequence combinations as depicted in Figure 2, or the binding site formed by such combination of HC and LC amino acid sequences.

Specifically, the antibody has an affinity to bind the LukGH heterodimer with a Kd of less than 10⁻⁸M, preferably less than 10⁻⁹M, preferably less than 10⁻¹⁰M, preferably less than 10⁻¹¹M, e.g. with an affinity in the picomolar range.

Specifically, the antibody has an affinity to bind the individual LukG and/or LukH antigens which is lower than the affinity to bind the LukGH heterodimer, preferably with a Kd of higher than 10⁻⁷M, preferably higher than 10⁻⁶M.

Specifically, the Kd difference to preferentially bind the LukGH heterodimer over the individual LukG or LukH antigens is at least 2 logs, preferably at least 3 logs.

Further provided herein is a method of producing functionally active antibody variants of a parent antibody which is any of the antibodies of the invention which comprises any of the HC and LC amino acid sequence combinations as depicted in Figure 2, or the binding site formed by such combination of HC and LC amino acid sequences, which method comprises engineering at least one point mutation in any of the framework regions (FR) or constant domains, or complementarity determining regions (CDR1 to CDR6) to obtain a variant antibody, and determining the functional activity of the variant antibody by the affinity to bind the LukGH heterodimer with a Kd of less than 10⁻⁸M, preferably less than 10⁻⁹M, preferably less than 10⁻¹⁰M, preferably less than 10⁻¹¹M, e.g. with an affinity in the picomolar range, wherein upon determining the functional activity, the functionally active variants are selected for production by a recombinant production method.

Specifically, the LukGH heterodimer is characterized by an antigenic structure which is resulting from assembly of the individual LukG and LukH antigens in solution.

Specifically, the LukGH heterodimer is provided as isolated and optionally purified complex antigen.

Specifically, the LukGH heterodimer comprises the LukG and LukH components as a heterodimer or heterooligomer.

Specifically, the LukGH heterodimer is composed of recombinant proteins and/or proteins derived from S. *aureus* strains.

Specifically, the LukG and LukH components are co-expressed by a recombinant host cell, purified from native sources and/or co-refolded.

Specifically, the antigen is provided as a protein complex in the soluble form.

According to a specific aspect, the antibody inhibits the binding of the LukGH heterodimer to phosphocholine or phosphatidylcholine, in particular the phosphatidylcholine of mammalian cell membranes.

Specifically, the antibody is capable of neutralizing the LukGH heterodimer.

Specifically, the antibody of the invention is cross-reactive between different LukGH variants.

Specifically, the antibody is cross-neutralizing the LukGH heterodimer and the LukGH heterodimer variants.

Specifically, the antibody is binding to the LukGH heterodimer derived from the USA300 clone, preferably from the TCH1516 strain, and at least one of the LukGH complex variants.

Specifically, the LukGH heterodimer variants have at least one point mutation in the amino acid sequences of any of the LukG or LukH components, as compared to the LukGH heterodimer derived from the USA300 clone, e.g. a change in one or more of the amino acid residues in the sequence. Even the very different LukGH heterodimer variants derived from MRSA252 and MSHR1132 strains may be cross-specifically bound by the antibody of the invention, and cross-neutralized.

Specifically, the antibody described herein is a cross-neutralizing antibody comprising at least one binding site that binds to LukGH from USA300 clone (eg strain TCH_1516) and at least one of the LukGH variants. Specifically the LukGH toxin is selected from the group consisting of genes expressed by the EMRSA16 MRSA252 strain or the MSHR1132 strain.

According to a specific aspect, the antibody exhibits *in vitro* neutralization potency in a cell-based assay with an IC50 of less than 100:1 mAb:toxin ratio (mol/mol), preferably less than 50:1, preferably less than 25:1, preferably less than 10:1, more preferably less than 1:1.

According to a further specific aspect, the antibody neutralizes the targeted LukGH heterodimer in animals, including both, human and non-human animals, and inhibits *S*. *aureus* pathogenesis *in vivo,* preferably any models of pneumonia, bacteremia, sepsis, abscess, skin infection, peritonitis, catheter and prothetic devices related infection and osteomyelitis.

Specifically, the antibody is a full-length monoclonal antibody, an antibody fragment thereof comprising at least one antibody domain incorporating the binding site, or a fusion protein comprising at least one antibody domain incorporating the binding site. Preferably, the antibody is selected from the group consisting of murine, chimeric, humanized or human antibodies, heavy-chain antibodies, Fab, Fd, scFv and single-domain antibodies like VH, VHH or VL, preferably a human IgG1 antibody.

Further provided herein is an expression cassette or a plasmid comprising a coding sequence to express a light chain and/or heavy chain of an antibody of the invention.

Further provided herein is a host cell comprising the expression cassette or the plasmid of the invention.

Specifically preferred is a host cell and a production method employing such host cell, which host cell comprises
- the plasmid or expression cassette of the invention, which incorporates a coding sequence to express the antibody light chain; and
- the plasmid or expression cassette of the invention, which incorporates a coding sequence to express the antibody heavy chain.

Further provided herein is a method of producing an antibody according to the invention, wherein a host cell of the invention is cultivated or maintained under conditions to produce said antibody.

Further provided herein is a method of producing functionally active antibody variants of a parent antibody which is any of the antibodies of the invention, e.g. an antibody as listed in Figure 1 or Figure 2, or comprising any of the HC and LC amino acid sequence combinations as depicted in Figure 1 or Figure 2, or comprising the binding site formed by such combination of HC and LC amino acid sequences, which method comprises engineering at least one point mutation in any of the framework regions (FR) or constant domains, or complementarity determining regions (CDR1 to CDR6) to obtain a variant antibody, and determining the functional activity of the variant antibody by any of
- the affinity to bind the LukGH heterodimer with a Kd of less than 10⁻⁸M, preferably less than 10⁻⁹M, preferably less than 10⁻¹⁰M, preferably less than 10⁻¹¹M, e.g. with an affinity in the picomolar range, and/or
- the binding of the variant antibody to the LukGH heterodimer in competition with the parent antibody;
wherein upon determining the functional activity, the functionally active variants are selected for production by a recombinant production method.

Functionally active variant antibodies may differ in any of the VH or VL sequences, or share the common VH and VL sequences, and comprise modifications in the respective FR. The variant antibody derived from the parent antibody by mutagenesis may be produced a method well-known in the art.

Exemplary parent antibodies are described in the examples section below and in Figure 1 and Figure 2. Specifically, the antibody is a functionally active derivative of a parent antibody as listed in Figure 1 or Figure 2. Variants with one or more modified CDR sequences, and/or with one or more modified FR sequences, such as sequences of FR1, FR2, FR3 or FR4, or a modified constant domain sequence may be engineered.

Further provided herein is a method of producing an antibody of the invention, comprising
(a) immunizing a non-human animal with the three-dimensional structure of the epitope as defined herein;
(b) forming immortalized cell lines from the isolated B-cells;
(c) screening the cell lines to identify a cell line producing a monoclonal antibody that binds to the LukGH heterodimer of *Staphylococcus aureus* and optionally and at least one of the LukGH heterodimer variants; and
(d) producing the monoclonal antibody, or a humanized or human form of the antibody, or a derivative thereof with the same epitope binding specificity as the monoclonal antibody.

According to a further aspect, the invention provides for a method of producing an antibody of the invention, comprising
(a) immunizing a non-human animal with the LukGH heterodimer of *Staphylococcus aureus* and isolating B-cells producing antibodies;
(b) forming immortalized cell lines from the isolated B-cells;
(c) screening the cell lines to identify a cell line producing a monoclonal antibody that binds to the LukGH heterodimer of *Staphylococcus aureus* and optionally and at least one of the LukGH heterodimer variants; and
(d) producing the monoclonal antibody, or a humanized or human form of the antibody, or a derivative thereof with the same epitope binding specificity as the monoclonal antibody.

According to a further aspect, the antibody is provided for medical use, specifically for use in treating a subject at risk of or suffering from a *S. aureus* infection comprising administering to the subject an effective amount of the antibody to limit the infection in the subject, to ameliorate a disease condition resulting from said infection or to inhibit S. *aureus* disease pathogenesis, such as pneumonia, sepsis, bacteremia, wound infection, abscesses, surgical site infection, endothalmitis, furunculosis, carbunculosis, endocarditis, peritonitis, osteomyelitis or joint infection.

Specifically the antibody is provided for protecting against *S. aureus* infections.

According to a further aspect, there is further provided a method of treatment, wherein the antibody provided herein is used in an effective amount to treat a subject at risk of or suffering from a *S. aureus* infection or a disease condition resulting from said infection, to limit the infection in the subject, to ameliorate a disease condition resulting from said infection, or to inhibit *S. aureus* disease pathogenesis, such as pneumonia, sepsis, bacteremia, wound infection, abscesses, surgical site infection, endothalmitis, furunculosis, carbunculosis, endocarditis, peritonitis, osteomyelitis or joint infection.

Specifically, the method of treatment is provided for protecting against pathogenic *S. aureus.*

According to a further aspect, there is further provided a pharmaceutical preparation comprising the antibody described herein, preferably comprising a parenteral or mucosal formulation, optionally containing a pharmaceutically acceptable carrier or excipient.

Such pharmaceutical composition may contain the antibody as the sole active substance, or in combination with other active substances, or a cocktail of active substances, such as a combination or cocktail of at least two or three different antibodies, e.g. wherein the other active substances are further targeting *S. aureus,* e.g. an OPK antibody or an antibody targeting at least one other toxin. Specifically, the cocktail of antibodies comprises one or more antibodies of the invention, each targeting toxins and the combination targeting more different epitopes or toxins than only one antibody in the cocktail.

According to a further aspect, the antibody is provided for diagnostic use to detect any *S. aureus* infections, including high toxin producing MRSA infections, such as necrotizing pneumonia, and toxin production in furunculosis and carbunculosis. Specifically, the antibody provided herein is used in an in vitro diagnostic method to detect any *S. aureus* infections.

According to a further aspect, there is further provided a diagnostic preparation of the antibody described herein, optionally containing the antibody with a label and/or a further diagnostic reagent with a label.

Specifically, the antibody is provided for diagnostic use as described herein, wherein a systemic infection with *S. aureus* in a subject is determined *ex vivo* by contacting a sample of body fluid of said subject with the antibody, wherein a specific immune reaction of the antibody determines the infection.

Therefore, the invention further refers to the respective method of diagnosing an *S. aureus* infection in a subject, in particular wherein a systemic infection with *S. aureus* in a subject is determined.

According to a further aspect, there is further provided isolated nucleic acid molecules encoding an antibody of the invention.

According to a further aspect, there is provided the isolated paratope of an antibody described herein, or a binding molecule comprising said paratope.

According to a further aspect, there is provided an isolated conformational epitope recognized by the antibody described herein. Such epitope may consist of a single epitope or a mixture of epitopes comprising epitope variants, each recognized by the antibody of the invention.

The epitope is specifically characterized by
a) a three-dimensional structure of the LukGH heterodimer of *S. aureus* comprising the rim domain of LukG and the structure coordinates of the contact amino acid residues Asn71, Tyr73, Trp74, Asn206, Leu207, Trp208, Lys210, Asp211, Trp262, and Phe267; or
b) a three-dimensional structure which is a homolog of a) wherein said homolog comprises a binding site that has a root mean square deviation from backbone atoms of contact amino acid residues of between 0.00 A and 2.00 A.

Specifically, the epitope is bound or recognized by a binding molecule.

According to a further aspect, there is provided a binding molecule which specifically recognizes the epitope of as described herein, preferably selected from the group consisting of a protein, a peptide, a peptidomimetic, a nucleic acid, a carbohydrate, a lipid, an oligopeptide, an aptamer and a small molecule compound, preferably an antibody, an antibody fragment thereof comprising at least one antibody domain incorporating the binding site, or a fusion protein comprising at least one antibody domain incorporating the binding site. Specifically, the binding molecule is a specific binder that recognizes the LukGH heterodimer of *S. aureus.* Specifically, the binder prevents the LukGH binding to the target cell and competes with the antibody of the invention.

According to a further aspect, there is provided a screening method or assay for identifying a binder which specifically recognizes the epitope as described herein, comprising the steps of:
- bringing a candidate compound into contact with the three-dimensional structure as described herein (specifically characterizing the conformational epitope); and
- assessing binding between the candidate compound and the three- dimensional structure, wherein binding between the candidate compound and the three-dimensional structure identifies the candidate compound as a specific binder that recognizes the LukGH heterodimer of *S. aureus.* For example, a positive functional binding reaction between the candidate compound and the three-dimensional structure or the epitope identifies the compound as protective binder.

According to a further aspect, there is provided an immunogen comprising:
a) an epitope as described herein; and
b) a carrier, preferably a pharmaceutically acceptable carrier, preferably comprising buffer and/or adjuvant substances.

Specifically, the immunogen is provided in a vaccine formulation, preferably for parenteral use.

Specifically, the immunogen as described herein is provided for use in treating a subject by administering an effective amount of said immunogen to protect the subject from an *S. aureus* infection, to prevent a disease condition resulting from said infection or to inhibit *S. aureus* pneumonia pathogenesis.

Specifically, the immunogen is used for eliciting a protective immune response.

According to a specific aspect, there is further provided a method of treatment wherein a subject at risk of a *S. aureus* infection is treated, which method comprises administering to the subject an effective amount of the immunogen to prevent infection in the subject, in particular to protect against pathogenic *S. aureus.*

According to a further aspect, the invention provides for isolated nucleic acid molecules encoding an antibody of the invention.

### FIGURES

Figure 1:
   Table 1: Amino acid sequences of LukGH specific mAbs
      Legend: Columns
      A ... SEQ ID VH FR1
      B ... SEQ ID VH CDR1
      C ... SEQ ID VH FR2
      D ... SEQ ID VH CDR2
      E ... SEQ ID VH FR3
      F ... SEQ ID VH CDR3
      G ... SEQ ID VH FR4
      H ... SEQ ID VL FR1
      I ... SEQ ID VL CDR4
      J ... SEQ ID VL FR2
      K ... SEQ ID VL CDR5
      L ... SEQ ID VL FR3
      M ... SEQ ID VL CDR6
      N ... SEQ ID VL FR4
      The nomenclature as used in Figure 1 shall have the following meaning:
      VH CDR1 = CDR1
      VH CDR2 = CDR2
      VH CDR3 = CDR3
      VL CDR4 = CDR4 = VL CDR1
      VL CDR5 = CDR5 = VL CDR2
      VL CDR6 = CDR6 = VL CDR3
      Table 1 is divided in eight parts (for antibodies of Group 1-8): Table 1.1 - 1.8.
      Table 1.1a shows the VH FR and CDR sequences of the antibodies of Group 1;
      Table 1.1b shows the VL FR and CDR sequences of the antibodies of Group 1;
      Table 1.2a shows the VH FR and CDR sequences of the antibodies of Group 2;
      Table 1.2b shows the VL FR and CDR sequences of the antibodies of Group 2;
      Table 1.3a shows the VH FR and CDR sequences of the antibodies of Group 3;
      Table 1.3b shows the VL FR and CDR sequences of the antibodies of Group 3;
      Table 1.4a shows the VH FR and CDR sequences of the antibodies of Group 4;
      Table 1.4b shows the VL FR and CDR sequences of the antibodies of Group 4;
      Table 1.5a shows the VH FR and CDR sequences of the antibodies of Group 5;
      Table 1.5b shows the VL FR and CDR sequences of the antibodies of Group 5;
      Table 1.6a shows the VH FR and CDR sequences of the antibodies of Group 6;
      Table 1.6b shows the VL FR and CDR sequences of the antibodies of Group 6;
      Table 1.7a shows the VH FR and CDR sequences of the antibodies of Group 7;
      Table 1.7b shows the VL FR and CDR sequences of the antibodies of Group 7;
      Table 1.8a shows the VH FR and CDR sequences of the antibodies of Group 8;
      Table 1.8b shows the VL FR and CDR sequences of the antibodies of Group 8;
   Table 2: Change in binding energies (ΔΔG) upon mutation of AB-32-9 contact residues to LukGH.
Figure 2: Full length amino acid sequences of selected LukGH specific mAbs
Figure 3: *S.aureus* LukG and LukH toxin sequences
   LukH nucleotide sequence of the USA300 TCH1516 strain (Genbank,accession number CP000730), SEQ ID 173;
   LukH amino acid sequence of the USA300 TCH1516 strain, SEQ ID 174;
   LukG nucleotide sequence of the USA300 TCH1516 strain, SEQ ID 175;
   LukG amino acid sequence of the USA300 TCH1516 strain, SEQ ID 176;
   LukH nucleotide sequence of the MRSA252 strain (Genbank,accession number BX571856), SEQ ID 177;
   LukH amino acid sequence of the MRSA252 strain, SEQ ID 178;
   LukG nucleotide sequence of the MRSA252 strain, SEQ ID 179;
   LukG amino acid sequence of the MRSA252 strain, SEQ ID 180;
   LukH nucleotide sequence of the MSHR1132 strain (Genbank,accession number FR821777), SEQ ID 181;
   LukH amino acid sequence of the MSHR1132 strain, SEQ ID 182;
   LukG nucleotide sequence of the MSHR1132 strain, SEQ ID 183;
   LukG amino acid sequence of the MSHR1132 strain, SEQ ID 184;
   LukH nucleotide sequence of the H19 strain (Patric, genome ID 72956; Genebank, accession number ACSS01000001 to ACSS01000063), SEQ ID 185;
   LukH amino acid sequence of the H19 strain, SEQ ID 186;
   LukG nucleotide sequence of the H19 strain, SEQ ID 187;
   LukG amino acid sequence of the H19 strain, SEQ ID 188.

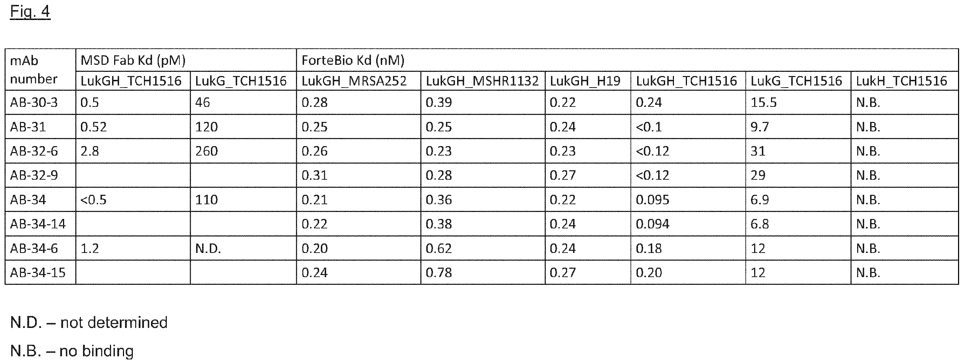

Figure 4: Binding affinity of selected LukGH mAbs

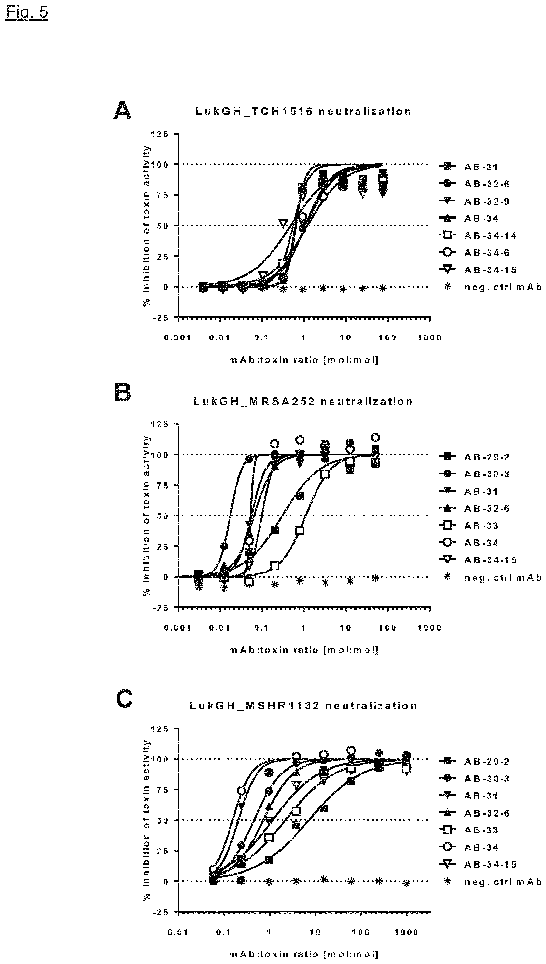

Figure 5: In vitro neutralization potency of LukGH mAbs: mAbs were serially diluted in assay medium and preincubated with recombinant LukGH_TCH1516 (A), LukGH_MRSA252 (B) and LukGH_MSHR1132 (C) before intoxication of human neutrophil-like differentiated HL-60 cells (A,B) or human neutrophils (C). After 4h incubation, cell viability was determined by luminescent measurement of cellular ATP levels. % inhibition of toxin activity was calculated using the following formula: % inhibition = [(normal activity - inhibited activity) / (normal activity)] x 100. A human IgG1 control mAb generated against an irrelevant antigen was included as control.

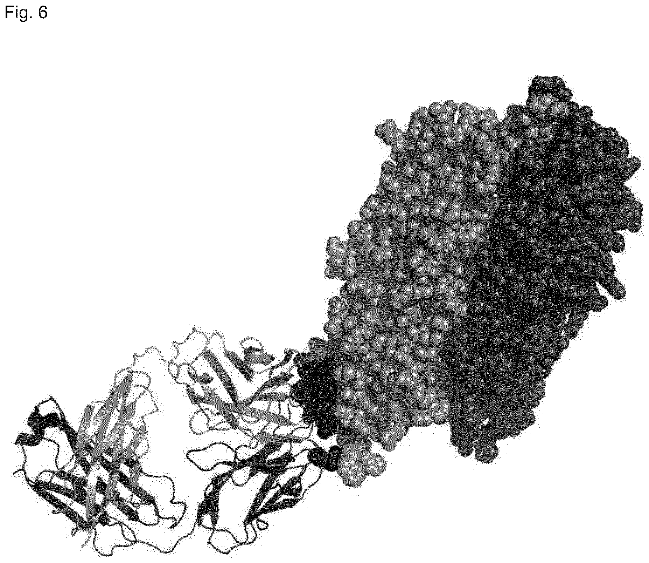

Figure 6: Structure of LukGH:AB-32-9 complex, with LukGH represented as grey spheres, contact residues as black spheres, and the Fab fragment of AB-32-9 as black cartoon for the light chain and grey carton for the heavy chain.

### DETAILED DESCRIPTION

The term "antibody" as used herein shall refer to polypeptides or proteins that consist of or comprise antibody domains, which are understood as constant and/or variable domains of the heavy and/or light chains of immunoglobulins, with or without a linker sequence. Polypeptides are understood as antibody domains, if comprising a beta-barrel structure consisting of at least two beta-strands of an antibody domain structure connected by a loop sequence. Antibody domains may be of native structure or modified by mutagenesis or derivatization, e.g. to modify the antigen binding properties or any other property, such as stability or functional properties, such as binding to the Fc receptors FcRn and/or Fcgamma receptor.

The antibody as used herein has a specific binding site to bind one or more antigens or one or more epitopes of such antigens, specifically comprising a CDR binding site of a single variable antibody domain, such as VH, VL or VHH, or a binding site of pairs of variable antibody domains, such as a VL/VH pair, an antibody comprising a VL/VH domain pair and constant antibody domains, such as Fab, F(ab'), (Fab)₂, scFv, Fv, or a full length antibody.

The term "antibody" as used herein shall particularly refer to antibody formats comprising or consisting of single variable antibody domain, such as VH, VL or VHH, or combinations of variable and/or constant antibody domains with or without a linking sequence or hinge region, including pairs of variable antibody domains, such as a VL/VH pair, an antibody comprising or consisting of a VL/VH domain pair and constant antibody domains, such as heavy-chain antibodies, Fab, F(ab'), (Fab)₂, scFv, Fd, Fv, or a full-length antibody, e.g. of an IgG type (e.g., an IgG1, IgG2, IgG3, or IgG4 subtype), IgA1, IgA2, IgD, IgE, or IgM antibody. The term "full length antibody" can be used to refer to any antibody molecule comprising at least most of the Fc domain and other domains commonly found in a naturally occurring antibody monomer. This phrase is used herein to emphasize that a particular antibody molecule is not an antibody fragment.

The term "antibody" shall specifically include antibodies in the isolated form, which are substantially free of other antibodies directed against different target antigens or comprising a different structural arrangement of antibody domains. Still, an isolated antibody may be comprised in a combination preparation, containing a combination of the isolated antibody, e.g. with at least one other antibody, such as monoclonal antibodies or antibody fragments having different specificities.

The term "antibody" shall apply to antibodies of animal origin, including human species, such as mammalian, including human, murine, rabbit, goat, lama, cow and horse, or avian, such as hen, which term shall particularly include recombinant antibodies which are based on a sequence of animal origin, e.g. human sequences.

The term "antibody" further applies to chimeric antibodies with sequences of origin of different species, such as sequences of murine and human origin.

The term "chimeric" as used with respect to an antibody refers to those antibodies wherein one portion of each of the amino acid sequences of heavy and light chains is homologous to corresponding sequences in antibodies derived from a particular species or belonging to a particular class, while the remaining segment of the chain is homologous to corresponding sequences in another species or class. Typically the variable region of both light and heavy chains mimics the variable regions of antibodies derived from one species of mammals, while the constant portions are homologous to sequences of antibodies derived from another. For example, the variable region can be derived from presently known sources using readily available B-cells or hybridomas from non-human host organisms in combination with constant regions derived from, for example, human cell preparations.

The term "antibody" may further apply to humanized antibodies.

The term "humanized" as used with respect to an antibody refers to a molecule having an antigen binding site that is substantially derived from an immunoglobulin from a non-human species, wherein the remaining immunoglobulin structure of the molecule is based upon the structure and/or sequence of a human immunoglobulin. The antigen binding site may either comprise complete variable domains fused onto constant domains or only the complementarity determining regions (CDR) grafted onto appropriate framework regions in the variable domains. Antigen-binding sites may be wild-type or modified, e.g. by one or more amino acid substitutions, preferably modified to resemble human immunoglobulins more closely. Some forms of humanized antibodies preserve all CDR sequences (for example a humanized mouse antibody which contains all six CDRs from the mouse antibody). Other forms have one or more CDRs which are altered with respect to the original antibody.

The term "antibody" further applies to human antibodies.

The term "human" as used with respect to an antibody, is understood to include antibodies having variable and constant regions derived from human germline immunoglobulin sequences. The human antibody of the invention may include amino acid residues not encoded by human germline immunoglobulin sequences (e.g., mutations introduced by random or site-specific mutagenesis in vitro or by somatic mutation in vivo), for example in the CDRs. Human antibodies include antibodies isolated from human immunoglobulin libraries or from animals transgenic for one or more human immunoglobulin.

The term "antibody" specifically applies to antibodies of any class or subclass. Depending on the amino acid sequence of the constant domain of their heavy chains, antibodies can be assigned to the major classes of antibodies IgA, IgD, IgE, IgG, and IgM, and several of these may be further divided into subclasses (isotypes), e.g., IgG1, IgG2, IgG3, IgG4, IgA1, and IgA2.

The term further applies to monoclonal or polyclonal antibodies, specifically a recombinant antibody, which term includes all antibodies and antibody structures that are prepared, expressed, created or isolated by recombinant means, such as antibodies originating from animals, e.g. mammalians including human, that comprises genes or sequences from different origin, e.g. chimeric, humanized antibodies, or hybridoma derived antibodies. Further examples refer to antibodies isolated from a host cell transformed to express the antibody, or antibodies isolated from a recombinant, combinatorial library of antibodies or antibody domains, or antibodies prepared, expressed, created or isolated by any other means that involve splicing of antibody gene sequences to other DNA sequences.

It is understood that the term "antibody" also refers to derivatives of an antibody, in particular functionally active derivatives. An antibody derivative is understood as any combination of one or more antibody domains or antibodies and/ or a fusion protein, in which any domain of the antibody may be fused at any position of one or more other proteins, such as other antibodies, e.g. a binding structure comprising CDR loops, a receptor polypeptide, but also ligands, scaffold proteins, enzymes, toxins and the like. A derivative of the antibody may be obtained by association or binding to other substances by various chemical techniques such as covalent coupling, electrostatic interaction, disulphide bonding etc. The other substances bound to the antibody may be lipids, carbohydrates, nucleic acids, organic and inorganic molecules or any combination thereof (e.g. PEG, prodrugs or drugs). In a specific embodiment, the antibody is a derivative comprising an additional tag allowing specific interaction with a biologically acceptable compound. There is not a specific limitation with respect to the tag usable in the present invention, as far as it has no or tolerable negative impact on the binding of the antibody to its target. Examples of suitable tags include His-tag, Myc-tag, FLAG-tag, Strep-tag, Calmodulin-tag, GST-tag, MBP-tag, and S-tag. In another specific embodiment, the antibody is a derivative comprising a label. The term "label" as used herein refers to a detectable compound or composition which is conjugated directly or indirectly to the antibody so as to generate a "labeled" antibody. The label may be detectable by itself, e.g. radioisotope labels or fluorescent labels, or, in the case of an enzymatic label, may catalyze chemical alteration of a substrate compound or composition which is detectable.

The preferred derivatives as described herein are functionally active with regard to the antigen binding, and alike the antibodies that are not derivatized, preferably have a potency to neutralize *S. aureus* and/or which are protective antibodies.

Antibodies derived from a parent antibody or antibody sequence, such as a parent CDR or FR sequence, are herein particularly understood as mutants or variants obtained by e.g. *in silico* or recombinant engineering or else by chemical derivatization or synthesis.

Specifically, an antibody derived from an antibody described herein may comprise at least one or more of the CDR regions or CDR variants thereof, e.g. at least 3 CDRs of the heavy chain variable region and/or at least 3 CDRs of the light chain variable region, with at least one point mutation in at least one of the CDR or FR regions, or in the constant region of the HC or LC, being functionally active, e.g. specifically binding the epitope formed by assembly of the individual LukG and LukH toxins, thus, an epitope of the LukGH dimer.

It is understood that the term "antibody" also refers to variants of an antibody, including antibodies with functionally active CDR variants of a parent CDR sequence, and functionally active variant antibodies of a parent antibody.

The term "variant" shall particularly refer to antibodies, such as mutant antibodies or fragments of antibodies, e.g. obtained by mutagenesis methods, in particular to delete, exchange, introduce inserts into a specific antibody amino acid sequence or region or chemically derivatize an amino acid sequence, e.g. in the constant domains to engineer the antibody stability, effector function or half-life, or in the variable domains to improve antigen-binding properties, e.g. by affinity maturation techniques available in the art. Any of the known mutagenesis methods may be employed, including point mutations at desired positions, e.g. obtained by randomization techniques. In some cases positions are chosen randomly, e.g. with either any of the possible amino acids or a selection of preferred amino acids to randomize the antibody sequences. The term "mutagenesis" refers to any art recognized technique for altering a polynucleotide or polypeptide sequence. Preferred types of mutagenesis include error prone PCR mutagenesis, saturation mutagenesis, or other site directed mutagenesis.

The term "variant" shall specifically encompass functionally active variants.

The term "functionally active variant" of a CDR sequence as used herein, is understood as a "functionally active CDR variant", and the "functionally active variant" of an antibody as used herein, is understood as "functionally active antibody variant". The functionally active variant means a sequence resulting from modification of this sequence (a parent antibody or a parent sequence) by insertion, deletion or substitution of one or more amino acids, or chemical derivatization of one or more amino acid residues in the amino acid sequence, or nucleotides within the nucleotide sequence, or at either or both of the distal ends of the sequence, e.g. in a CDR sequence the N-terminal and/or C-terminal 1, 2, 3, or 4 amino acids, and/or the centric 1, 2, 3, or 4 amino acids (i.e. in the midst of the CDR sequence), and which modification does not affect, in particular impair, the activity of this sequence. In the case of a binding site having specificity to a selected target antigen, the functionally active variant of an antibody would still have the predetermined binding specificity, though this could be changed, e.g. to change the fine specificity to a specific epitope, the affinity, the avidity, the Kon or Koff rate, etc. For example, an affinity matured antibody is specifically understood as a functionally active variant antibody. Hence, the modified CDR sequence in an affinity matured antibody is understood as a functionally active CDR variant. Further modifications may be made through mutagenesis, e.g. to widen the cross-specificity to target more variants of the LukGH heterodimer or the LukGH dimer, or further toxins or toxin components than the parent antibody, or to increase its reactivity.

Specifically, the functionally active variants of an antibody described herein has the binding site that binds to the LukGH complex or LukGH dimer, as further described herein. A further indicator of functional activity shall be the competitive binding of any of the LukG, LukH or LukGH heterodimer to the cell membranes, in particular to phosphocholine.

The functional activity is preferably determined in an assay for determining the neutralization potency of antibodies against cytolytic toxins, e.g. determined in a standard assay by measuring increased viability or functionality of cells susceptible to the given toxin. For example, the functional activity is determined if the antibody exhibits *in vitro* neutralization potency in a cell-based assay with an IC50 of less than 100:1 mAb:toxin ratio (mol/mol), preferably less than 50:1, preferably less than 25:1, preferably less than 10:1, more preferably less than 1:1. Neutralization is typically expressed by percent of viable cells with and without antibodies. For highly potent antibodies, a preferred way of expressing neutralization potency is the antibody:toxin molar ratio, where lower values correspond to higher potency. Values below 10 define a high functional activity. Optionally, values are in the most stringent assay between 1 and 10.

Functionally active variants may be obtained, e.g. by changing the sequence of a parent antibody, e.g. an antibody as listed in Table 1 (Figure 1) or in Figure 2, comprising the binding site, i.e. the binding site formed by the CDR region, or formed by the VH and the VL region, which parent antibody or sequence is characterized by its specific binding to LukGH, but with modifications within an antibody region besides the binding site, or derived from such parent antibody, by a modification within the binding site, that does not impair the antigen binding, and preferably would have a biological activity similar to the parent antibody, including the ability to bind toxins of *S. aureus* and/or to neutralize *S. aureus* with a specific potency, e.g. with substantially the same biological activity, as determined by a specific binding assay or functional test to target *S. aureus* or *S. aureus* toxins.

Specifically, any of the following CDR sequences, or one or more of the following CDR sequences may be modified to include the following
a) in VH CDR1 at position 7, the amino acid residue is selected from S, A, D, E, F, G, H, I, K, L, M, N, Q, R, T, V, W, and Y, preferentially any of E, F, H, I, K, L, M, R, V, W or Y, and more preferentially is any of E, F, M, W or Y; and/or
b) in VH CDR2 at position 1, the amino acid residue is selected from N, A, D, E, F, H, L, S, T, V and Y, preferentially any of F, H or Y; and/or
c) in VH CDR2 at position 3, the amino acid residue is selected from Y, H, T and W; and/or
d) in VH CDR2 at position 5, the amino acid residue is selected from S, A, E, F, H, I, K, L, M, N, Q, R, T, V, W and Y, preferentially any of N, R or W, and more preferentially is N or W; and/or
e) in VH CDR2 at position 7, the amino acid residue is selected from S, D, F, H, K, L, M, N, R and W; and/or
f) in VH CDR2 at position 9, the amino acid residue is selected from Y, D, E, F, N, S and W, preferentially D or H, and more preferentially is H; and/or
g) in VH CDR3 at position 4, the amino acid residue is selected from R, A, D, E, F, G, H, I, K, L, M, N, Q, S, T, V and W, preferentially D or H; and/or
h) in VH CDR3 at position 5, the amino acid residue is selected from G, A, F and Y; and/or
i) in VH CDR3 at position 6, the amino acid residue is selected from M, E, F, H and Q, preferentially F or H; and/or
j) in VH CDR3 at position 7, the amino acid residue is selected from H, A, D, E, F, G, I, K, L, M, N, Q, R, S, T, W and Y, preferentially any of E, K, Q, R, W or Y, and more preferentially is W or Y; and/or
k) in VL CDR4 at position 7, the amino acid residue is selected from the group consisting of N, A, D, E, F, G, H, K, L, M, Q, R, S, W and Y, preferentially any of F, L, W, or Y, and more preferentially is L or W; and/or
l) in VL CDR4 at position 8, the amino acid residue is selected from S, A, D, E, F, G, H, I, K, L, M, N, Q, R, T, V, W, and Y, preferentially I or W; and/or
m) in VL CDR4 at position 9, the amino acid residue is selected from Y, F, R and W, and preferentially R or W; and/or
n) in VL CDR5 at position 1, the amino acid residue is selected from A, G, S, W and Y, and preferentially is G; and/or
o) in VL CDR6 at position 4, the amino acid residue is selected from F, H, M, W and Y; and/or
p) in VL CDR6 at position 5, the amino acid residue is selected from D, A, D, E, F, G, H, I, K, L, M, N, Q, R, S, T, V, W, and Y; and/or
q) in VL CDR6 at position 8, the amino acid residue is selected from F, H, R and W.

The term "substantially the same biological activity" as used herein refers to the activity as indicated by substantially the same activity being at least 20%, at least 50%, at least 75%, at least 90%, e.g. at least 100%, or at least 125%, or at least 150%, or at least 175%, or e.g. up to 200% of the activity as determined for the comparable or parent antibody.

The preferred variants or derivatives as described herein are functionally active with regard to the antigen binding, preferably which have a potency to specifically bind the individual toxins, and not significantly binding to other antigens that are not target toxins, e.g. with a Kd value difference of at least 2 logs, preferably at least 3 logs. The antigen binding by a functionally active variant is typically not impaired, corresponding to about substantially the same binding affinity as the parent antibody or sequence, or antibody comprising a sequence variant, e.g. with a Kd value difference of less than 2 logs, preferably less than 3 logs, however, with the possibility of even improved affinity, e.g. with a Kd value difference of at least 1 log, preferably at least 2 logs.

Functional activity as determined by the specific targeting of the LukGH heterodimer is specifically further characterized by the preferential binding of the LukGH heterodimer over the individual toxins LukG and LukH. It specifically can be demonstrated that binding of the antibody described herein to the heterodimeric or oligomeric LukGH antigen is far improved as compared to binding of any of or both of the separated (monomeric) LukG or LukH, e.g. as characterized by a differential affinity or Kd of at least 1 or 2 logs difference.

In a preferred embodiment the functionally active variant of a parent antibody
a) is a biologically active fragment of the antibody, the fragment comprising at least 50% of the sequence of the molecule, preferably at least 60%, at least 70%, at least 80%, at least 90%, or at least 95% and most preferably at least 97%, 98% or 99%;
b) is derived from the antibody by at least one amino acid substitution, addition and/or deletion, wherein the functionally active variant has a sequence identity to the molecule or part of it, such as an antibody of at least 50% sequence identity, preferably at least 60%, more preferably at least 70%, more preferably at least 80%, still more preferably at least 90%, even more preferably at least 95% and most preferably at least 97%, 98% or 99%; and/or
c) consists of the antibody or a functionally active variant thereof and additionally at least one amino acid or nucleotide heterologous to the polypeptide or the nucleotide sequence.

According to a preferred aspect, the functionally active variant of an antibody described herein is essentially identical to the variant described above, but differs from its polypeptide or the nucleotide sequence, respectively, in that it is derived from a homologous sequence of a different species. These are referred to as naturally occurring variants or analogs.

The term "functionally active variant" also includes naturally occurring allelic variants, as well as mutants or any other non-naturally occurring variants. As is known in the art, an allelic variant is an alternate form of a (poly) peptide that is characterized as having a substitution, deletion, or addition of one or more amino acids that does essentially not alter the biological function of the polypeptide.

Functionally active variants may be obtained by sequence alterations in the polypeptide or the nucleotide sequence, e.g. by one or more point mutations, wherein the sequence alterations retains or improves a function of the unaltered polypeptide or the nucleotide sequence, when used in combination. Such sequence alterations can include, but are not limited to, (conservative) substitutions, additions, deletions, mutations and insertions.

Specific functionally active variants are CDR variants. A CDR variant includes an amino acid sequence modified by at least one amino acid in the CDR region, wherein said modification can be a chemical or a partial alteration of the amino acid sequence, which modification permits the variant to retain the biological characteristics of the unmodified sequence. A partial alteration of the CDR amino acid sequence may be by deletion or substitution of one to several amino acids, e.g. 1, 2, 3, 4 or 5 amino acids, or by addition or insertion of one to several amino acids, e.g. 1, 2, 3, 4 or 5 amino acids, or by a chemical derivatization of one to several amino acids, e.g. 1, 2, 3, 4 or 5 amino acids, or combination thereof. The substitutions in amino acid residues may be conservative substitutions, for example, substituting one hydrophobic amino acid for an alternative hydrophobic amino acid.

Conservative substitutions are those that take place within a family of amino acids that are related in their side chains and chemical properties. Examples of such families are amino acids with basic side chains, with acidic side chains, with non-polar aliphatic side chains, with non-polar aromatic side chains, with uncharged polar side chains, with small side chains, with large side chains etc.

A point mutation is particularly understood as the engineering of a polynucleotide that results in the expression of an amino acid sequence that differs from the non-engineered amino acid sequence in the substitution or exchange, deletion or insertion of one or more single (non-consecutive) or doublets of amino acids for different amino acids.

Preferred point mutations refer to the exchange of amino acids of the same polarity and/or charge. In this regard, amino acids refer to twenty naturally occurring amino acids encoded by sixty-four triplet codons. These 20 amino acids can be split into those that have neutral charges, positive charges, and negative charges:
The "neutral" amino acids are shown below along with their respective three-letter and single-letter code and polarity:
   Alanine: (Ala, A) nonpolar, neutral;
   Asparagine: (Asn, N) polar, neutral;
   Cysteine: (Cys, C) nonpolar, neutral;
   Glutamine: (Gln, Q) polar, neutral;
   Glycine: (Gly, G) nonpolar, neutral;
   Isoleucine: (Ile, I) nonpolar, neutral;
   Leucine: (Leu, L) nonpolar, neutral;
   Methionine: (Met, M) nonpolar, neutral;
   Phenylalanine: (Phe, F) nonpolar, neutral;
   Proline: (Pro, P) nonpolar, neutral;
   Serine: (Ser, S) polar, neutral;
   Threonine: (Thr, T) polar, neutral;
   Tryptophan: (Trp, W) nonpolar, neutral;
   Tyrosine: (Tyr, Y) polar, neutral;
   Valine: (Val, V) nonpolar, neutral; and
   Histidine: (His, H) polar, positive (10%) neutral (90%).
The "positively" charged amino acids are:
   Arginine: (Arg, R) polar, positive; and
   Lysine: (Lys, K) polar, positive.
The "negatively" charged amino acids are:
   Aspartic acid: (Asp, D) polar, negative; and
   Glutamic acid: (Glu, E) polar, negative.

"Percent (%) amino acid sequence identity" with respect to the antibody sequences and homologs described herein is defined as the percentage of amino acid residues in a candidate sequence that are identical with the amino acid residues in the specific polypeptide sequence, after aligning the sequence and introducing gaps, if necessary, to achieve the maximum percent sequence identity, and not considering any conservative substitutions as part of the sequence identity. Those skilled in the art can determine appropriate parameters for measuring alignment, including any algorithms needed to achieve maximal alignment over the full length of the sequences being compared.

An antibody variant is specifically understood to include homologs, analogs, fragments, modifications or variants with a specific glycosylation pattern, e.g. produced by glycoengineering, which are functional and may serve as functional equivalents, e.g. binding to the specific targets and with functional properties. The preferred variants as described herein are functionally active with regard to the antigen binding, preferably which have a potency to neutralize *S. aureus* and/or which are protective antibodies.

An antibody described herein may or may not exhibit Fc effector function. Though the mode of action is mainly mediated by neutralizing antibodies without Fc effector functions, Fc can recruit complement and aid elimination of the target antigen, such as a toxin, from the circulation via formation of immune complexes.

Specific antibodies may be devoid of an active Fc moiety, thus, either composed of antibody domains that do not contain an Fc part of an antibody or that do not contain an Fcgamma receptor binding site, or comprising antibody domains lacking Fc effector function, e.g. by modifications to reduce Fc effector functions, in particular to abrogate or reduce ADCC and/or CDC activity. Alternative antibodies may be engineered to incorporate modifications to increase Fc effector functions, in particular to enhance ADCC and/or CDC activity.

Such modifications may be effected by mutagenesis, e.g. mutations in the Fcgamma receptor binding site or by derivatives or agents to interfere with ADCC and/or CDC activity of an antibody format, so to achieve reduction or increase of Fc effector function.

A significant reduction of Fc effector function is typically understood to refer to Fc effector function of less than 10% of the unmodified (wild-type) format, preferably less than 5%, as measured by ADCC and/or CDC activity.

A significant increase of Fc effector function is typically understood to refer to an increase in Fc effector function of at least 10% of the unmodified (wild-type) format, preferably at least 20%, 30%, 40% or 50%, as measured by ADCC and/or CDC activity.

The term "glycoengineered" variants with respect to antibody sequences shall refer to glycosylation variants having modified immunogenic or immunomodulatory (e.g. anti-inflammatory) properties, ADCC and/ or CDC, as a result of the glycoengineering. All antibodies contain carbohydrate structures at conserved positions in the heavy chain constant regions, with each isotype possessing a distinct array of N-linked carbohydrate structures, which variably affect protein assembly, secretion or functional activity. IgG1 type antibodies are glycoproteins that have a conserved N linked glycosylation site at Asn297 in each CH2 domain. The two complex bi-antennary oligosaccharides attached to Asn297 are buried between the CH2 domains, forming extensive contacts with the polypeptide backbone, and their presence is essential for the antibody to mediate effector functions such as antibody dependent cellular cytotoxicity (ADCC). Removal of N-Glycan at N297, e.g. through mutating N297, e.g. to A, or T299 typically results in aglycosylated antibody formats with reduced ADCC. Specifically, the antibody described herein may be glycosylated or glycoengineered, or aglycosylated antibodies.

Major differences in antibody glycosylation occur between cell lines, and even minor differences are seen for a given cell line grown under different culture conditions. Expression in bacterial cells typically provides for an aglycosylated antibody. CHO cells with tetracycline-regulated expression of β(1,4)-N-acetylglucosaminyltransferase III (GnTIII), a glycosyltransferase catalyzing formation of bisecting GlcNAc, was reported to have improved ADCC activity (Umana et al., 1999, Nature Biotech. 17:176-180). In addition to the choice of host cells, factors that affect glycosylation during recombinant production of antibodies include growth mode, media formulation, culture density, oxygenation, pH, purification schemes and the like.

The term "antigen-binding site" or "binding site" refers to the part of an antibody that participates in antigen binding. The antigen binding site is formed by amino acid residues of the N-terminal variable ("V") regions of the heavy ("H") and/or light ("L") chains, or the variable domains thereof. Three highly divergent stretches within the V regions of the heavy and light chains, referred to as "hypervariable regions", are interposed between more conserved flanking stretches known as framework regions, The antigen-binding site provides for a surface that is complementary to the three-dimensional surface of a bound epitope or antigen, and the hypervariable regions are referred to as "complementarity-determining regions", or "CDRs." The binding site incorporated in the CDRs is herein also called "CDR binding site".

Specifically, the CDR sequences as referred to herein are understood as those amino acid sequences of an antibody as determined according to Kabat nomenclature (see Kabat et al., Sequences of Proteins of Immunological Interest, 5th Ed. Public Health Service, U.S. Department of Health and Human Services. (1991)).

The term "antigen" as used herein interchangeably with the terms "target" or "target antigen" shall refer to a whole target molecule or a fragment of such molecule recognized by an antibody binding site. Specifically, substructures of an antigen, e.g. a polypeptide or carbohydrate structure, generally referred to as "epitopes", e.g. B-cell epitopes or T-cell epitope, which are immunologically relevant, may be recognized by such binding site.

The term "epitope" as used herein shall in particular refer to a molecular structure which may completely make up a specific binding partner or be part of a specific binding partner to a binding site of an antibody. An epitope may either be composed of a carbohydrate, a peptidic structure, a fatty acid, an organic, biochemical or inorganic substance or derivatives thereof and any combinations thereof. If an epitope is comprised in a peptidic structure, such as a peptide, a polypeptide or a protein, it will usually include at least 3 amino acids, preferably 5 to 40 amino acids, and more preferably between about 10-20 amino acids. Epitopes can be either linear or conformational epitopes. A linear epitope is comprised of a single segment of a primary sequence of a polypeptide or carbohydrate chain. Linear epitopes can be contiguous or overlapping.

Conformational epitopes are comprised of amino acids or carbohydrates brought together by folding the polypeptide to form a tertiary structure and the amino acids are not necessarily adjacent to one another in the linear sequence. Specifically and with regard to polypeptide antigens a conformational or discontinuous epitope is characterized by the presence of two or more discrete amino acid residues, separated in the primary sequence, but assembling to a consistent structure on the surface of the molecule when the polypeptide folds into the native protein/antigen. Specifically, the conformational epitope is an epitope which is comprised of a series of amino acid residues which are non-linear in alignment that is that the residues are spaced or grouped in a non-continuous manner along the length of a polypeptide sequence. Such conformational epitope is characterized by a three-dimensional structure with specific structure coordinates as determined by contacting amino acid residues and/or crystallographic analysis, e.g. analysis of a crystal formed by the immune complex of the epitope bound by a specific antibody or Fab fragment.

In particular, the binding residues which contribute to an epitope are herein understood as the contacting amino acid residues.

The structure coordinates are typically understood as Cartesian atomic coordinates derived from mathematical equations related to the patterns obtained on diffraction of a monochromatic beam of X-rays by the atoms, i.e. scattering centres, of the antigen or the immune complex in a crystal form. The diffraction data are used to calculate an electron density map of the repeating unit of the crystal. The electron density maps are then used to establish the individual atoms of the epitope, e.g. as bound by the specific antibody or Fab. It is understood that a set of structure coordinates for an antigen is a relative set of points that define a shape in three dimensions. Slight variations in the individual coordinates will have little effect on overall shape. For the purpose described herein, any three- dimensional structure that has a root mean square deviation of conserved residue backbone atoms between 0.00 A and 2.00 A when superimposed on the relevant backbone atoms described by the structure coordinates of the antigen are considered identical or substantially identical. For the purpose described herein, structure coordinates are considered identical or substantially identical even if slight variations are present in the individual coordinates if these do not affect the overall shape defined by the structure coordinates.

The antibody as described herein is specifically recognizing the rim domain of the LukG toxin, in particular the LukG as complexed with the LukH toxin to form the LukGH heterodimer. The rim domain is understood as the domain of the toxin that is juxtaposed to the outer leaflet of the host plasma membrane, which rim domain is involved in cell membrane binding. Thus, the rim region serves as a membrane anchor. The epitope targeted by the antibody of the invention, which is located in the rim region or the rim domain, thus, has the potential of being immunorelevant, i.e. relevant for protection by active or passive immunotherapy.

Based on the epitope identified herein, it is feasible to additionally provide for a respective paratope, e.g. the paratope of an antibody of the invention, such as the part of a full length antibody which recognizes the epitope, the antigen-binding site of an antibody. It is typically a small region of 15-22 amino acids of the antibody's Fv region. Such paratope may be incorporated in a suitable scaffold to obtain a scaffold-type molecule, e.g. a fusion protein or artificial scaffolds, to obtain a specific binder or binding molecule with the desired cross-reactive binding characteristics.

The term "binding molecule" as used herein is understood as an epitope-binding molecule or an antigen-binding molecule that specifically recognizes the target, and in particular exhibiting cross-specificity to the target toxins. Specific examples of binding molecules are selected from the group consisting of a protein, a peptide, a peptidomimetic, a nucleic acid, a carbohydrate, a lipid, an oligopeptide, an aptamer and a small molecule compound, preferably an antibody, an antibody fragment thereof comprising at least one antibody domain incorporating the binding site, or a fusion protein comprising at least one antibody domain incorporating the binding site.

A binding molecule may e.g. be selected from suitable libraries of binders, e.g. antibody libraries, or libraries of other compounds or scaffolds, e.g. DARPins, HEAT repeat proteins, ARM repeat proteins, tetratricopeptide repeat proteins, and other scaffolds based on naturally occurring repeat proteins, by suitable screening methods to obtain a candidate compound, which is then further characterized for its binding characteristics.

The term "expression" is understood in the following way. Nucleic acid molecules containing a desired coding sequence of an expression product such as e.g. an antibody as described herein, and control sequences such as e.g. a promoter in operable linkage, may be used for expression purposes. Hosts transformed or transfected with these sequences are capable of producing the encoded proteins. In order to effect transformation, the expression system may be included in a vector; however, the relevant DNA may also be integrated into the host chromosome. Specifically the term refers to a host cell and compatible vector under suitable conditions, e.g. for the expression of a protein coded for by foreign DNA carried by the vector and introduced to the host cell.

Coding DNA is a DNA sequence that encodes a particular amino acid sequence for a particular polypeptide or protein such as e.g. an antibody. Promoter DNA is a DNA sequence which initiates, regulates, or otherwise mediates or controls the expression of the coding DNA. Promoter DNA and coding DNA may be from the same gene or from different genes, and may be from the same or different organisms. Recombinant cloning vectors will often include one or more replication systems for cloning or expression, one or more markers for selection in the host, e.g. antibiotic resistance, and one or more expression cassettes.

"Vectors" used herein are defined as DNA sequences that are required for the transcription of cloned recombinant nucleotide sequences, i.e. of recombinant genes and the translation of their mRNA in a suitable host organism.

An "expression cassette" refers to a DNA coding sequence or segment of DNA that code for an expression product that can be inserted into a vector at defined restriction sites. The cassette restriction sites are designed to ensure insertion of the cassette in the proper reading frame. Generally, foreign DNA is inserted at one or more restriction sites of the vector DNA, and then is carried by the vector into a host cell along with the transmissible vector DNA. A segment or sequence of DNA having inserted or added DNA, such as an expression vector, can also be called a "DNA construct".

Expression vectors comprise the expression cassette and additionally usually comprise an origin for autonomous replication in the host cells or a genome integration site, one or more selectable markers (e.g. an amino acid synthesis gene or a gene conferring resistance to antibiotics such as zeocin, kanamycin, G418 or hygromycin), a number of restriction enzyme cleavage sites, a suitable promoter sequence and a transcription terminator, which components are operably linked together. The term "vector" as used herein includes autonomously replicating nucleotide sequences as well as genome integrating nucleotide sequences. A common type of vector is a "plasmid", which generally is a self-contained molecule of double-stranded DNA that can readily accept additional (foreign) DNA and which can readily be introduced into a suitable host cell. A plasmid vector often contains coding DNA and promoter DNA and has one or more restriction sites suitable for inserting foreign DNA. Specifically, the term "vector" or "plasmid" refers to a vehicle by which a DNA or RNA sequence (e.g. a foreign gene) can be introduced into a host cell, so as to transform the host and promote expression (e.g. transcription and translation) of the introduced sequence.

The term "host cell" as used herein shall refer to primary subject cells transformed to produce a particular recombinant protein, such as an antibody as described herein, and any progeny thereof. It should be understood that not all progeny are exactly identical to the parental cell (due to deliberate or inadvertent mutations or differences in environment), however, such altered progeny are included in these terms, so long as the progeny retain the same functionality as that of the originally transformed cell. The term "host cell line" refers to a cell line of host cells as used for expressing a recombinant gene to produce recombinant polypeptides such as recombinant antibodies. The term "cell line" as used herein refers to an established clone of a particular cell type that has acquired the ability to proliferate over a prolonged period of time. Such host cell or host cell line may be maintained in cell culture and/or cultivated to produce a recombinant polypeptide.

An "immune response" to a composition, e.g. an immunogenic composition, herein also termed "immunogen" comprising an antigen or epitope, or a vaccine as described herein is the development in the host or subject of a cellular- and/or antibody-mediated immune response to the composition or vaccine of interest. Usually, such a response consists of the subject producing antibodies, B cells, helper T cells, suppressor T cells, and/or cytotoxic T cells directed specifically to an antigen or antigens included in the composition or vaccine of interest.

A "protective immune response" is understood as an immune response that provides a significantly better outcome of an induced or natural infection or toxin challenge in comparison to that of the non-immune population. Protective immune response against toxins is mainly mediated by neutralizing antibodies having high affinity, e.g. with a Kd of less than 10⁻⁸M. The benefit of neutralization of toxins is the protection of targets cells and prevention of inflammation. Fc mediated immune complex formation can contribute as well by removing the toxin from the circulation (via the RES cells).

An immunogen or immunogenic composition usually comprises the antigen or epitope and a carrier, which may specifically comprise an adjuvant. The term "adjuvant" refers to a compound that when administered in conjunction with an antigen augments and/or redirects the immune response to the antigen, but when administered alone does not generate an immune response to the antigen. Adjuvants can augment an immune response by several mechanisms including lymphocyte recruitment, stimulation of B and/or T cells, and stimulation of macrophages. Exemplary carriers are liposomes or cationic peptides; exemplary adjuvants are aluminium phosphate or aluminium hydroxide, MF59 or CpG oligonucleotide.

The term "isolated" or "isolation" as used herein with respect to a nucleic acid, an antibody or other compound shall refer to such compound that has been sufficiently separated from the environment with which it would naturally be associated, so as to exist in "substantially pure" form. "Isolated" does not necessarily mean the exclusion of artificial or synthetic mixtures with other compounds or materials, or the presence of impurities that do not interfere with the fundamental activity, and that may be present, for example, due to incomplete purification. In particular, isolated nucleic acid molecules of the present invention are also meant to include those which are not naturally occurring, e.g. codon-optimized nucleic acids or cDNA, or chemically synthesized.

Likewise, the isolated antibody is specifically non-naturally occurring, e.g. as provided in a combination preparation with another antibody, which combination does not occur in nature (such as a combination with one or more monospecific antibody and/or with a cross-specific antibody which recognizes at least two different targets), or an optimized or affinity-maturated variant of a naturally occurring antibody, or an antibody with a framework-region which is engineered to improve the manufacturability of the antibody.

With reference to nucleic acids referred to herein, the term "isolated nucleic acid" is sometimes used. This term, when applied to DNA, refers to a DNA molecule that is separated from sequences with which it is immediately contiguous in the naturally occurring genome of the organism in which it originated. For example, an "isolated nucleic acid" may comprise a DNA molecule inserted into a vector, such as a plasmid or virus vector, or integrated into the genomic DNA of a prokaryotic or eukaryotic cell or host organism. When applied to RNA, the term "isolated nucleic acid" refers primarily to an RNA molecule encoded by an isolated DNA molecule as defined above. Alternatively, the term may refer to an RNA molecule that has been sufficiently separated from other nucleic acids with which it would be associated in its natural state (i.e., in cells or tissues). An "isolated nucleic acid" (either DNA or RNA) may further represent a molecule produced directly by biological or synthetic means and separated from other components present during its production.

With reference to polypeptides or proteins, such as isolated antibodies or epitopes described herein, the term "isolated" shall specifically refer to compounds that are free or substantially free of material with which they are naturally associated such as other compounds with which they are found in their natural environment, or the environment in which they are prepared (e g. cell culture) when such preparation is by recombinant DNA technology practiced *in vitro* or *in vivo.* Isolated compounds can be formulated with diluents or adjuvants and still for practical purposes be isolated - for example, the polypeptides or polynucleotides can be mixed with pharmaceutically acceptable carriers or excipients when used in diagnosis or therapy.

The term "LukGH complex" as used herein shall refer to the dimer or oligomer, including 1:1 dimers or any other ratio of the LukG and the LukH components, preferably a complex comprising at least 1 LukG and at least 1 LukH component, or at least 2, or at least 3, or at least 4 of any of the LukG or LukH components or of both LukG and LukH components. The LukGH dimer is herein understood as a heterodimer of one molecule LukG and one molecule LukH, which assemble in solution, specifically by electrostatic or hydrophilic/hydrophobic interactions. It was surprisingly found that LukH and LukG forms a complex in solution without being in contact with target cells

The term "neutralizing" or "neutralization" is used herein in the broadest sense and refers to any molecule that inhibits a pathogen, such as *S. aureus* from infecting a subject, or to inhibit the pathogen from promoting infections by producing potent protein toxins, or to inhibit the toxins from damaging a target cell in a subject, irrespective of the mechanism by which neutralization is achieved. Neutralization can be achieved, e.g., by an antibody that inhibits the binding and/or interaction of the *S. aureus* toxin(s) with its cognate receptor on target cells. In certain embodiments, the antibodies described herein can neutralize the toxin activity wherein the *in vivo* or *in vitro* effects of the interaction between the toxin and the target cell, such as red blood cells are reduced or eliminated. Neutralization can further occur by inhibition of forming active toxin, for example in the case of the *S. aureus* LukGH complex by inhibition of binding of the LukG and LukF antigens to each other, or inhibition of binding the LukGH dimer or oligomer to a target cell, or inhibition of formation of the oligomeric pores in cytomembranes.

The neutralization potency of antibodies against cytolytic toxins is typically determined in a standard assay by measuring increased viability or functionality of cells susceptible to the given toxin. Neutralization can be expressed by percent of viable cells with and without antibodies. For highly potent antibodies, a preferred way of expressing neutralization potency is the antibody:toxin molar ratio, where lower values correspond to higher potency. Values below 10 define high, while values below 1 define very high potency.

The term "cross-neutralizing" as used herein shall refer to neutralizing a number of toxins, e.g. toxins incorporating an epitope of the LukGH complex of at least two, preferably at least three, or at least four or at least three different *S. aureus* strains expressing different LukGH variants, which epitope is recognized by the antibody of the invention. Such epitope prevalent on the different LukGH heterodimer variants is also called "cross-reactive" epitope.

The term *"Staphylococcus aureus"* or *"S. aureus"* or "pathogenic *S*. *aureus"* is understood in the following way. *Staphylococcus aureus* bacteria are normally found on the skin or in the nose of people and animals. The bacteria are generally harmless, unless they enter the body through a cut or other wound. Typically, infections are minor skin problems in healthy people. Historically, infections were treated by broad-spectrum antibiotics, such as methicillin. Now, though, certain strains have emerged that are resistant to methicillin and other beta-lactam antibiotics, such as penicillin and cephalosporins. They are referred to as methicillin-resistant *Staphylococcus aureus* (also known as multi-drug resistant *Staphylococcus aureus,* or "MRSA").

*Staphylococcus aureus,* an important human pathogen, expresses a multitude of secreted toxins (exotoxins). These can attack various host cell types, including erythrocytes, neutrophil granulocytes and other immune cells, as well as epithelial cells of the lung or skin. A prominent member of *S. aureus* toxins is alpha hemolysin (Hla), which exerts cytolytic function on lymphocytes, macrophages, lung epithelial cells and pulmonary endothelial cells.

*S. aureus* infections, including MRSA, generally start as small red bumps that resemble pimples, boils or spider bites. These bumps or blemishes can quickly turn into deep, painful abscesses that require surgical draining. Sometimes the bacteria remain confined to the skin. On occasion, they can burrow deep into the body, causing potentially life-threatening infections in a broad range of human tissue, including skin, soft tissue, bones, joints, surgical wounds, the bloodstream, heart valves, lungs, or other organs. Thus, *S. aureus* infections can result in disease conditions associated therewith, which are potentially fatal diseases, such as necrotizing fasciitis, endocarditis, sepsis, bacteremia, peritonitis, toxic shock syndrome, and various forms of pneumonia, including necrotizing pneumonia, and toxin production in furunculosis and carbunculosis. MRSA infection is especially troublesome in hospital or nursing home settings where patients are at risk of or prone to open wounds, invasive devices, and weakened immune systems and, thus, are at greater risk for infection than the general public.

Antibodies neutralizing *S*. *aureus* toxins are interfering with the pathogens and pathogenic reactions, thus able to limit or prevent infection and/ or to ameliorate a disease condition resulting from such infection, or to inhibit *S*. *aureus* pathogenesis, in particular pneumonia, peritonitis, osteomyelitis, bacteremia and sepsis pathogenesis. In this regard "protective antibodies" are understood herein as neutralizing antibodies that are responsible for immunity to an infectious agent observed in active or passive immunity. In particular, protective antibodies as described herein are able to neutralize toxic effects (such as cytolysis, induction of pro-inflammatory cytokine expression by target cells) of secreted virulence factors (exotoxins) and hence interfere with pathogenic potential of *S. aureus.*

The term "recombinant" as used herein shall mean "being prepared by or the result of genetic engineering". A recombinant host specifically comprises an expression vector or cloning vector, or it has been genetically engineered to contain a recombinant nucleic acid sequence, in particular employing nucleotide sequence foreign to the host. A recombinant protein is produced by expressing a respective recombinant nucleic acid in a host. The term "recombinant antibody", as used herein, includes antibodies that are prepared, expressed, created or isolated by recombinant means, such as (a) antibodies isolated from an animal (e.g., a mouse) that is transgenic or transchromosomal for human immunoglobulin genes or a hybridoma prepared therefrom, (b) antibodies isolated from a host cell transformed to express the antibody, e.g., from a transfectoma, (c) antibodies isolated from a recombinant, combinatorial human antibody library, and (d) antibodies prepared, expressed, created or isolated by any other means that involve splicing of human immunoglobulin gene sequences to other DNA sequences. Such recombinant antibodies comprise antibodies engineered to include rearrangements and mutations which occur, for example, during antibody maturation.

As used herein, the term "specificity" or "specific binding" refers to a binding reaction which is determinative of the cognate ligand of interest in a heterogeneous population of molecules. Thus, under designated conditions (e.g. immunoassay conditions), an antibody specifically binds to its particular target and does not bind in a significant amount to other molecules present in a sample. The specific binding means that binding is selective in terms of target identity, high, medium or low binding affinity or avidity, as selected. Selective binding is usually achieved if the binding constant or binding dynamics is at least 10 fold different (understood as at least 1 log difference), preferably the difference is at least 100 fold (understood as at least 2 logs difference), and more preferred a least 1000 fold (understood as at least 3 logs difference) as compared to another antigen.

The term "specificity" or "specific binding" is also understood to apply to binders which bind to one or more molecules, e.g. cross-specific binders. Preferred cross-specific (also called polyspecific or cross-reactive) binders targeting at least two different antigens or targeting a cross-reactive epitope on at least two different antigens, specifically bind the antigens with substantially similar binding affinity, e.g. with less than 100 fold difference or even less than 10 fold difference.

For example, a cross-specific antibody will be able to bind to the various antigens carrying a cross-reactive epitope. Such binding site of an antibody or and antibody with a specificity to bind at least two different antigens or a cross-reactive epitope of at least two different antigens is also called a polyspecific or cross-specific binding site and antibody, respectively. For example, an antibody may have a polyspecific binding site specifically binding an epitope cross-reactive a number of different antigens with sequence homology within the epitope and/or structural similarities to provide for a conformational epitope of essentially the same structure, e.g. cross-reactive at least two different LukGH complex variants.

The immunospeciffcity of an antibody, its binding capacity and the attendant affinity the antibody exhibits for a cross-reactive binding sequence, are determined by a cross-reactive binding sequence with which the antibody immunoreacts (binds). The cross-reactive binding sequence specificity can be defined, at least in part, by the amino acid residues of the variable region of the heavy chain of the immunoglobulin the antibody and/ or by the light chain variable region amino acid residue sequence.

Use of the term "having the same specificity", "having the same binding site" or "binding the same epitope" indicates that equivalent monoclonal antibodies exhibit the same or essentially the same, i.e. similar immunoreaction (binding) characteristics and compete for binding to a pre-selected target binding sequence. The relative specificity of an antibody molecule for a particular target can be relatively determined by competition assays, e.g. as described in Harlow, et al., ANTIBODIES: A LABORATORY MANUAL, Cold Spring Harbor Laboratory Press, Cold Spring Harbor, N.Y., 1988).

The term "subject" as used herein shall refer to a warm-blooded mammalian, particularly a human being or a non-human animal. MRSA is a critically important human pathogen that is also an emerging concern in veterinary medicine. It is present in a wide range of non-human animal species. Thus, the term "subject" may also particularly refer to animals including dogs, cats, rabbits, horses, cattle, pigs and poultry. In particular the medical use of the invention or the respective method of treatment applies to a subject in need of prophylaxis or treatment of a disease condition associated with a *S. aureus* infection. The subject may be a patient at risk of a *S: aureus* infection or suffering from disease, including early stage or late stage disease. The term "patient" includes human and other mammalian subjects that receive either prophylactic or therapeutic treatment. The term "treatment" is thus meant to include both prophylactic and therapeutic treatment.

A subject is e.g. treated for prophylaxis or therapy of *S. aureus* disease conditions. In particular, the subject is treated, which is either at risk of infection or developing such disease or disease recurrence, or a subject that is suffering from such infection and/ or disease associated with such infection.

Specifically the term "prophylaxis" refers to preventive measures which is intended to encompass prevention of the onset of pathogenesis or prophylactic measures to reduce the risk of pathogenesis.

Specifically, the method for treating, preventing, or delaying a disease condition in a subject as described herein, is by interfering with the pathogenesis of *S. aureus* as causal agent of the condition, wherein the pathogenesis includes a step of forming a pore on the subject's cellular membrane, e.g. by the specific virulence factors or toxins.

The term "toxin" as used herein shall refer to the alpha-toxin (Hla) and the bi-component toxins of *S. aureus.* It is specifically understood that the toxins targeted by the antibody described herein are either the toxins as such, e.g. the soluble monomeric toxins or in the form of the pore forming toxins as expressed by *S. aureus,* or toxin components, such as the components of the bi-component toxins. Therefore, the term "toxin" as used herein shall refer to both, the toxin or the toxin components bearing the immunorelevant epitope.

The virulence of *S. aureus* is due to a combination of numerous virulence factors, which include surface-associated proteins that allow the bacterium to adhere to eukaryotic cell membranes, a capsular polysaccharide that protects it from opsonophagocytosis, and several exotoxins. *S. aureus* causes disease mainly through the production of secreted virulence factors such as hemolysins, enterotoxins and toxic shock syndrome toxin. These secreted virulence factors suppress the immune response by inactivating many immunological mechanisms in the host, and cause tissue destruction and help establish the infection. The latter is accomplished by a group of pore forming toxins, the most prominent of which is Hla, a key virulence factor for *S. aureus* pneumonia.

*S. aureus* produces a diverse array of further virulence factors and toxins that enable this bacterium to counteract and withstand attack by different kinds of immune cells, specifically subpopulations of white blood cells that make up the body's primary defense system. The production of these virulence factors and toxins allow *S. aureus* to maintain an infectious state. Among these virulence factors, *S. aureus* produces the LukG and LukH leukocidin antigens.

The toxicity of the leukocidins towards mammalian cells involves the action of two components. The first subunit is named class S component, and the second subunit is named class F component. The S and F subunits act synergistically to form pores on white blood cells including monocytes, macrophages, dendritic cells and neutrophils (collectively known as phagocytes). The gamma hemolysins, especially HIgAB and HlgA-LukD also act on red blood cells and LukED on T cells. The repertoire of bi-component leukotoxins produced by *S. aureus* is known to include cognate and non-cognate pairs of the F and S components, e.g. gamma-hemolysins, PVL toxins and PVL-like toxins, including HlgAB, HlgCB, LukSF, LukED, LukGH, LukS-HIgB, LukSD, HIgA-LukD, HIgA-LukF, LukG-HIgA, LukEF, LukE-HIgB, HIgC-LukD or HIgC-LukF.

The term "substantially pure" or "purified" as used herein shall refer to a preparation comprising at least 50% (w/w), preferably at least 60%, 70%, 80%, 90% or 95% of a compound, such as a nucleic acid molecule or an antibody. Purity is measured by methods appropriate for the compound (e.g. chromatographic methods, polyacrylamide gel electrophoresis, HPLC analysis, and the like).

The term "therapeutically effective amount", used herein interchangeably with any of the terms "effective amount" or "sufficient amount" of a compound, e.g. an antibody or immunogen described herein, is a quantity or activity sufficient to, when administered to the subject effect beneficial or desired results, including clinical results, and, as such, an effective amount or synonym thereof depends upon the context in which it is being applied.

An effective amount is intended to mean that amount of a compound that is sufficient to treat, prevent or inhibit such diseases or disorder. In the context of disease, therapeutically effective amounts of the antibody as described herein are specifically used to treat, modulate, attenuate, reverse, or affect a disease or condition that benefits from an inhibition of *S. aureus* or *S. aureus* pathogenesis.

The amount of the compound that will correspond to such an effective amount will vary depending on various factors, such as the given drug or compound, the pharmaceutical formulation, the route of administration, the type of disease or disorder, the identity of the subject or host being treated, and the like, but can nevertheless be routinely determined by one skilled in the art.

The antibody or the immunogen described herein may be used prophylactically to inhibit onset of *S. aureus* infection, or therapeutically to treat *S. aureus* infection, particularly *S. aureus* infections such as MRSA that are known to be refractory or in the case of the specific subject, have proven refractory to treatment with other conventional antibiotic therapy.

A therapeutically effective amount of the antibody as described herein, such as provided to a human patient in need thereof, may specifically be in the range of 0.5-50 mg/kg, preferably 5-40 mg/kg, even more preferred up to 20 mg/kg, up to 10 mg/kg, up to 5 mg/kg, though higher doses may be indicated e.g. for treating acute disease conditions.

Moreover, a treatment or prevention regime of a subject with a therapeutically effective amount of the antibody described herein may consist of a single administration, or alternatively comprise a series of applications. For example, the antibody may be administered at least once a year, at least once a half-year or at least once a month. However, in another embodiment, the antibody may be administered to the subject from about one time per week to about a daily administration for a given treatment. The length of the treatment period depends on a variety of factors, such as the severity of the disease, either acute or chronic disease, the age of the patient, the concentration and the activity of the antibody format. It will also be appreciated that the effective dosage used for the treatment or prophylaxis may increase or decrease over the course of a particular treatment or prophylaxis regime. Changes in dosage may result and become apparent by standard diagnostic assays known in the art. In some instances, chronic administration may be required.

An effective amount of an immunogen as described herein, such as provided to a patient at risk of developing a disease condition associated with an *S. aureus* infection, may specifically be in the range of 1-20 mg/kg per dose.

For example, the immunogen may be administered as a first dose followed by one or more booster dose(s), within a certain timeframe, according to a prime-boost immunization scheme to induce a long-lasting, efficacious immune response to *S. aureus* infection. A preferred vaccination schedule would encompass administration of three doses, e.g. a first dose on day 0, a second dose on day 5-40, and a third dose on day 10-100, preferably on days 0, 28 and 90. According to a preferred accelerated schedule the administration may be on days 0,7 and 14. Accelerated schedules may be indicated for prophylaxis, e.g. for patients facing elective surgery. Usually alum is used as an adjuvant, e.g. as phosphate or hydroxide.

Further described herein are exemplary antibodies as detailed in the figures provided herein, and further antibody variants, in particular including variants binding to essentially the same epitope, as the parent antibody which is characterized by the specific binding site formed by the VH and the VL amino acid sequences, or else by the HC and the LC amino acid sequences of Figure 2, or the binding sites formed by such VH/VL domains. Such antibodies may e.g. be functionally active variant antibodies obtained by modifying the respective CDR or antibody sequence of the parent antibody. It is well understood that any antibody sequence as described herein is considered a "parent" sequence which is subject to variation, e.g. by point mutations.

Exemplary parent antibodies are described in the examples section below and in Figures 1 and 2. The antibodies designated #AB-29, #AB-30, #AB-31, #AB-32, #AB-33, #AB-34, #AB-35, and #AB-36, are e.g. used as a parent antibody to produce functionally active CDR variants with one or more modified CDR sequences, and functionally active antibody variants with one or more modified FR sequences, such as sequences of FR1, FR2, FR3 or FR4, or a constant domain sequence, and/or with one or more modified CDR sequences. The variant antibody derived from the parent antibody by mutagenesis are exemplified below (see Figure 1). These variant antibodies bind to the target antigen, thus, are considered functionally active. It is feasible that also variant VH or VL domains or variant HC or LC chains, e.g. with modifications in the respective FR or CDR sequences may be used, which are functionally active. It is also feasible that some of the FR or CDR sequences of the antibodies described herein may be exchanged by those of other antibodies, e.g. of antibodies as listed in Figure 1.

Specifically, any of the antibodies designated #AB-30-3, #AB-31, #AB-32-6, #AB-34, #AB-34-6, #AB-30-8, #AB-32-9, #AB-34-14, or #AB-34-15 or any functionally active variant thereof may be produced employing the sequences as provided herein by recombinant means, optionally with further immunoglobulin sequences, e.g. to produce an IgG antibody.

Further antibody variants are feasible, which comprise the same binding site. Specifically, there is provided an antibody comprising the variable region of the antibodies of Figure 2, or at least one of the CDR sequences, preferably at least two, at least 3, at least 4, at least 5 or at least six of the CDR sequences, or CDR variants thereof which are functionally active. More specifically, there is provided the antibody designated #AB-30-3, #AB-31, #AB-32-6, #AB-34, #AB-34-6, #AB-30-8, #AB-32-9, #AB-34-14, or #AB-34-15, which are characterized by the sequences provided in Figure 2. These antibodies are particularly useful to engineer functionally active antibody variants, e.g. to improve their affinity, stability or manufacturability.

Further described herein are such functionally active variant antibodies, preferably monoclonal antibodies, most preferably human antibodies, comprising a heavy chain and a light chain, wherein any of the heavy chain or VH variable region or the respective CDRs comprises an amino acid sequence as derived from a parent antibody, which is one of the antibodies designated #AB-30-3, #AB-31, #AB-32-6, #AB-34, #AB-34-6, #AB-30-8, #AB-32-9, #AB-34-14, or #AB-34-15, by modification of at least one FR or CDR sequences.

Further described herein are such functionally active variant antibodies, preferably monoclonal antibodies, most preferably human antibodies, comprising a heavy chain and a light chain, wherein any of the light chain or VL variable region or the respective CDRs comprises an amino acid sequence as derived from a parent antibody, which is one of the antibodies designated #AB-30-3, #AB-31, #AB-32-6, #AB-34, #AB-34-6, #AB-30-8, #AB-32-9, #AB-34-14, or #AB-34-15, by modification of at least one FR or CDR sequences.

Further described herein are such variant antibodies, preferably monoclonal antibodies, most preferably human antibodies, comprising a heavy chain and a light chain, wherein any of the heavy and light chain, or the VH/VL variable regions, or the respective CDRs comprises an amino acid sequence as derived from a parent antibody, which is one of the antibodies designated #AB-30-3, #AB-31, #AB-32-6, #AB-34, #AB-34-6, #AB-30-8, #AB-32-9, #AB-34-14, or #AB-34-15, by modification of at least one FR or CDR sequences.

Also described herein are such variant antibodies, comprising the respective binding sequences, such as the variable sequences and/or the CDR sequences, as derived from the parent antibodies above, wherein the binding sequences or the CDR comprises a sequence that has at least 60%, preferably at least 70%, or at least 80%, or at least 90%, or at least 95%, or at least 99% identity to the amino acid sequence as derived from the parent antibodies, and wherein the variant is a functionally active variant.

In particular, the functional activity is determined by the specificity and affinity to bind the LukGH heterodimer, optionally the preferential binding of the LukGH heterodimer over the binding of any of the individual LukG or LukH antigens, e.g. by binding the same epitope or substantially the same epitope as the respective parent antibody.

Antibodies are said to "bind to the same epitope" or "comprising the same binding site" or have "essentially the same binding" characteristics, if the antibodies cross-compete so that only one antibody can bind to the epitope at a given point of time, i.e. one antibody prevents the binding or modulating effect of the other.

The term "compete" or "cross-compete", as used herein with regard to an antibody, means that a first antibody, or an antigen-binding portion thereof, binds to an epitope in a manner sufficiently similar to the binding of a second antibody, or an antigen-binding portion thereof, such that the result of binding of the first antibody with its cognate epitope is detectably decreased in the presence of the second antibody compared to the binding of the first antibody in the absence of the second antibody. The alternative, where the binding of the second antibody to its epitope is also detectably decreased in the presence of the first antibody, can, but need not be the case. That is, a first antibody can inhibit the binding of a second antibody to its epitope without that second antibody inhibiting the binding of the first antibody to its respective epitope. However, where each antibody detectably inhibits the binding of the other antibody with its cognate epitope, whether to the same, greater, or lesser extent, the antibodies are said to "cross-compete" with each other for binding of their respective epitope(s). Both competing and cross-competing antibodies are provided herein.

Competition herein means a greater relative inhibition than about 30% as determined by competition ELISA analysis or by ForteBio analysis, e.g. as described in the Examples section. It may be desirable to set a higher threshold of relative inhibition as criteria of what is a suitable level of competition in a particular context, e.g., where the competition analysis is used to select or screen for new antibodies designed with the intended function of the binding of additional or other toxins of *S. aureus.* Thus, for example, it is possible to set criteria for the competitive binding, wherein at least 40% relative inhibition is detected, or at least 50%, at least 60%, at least 70%, at least 80%, at least 90% or even at least 100%, before an antibody is considered sufficiently competitive.

As described herein, in one aspect antibody molecules characterized by, e.g. the ability to compete with any of the antibodies designated #AB-30-3, #AB-31, #AB-32-6, #AB-34, #AB-34-6, #AB-30-8, #AB-32-9, #AB-34-14, or #AB-34-15, for binding to the LukGH complex or heterodimer are provided herein.

Preferred antibodies are binding said individual antigens with a high affinity, in particular with a high on and/or a low off rate, or a high avidity of binding. The binding affinity of an antibody is usually characterized in terms of the concentration of the antibody, at which half of the antigen binding sites are occupied, known as the dissociation constant (Kd, or K_{D}). Usually a binder is considered a high affinity binder with a Kd<10⁻⁸ M, preferably a Kd<10⁻⁹ M, even more preferred is a Kd<10⁻¹⁰ M.

Yet, in a particularly preferred embodiment the individual antigen binding affinities are of medium affinity, e.g. with a Kd of less than 10⁻⁶ and up to 10⁻⁸ M, e.g. when binding to at least two antigens.

Medium affinity binders may be provided herein, preferably in conjunction with an affinity maturation process, if necessary.

Affinity maturation is the process by which antibodies with increased affinity for a target antigen are produced. Any one or more methods of preparing and/or using affinity maturation libraries available in the art may be employed in order to generate affinity matured antibodies in accordance with various embodiments disclosed herein. Exemplary such affinity maturation methods and uses, such as random mutagenesis, bacterial mutator strains passaging, site-directed mutagenesis, mutational hotspots targeting, parsimonious mutagenesis, antibody shuffling, light chain shuffling, heavy chain shuffling, CDR1 and/or CDR1 mutagenesis, and methods of producing and using affinity maturation libraries amenable to implementing methods and uses in accordance with various embodiments disclosed herein, include, for example, those disclosed in: Prassler et al. (2009); Immunotherapy, Vol. 1(4), pp. 571-583; Sheedy et al. (2007), Biotechnol. Adv., Vol. 25(4), pp. 333-352; WO2012/009568; WO2009/036379; WO2010/105256; US2002/0177170; WO2003/074679.

With structural changes of an antibody, including amino acid mutagenesis or as a consequence of somatic mutation in immunoglobulin gene segments, variants of a binding site to an antigen are produced and selected for greater affinities. Affinity matured antibodies may exhibit a several logfold greater affinity than a parent antibody. Single parent antibodies may be subject to affinity maturation. Alternatively pools of antibodies with similar binding affinity to the target antigen may be considered as parent structures that are varied to obtain affinity matured single antibodies or affinity matured pools of such antibodies.

The preferred affinity maturated variant of an antibody described herein exhibits at least a 2 fold increase in affinity of binding, preferably at least a 5, preferably at least 10, preferably at least 50, or preferably at least 100 fold increase. The affinity maturation may be employed in the course of the selection campaigns employing respective libraries of parent molecules, either with antibodies having medium binding affinity to obtain the antibody described herein having the specific target binding property of a binding affinity Kd<10⁻⁸ M. Alternatively, the affinity may be even more increased by affinity maturation of the antibody described herein to obtain the high values corresponding to a Kd of less than 10⁻⁹ M, preferably less than 10⁻¹⁰ M or even less than 10⁻¹¹ M, most preferred in the picomolar range.

In certain embodiments binding affinity is determined by an affinity ELISA assay. In certain embodiments binding affinity is determined by a BIAcore, ForteBio or MSD assays. In certain embodiments binding affinity is determined by a kinetic method. In certain embodiments binding affinity is determined by an equilibrium/solution method.

Phagocytic effector cells may be activated through another route employing activation of complement. Antibodies that bind to surface antigens on microorganisms attract the first component of the complement cascade with their Fc region and initiate activation of the "classical" complement system. These results in the stimulation of phagocytic effector cells, which ultimately kill the target by complement dependent cytotoxicity (CDC).

According to a specific embodiment, the antibody described herein has a cytotoxic activity in the presence of immune-effector cells as measured in a standard ADCC or CDC assay. A cytotoxic activity as determined by either of an ADCC or CDC assay may be shown for an antibody described herein, if there is a significant increase in the percentage of cytolysis as compared to a control. The cytotoxic activity related to ADCC or CDC is preferably measured as the absolute percentage increase, which is preferably higher than 5%, more preferably higher than 10%, even more preferred higher than 20%. Complement fixation might be specifically relevant, this mechanism can eliminate toxins from the infection site or blood by removal of the immune complexes formed.

According to a specific embodiment, the antibody described herein has an immunomodulatory function exerted by the Fc part of IgGs. Altered glycosylation increasing the sialylation content, e.g. on the terminal galactose residues, possibly have an anti-inflammatory effect via DC-SIGN signaling. Preferential binding to Fcgamma receptor IIb (inhibitory) over the la, IIa and III Fcgamma receptors possibly provides an anti-inflammatory effect.

Further described herein are cross-reactive antibodies, which are obtained by a process to identify neutralizing antibodies with multiple specificities, e.g. by a cross-reactive discovery selection scheme. Accordingly, an antibody library including antibodies showing reactivity with two targets, targets A and B, may first be selected for reactivity with one of the targets, e.g. target A, followed by selection for reactivity with the other target, e.g. target B. Each successive selection round reinforces the reactive strength of the resulting pool towards both targets. Accordingly, this method is particularly useful for identifying antibodies with cross-reactivity directed to the two different targets, and the potential to cross-neutralize a pathogen. The method can be extended to identifying antibodies showing reactivity towards further targets, by including additional rounds of enrichment towards the additional target(s).

Cross-reactive antibodies, in some instances, emerge through screening against single antigens. To increase the likelihood of isolating cross-reactivity clones one would apply multiple selective pressures by processively screening against multiple antigens. Special mAb selection strategies employ the different toxin components in an alternating fashion. For example, neutralizing LukGH mAbs are tested for binding to the LukGH heterodimer obtained from different strains.

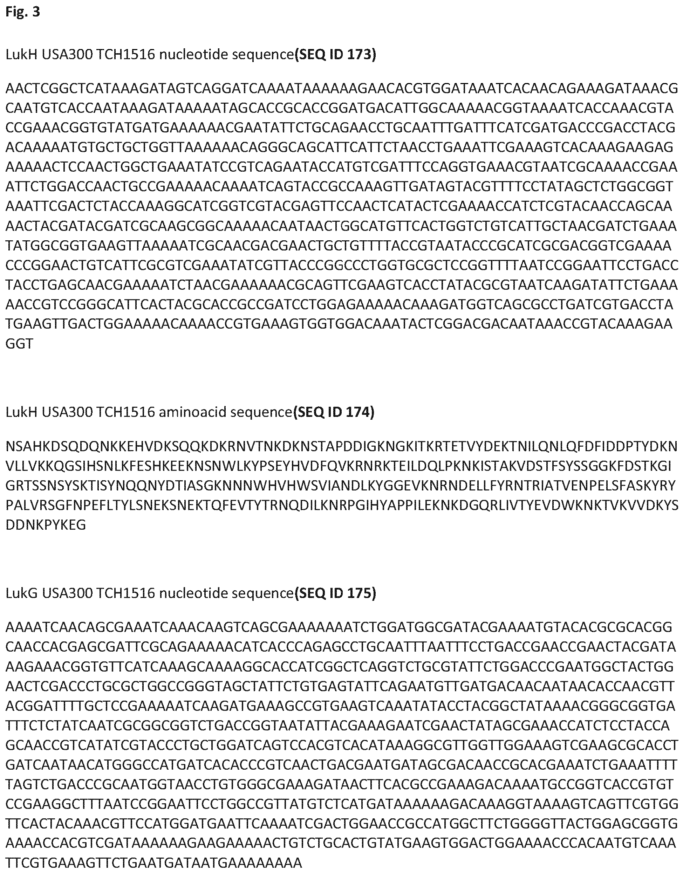

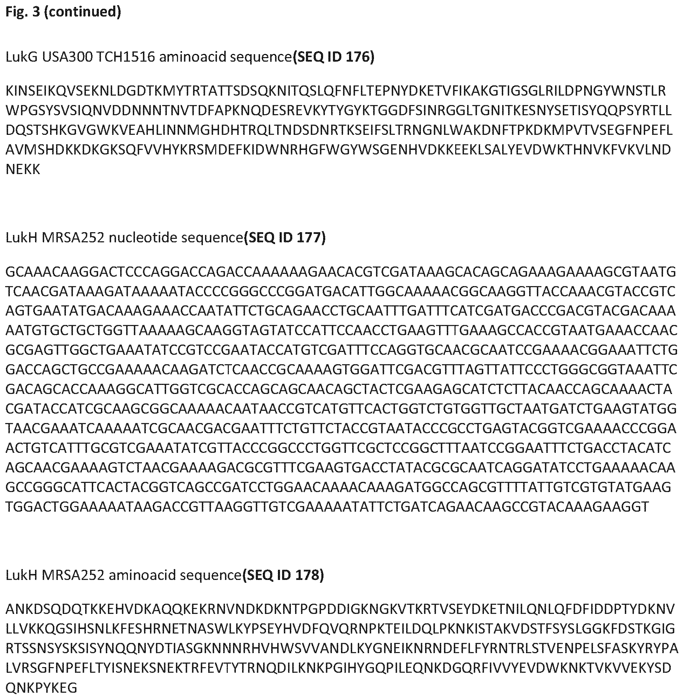

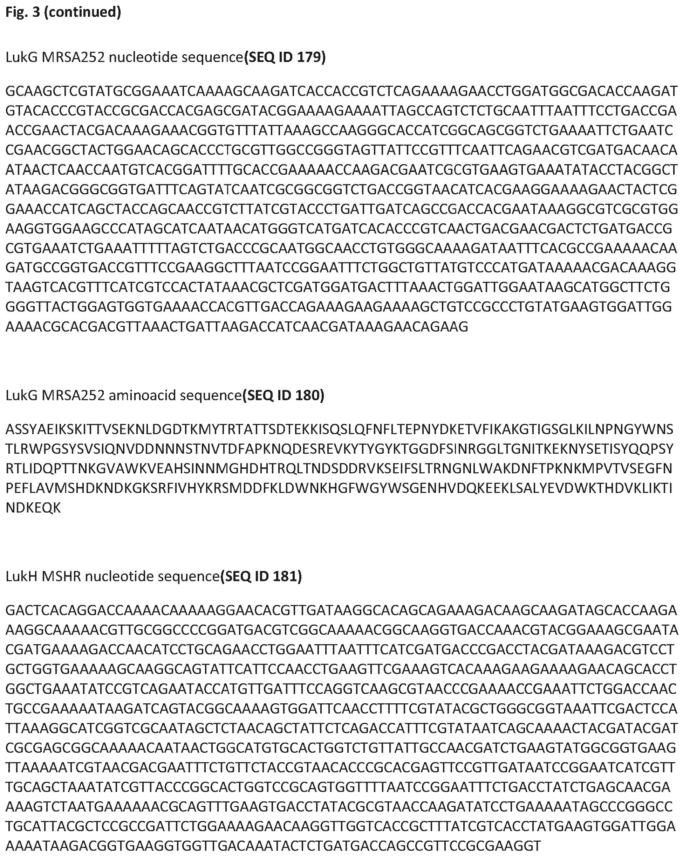

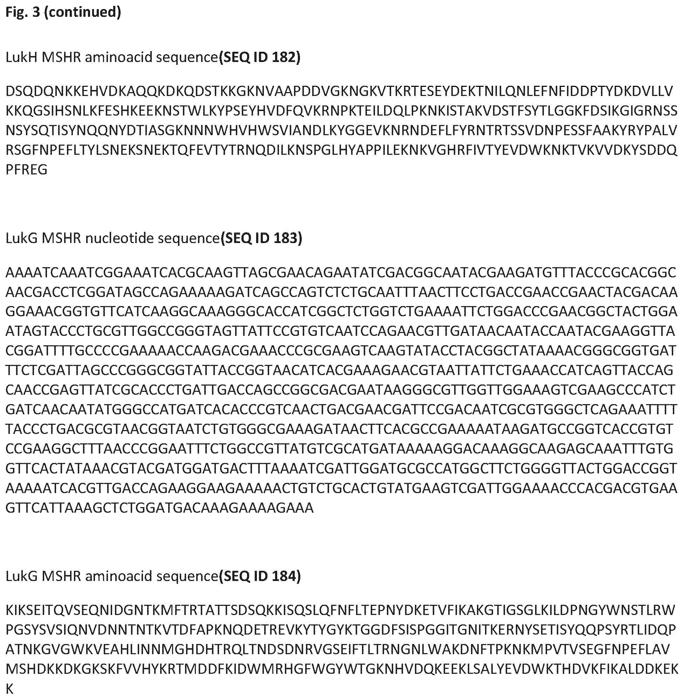

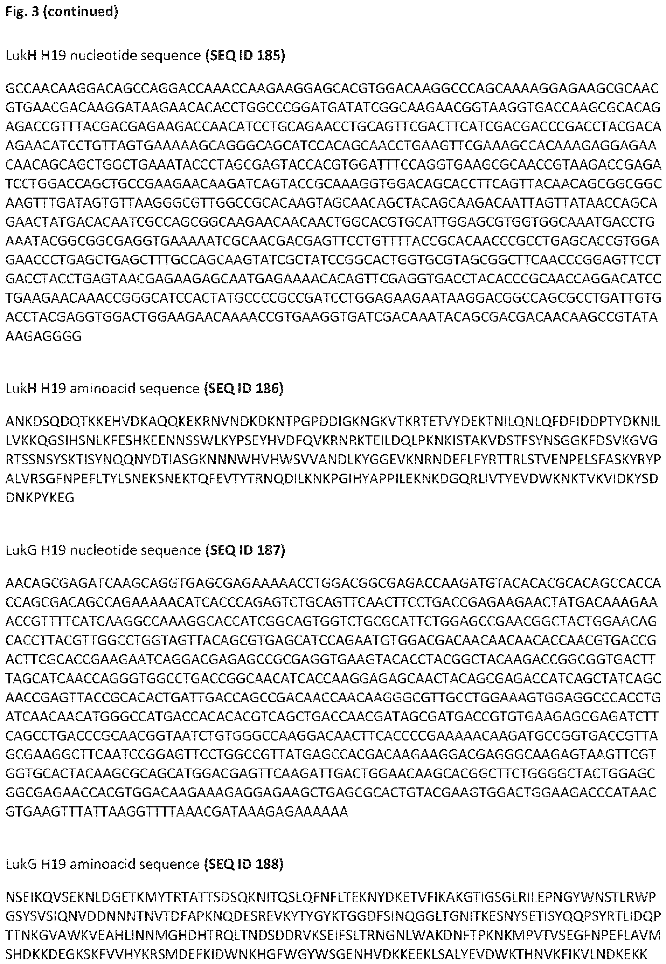

The recombinant toxins produced by recombinant techniques employing the respective sequences of Figure 3, or toxins isolated from *S. aureus* culture supernatants may be used for selecting antibodies from an antibody library, e.g. a yeast-displayed antibody library see, for example: Blaise L, Wehnert A, Steukers MP, van den Beucken T, Hoogenboom HR, Hufton SE. Construction and diversification of yeast cell surface displayed libraries by yeast mating: application to the affinity maturation of Fab antibody fragments. Gene. 2004 Nov 24;342(2):211-8; Boder ET, Wittrup KD. Yeast surface display for screening combinatorial polypeptide libraries. Nat Biotechnol. 1997 Jun;15(6):553-7; Kuroda K, Ueda M. Cell surface engineering of yeast for applications in white biotechnology. Biotechnol Lett. 2011 Jan;33(1):1-9. doi: 10.1007/s10529-010-0403-9. Review; Lauer TM, Agrawal NJ, Chennamsetty N, Egodage K, Helk B, Trout BL. Developability index: a rapid in silico tool for the screening of antibody aggregation propensity. J Pharm Sci. 2012 Jan;101(1):102-15; Orcutt K.D. and Wittrup K.D. (2010), 207-233 doi: 10.1007/978-3-642-01144-3_15; Rakestraw JA, Aird D, Aha PM, Baynes BM, Lipovsek D. Secretion-and-capture cell-surface display for selection of target-binding proteins. Protein Eng Des Sel. 2011 Jun;24(6):525-30; US Patent No. 6,423,538; US Patent No. 6,696,251; US Patent No. 6,699,658; published PCT application publication No. WO2008118476.

In either event, cross-reactivity can be further improved by antibody optimization methods known in the art. For example, certain regions of the variable regions of the immunoglobulin chains described herein may be subjected to one or more optimization strategies, including light chain shuffling, destinational mutagenesis, CDR amalgamation, and directed mutagenesis of selected CDR and/or framework regions.

Screening methods for identifying antibodies with the desired neutralizing properties may be inhibition of toxin binding to the target cells, inhibition of formation of dimers or oligomers, inhibition of pore formation, inhibition of cell lysis, inhibition of the induction of cytokines, lymphokines, and any pro-inflammatory signaling, and/or inhibition of *in vivo* effect on animals (death, hemolysis, overshooting inflammation, organ dysfunction). Reactivity can be assessed based on direct binding to the desired toxins, e.g. using standard assays.

Once neutralizing or cross-neutralizing antibodies with the desired properties have been identified, the dominant epitope or epitopes recognized by the antibodies may be determined. Methods for epitope mapping are well-known in the art and are disclosed, for example, in Epitope Mapping: A Practical Approach, Westwood and Hay, eds., Oxford University Press, 2001.

Epitope mapping concerns the identification of the epitope to which an antibody binds. There are many methods known to those of skill in the art for determining the location of epitopes on proteins, including crystallography analysis of the antibody-antigen complex, competition assays, gene fragment expression assays, and synthetic peptide-based assays. An antibody that "binds the same epitope" as a reference antibody is herein understood in the following way. When two antibodies recognize epitopes that are identical or sterically overlapping epitopes, the antibodies are referred to as binding the same or essentially the same or substantially the same epitopes. A commonly used method for determining whether two antibodies bind to identical or sterically overlapping epitopes is the competition assay, which can be configured in all number of different formats, using either labeled antigen or labeled antibody. Usually, an antigen is immobilized on a 96-well plate, and the ability of unlabeled antibodies to block the binding of labeled antibodies is measured using radioactive or enzyme labels.

Once antibodies with the desired neutralizing or cross-neutralizing properties are identified, such antibodies, including antibody fragments can be produced by methods well-known in the art, including, for example, hybridoma techniques or recombinant DNA technology.

In the hybridoma method, a mouse or other appropriate host animal, such as a hamster, is immunized to elicit lymphocytes that produce or are capable of producing antibodies that will specifically bind to the protein used for immunization. Alternatively, lymphocytes may be immunized *in vitro.* Lymphocytes then are fused with myeloma cells using a suitable fusing agent, such as polyethylene glycol, to form a hybridoma cell.

Culture medium in which hybridoma cells are growing is assayed for production of monoclonal antibodies directed against the antigen. Preferably, the binding specificity of monoclonal antibodies produced by hybridoma cells is determined by immunoprecipitation or by an *in vitro* binding assay, such as radioimmunoassay (RIA) or enzyme-linked immunoabsorbent assay (ELISA).

Recombinant monoclonal antibodies can, for example, be produced by isolating the DNA encoding the required antibody chains and transfecting a recombinant host cell with the coding sequences for expression, using well known recombinant expression vectors, e.g. the plasmids or expression cassette(s) comprising the nucleotide sequences encoding the antibody sequences. Recombinant host cells can be prokaryotic and eukaryotic cells, such as those described above.

According to a specific aspect, the nucleotide sequence may be used for genetic manipulation to humanize the antibody or to improve the affinity, or other characteristics of the antibody. For example, the constant region may be engineered to more nearly resemble human constant regions to avoid immune response, if the antibody is used in clinical trials and treatments in humans. It may be desirable to genetically manipulate the antibody sequence to obtain greater affinity to the target toxins and greater efficacy against *S. aureus.* It will be apparent to one of skill in the art that one or more polynucleotide changes can be made to the antibody and still maintain its binding ability to the target toxins.

The production of antibody molecules, by various means, is generally well understood. US Patent 6331415 (Cabilly et al.), for example, describes a method for the recombinant production of antibodies where the heavy and light chains are expressed simultaneously from a single vector or from two separate vectors in a single cell. Wibbenmeyer et al., (1999, Biochim Biophys Acta 1430(2):191 -202) and Lee and Kwak (2003, J. Biotechnology 101 :189-198) describe the production of monoclonal antibodies from separately produced heavy and light chains, using plasmids expressed in separate cultures of *E. coli.* Various other techniques relevant to the production of antibodies are provided in, e.g., Harlow, et al., ANTIBODIES: A LABORATORY MANUAL, Cold Spring Harbor Laboratory Press, Cold Spring Harbor, N.Y., (1988).

If desired, the antibody of the invention, e.g. any of the antibodies designated #AB-30-3, #AB-31, #AB-32-6, #AB-34, #AB-34-6, #AB-30-8, #AB-32-9, #AB-34-14, or #AB-34-15, may be sequenced and the polynucleotide sequence may then be cloned into a vector for expression or propagation. The sequence encoding the antibody may be maintained in vector in a host cell and the host cell can then be expanded and frozen for future use. Production of recombinant monoclonal antibodies in cell culture can be carried out through cloning of antibody genes from B cells by means known in the art.

In another aspect, the invention provides an isolated nucleic acid comprising a sequence that codes for production of the recombinant antibody of the present invention.

In another aspect, herein described is an isolated nucleic acid comprising a sequence that codes for production of the recombinant epitope described herein, or a molecule comprising such epitope. However, the epitope may also be synthetically produced, e.g. through any of the synthesis methods well-known in the art.

An antibody or epitope encoding nucleic acid can have any suitable characteristics and comprise any suitable features or combinations thereof. Thus, for example, an antibody or epitope encoding nucleic acid may be in the form of DNA, RNA, or a hybrid thereof, and may include non-naturally-occurring bases, a modified backbone, e.g., a phosphorothioate backbone that promotes stability of the nucleic acid, or both. The nucleic acid advantageously may be incorporated in an expression cassette, vector or plasmid as described herein, comprising features that promote desired expression, replication, and/or selection in target host cell(s). Examples of such features include an origin of replication component, a selection gene component, a promoter component, an enhancer element component, a polyadenylation sequence component, a termination component, and the like, numerous suitable examples of which are known.

The present disclosure further provides the recombinant DNA constructs comprising one or more of the nucleotide sequences described herein. These recombinant constructs are used in connection with a vector, such as a plasmid, phagemid, phage or viral vector, into which a DNA molecule encoding any disclosed antibody is inserted.

Monoclonal antibodies are produced using any method that produces antibody molecules by continuous cell lines in culture. Examples of suitable methods for preparing monoclonal antibodies include the hybridoma methods of Kohler et al. (1975, Nature 256:495-497) and the human B-cell hybridoma method (Kozbor, 1984, J. Immunol. 133:3001; and Brodeur et al., 1987, Monoclonal Antibody Production Techniques and Applications, (Marcel Dekker, Inc., New York), pp. 51-63).

Further provided herein are pharmaceutical compositions which comprise an antibody or an immunogen as described herein and a pharmaceutically acceptable carrier or excipient. These pharmaceutical compositions can be administered as a bolus injection or infusion or by continuous infusion. Pharmaceutical carriers suitable for facilitating such means of administration are well known in the art.

Pharmaceutically acceptable carriers generally include any and all suitable solvents, dispersion media, coatings, antibacterial and antifungal agents, isotonic and absorption delaying agents, and the like that are physiologically compatible with an antibody or related composition or combination provided herein. Further examples of pharmaceutically acceptable carriers include sterile water, saline, phosphate buffered saline, dextrose, glycerol, ethanol, and the like, as well as combinations of any thereof.

In one such aspect, an antibody can be combined with one or more carriers appropriate a desired route of administration, antibodies may be, e.g. admixed with any of lactose, sucrose, starch, cellulose esters of alkanoic acids, stearic acid, talc, magnesium stearate, magnesium oxide, sodium and calcium salts of phosphoric and sulphuric acids, acacia, gelatin, sodium alginate, polyvinylpyrrolidine, polyvinyl alcohol, and optionally further tableted or encapsulated for conventional administration. Alternatively, an antibody may be dissolved in saline, water, polyethylene glycol, propylene glycol, carboxymethyl cellulose colloidal solutions, ethanol, corn oil, peanut oil, cottonseed oil, sesame oil, tragacanth gum, and/or various buffers. Other carriers, adjuvants, and modes of administration are well known in the pharmaceutical arts. A carrier may include a controlled release material or time delay material, such as glyceryl monostearate or glyceryl distearate alone or with a wax, or other materials well known in the art.

Additional pharmaceutically acceptable carriers are known in the art and described in, e.g. REMINGTON'S PHARMACEUTICAL SCIENCES. Liquid formulations can be solutions, emulsions or suspensions and can include excipients such as suspending agents, solubilizers, surfactants, preservatives, and chelating agents.

Pharmaceutical compositions are contemplated wherein an antibody or immunogen described herein and one or more therapeutically active agents are formulated. Stable formulations of the antibody or immunogen described herein are prepared for storage by mixing said immunoglobulin having the desired degree of purity with optional pharmaceutically acceptable carriers, excipients or stabilizers, in the form of lyophilized formulations or aqueous solutions. The formulations to be used for *in vivo* administration are specifically sterile, preferably in the form of a sterile aqueous solution. This is readily accomplished by filtration through sterile filtration membranes or other methods. The antibody and other therapeutically active agents disclosed herein may also be formulated as immunoliposomes, and/or entrapped in microcapsules.

Administration of the pharmaceutical composition comprising an antibody or immunogen described herein, may be done in a variety of ways, including orally, subcutaneously, intravenously, intranasally, intraotically, transdermally, mucosal, topically, e.g., gels, salves, lotions, creams, etc., intraperitoneally, intramuscularly, intrapulmonary, e.g. employing inhalable technology or pulmonary delivery systems, vaginally, parenterally, rectally, or intraocularly.

Examplary formulations as used for parenteral administration include those suitable for subcutaneous, intramuscular or intravenous injection as, for example, a sterile solution, emulsion or suspension.

In one embodiment, the antibody or immunogen described herein is the only therapeutically active agent administered to a subject, e.g. as a disease modifying or preventing monotherapy.

In another embodiment, the antibody or immunogen described herein is combined with further antibodies or immunogens in a cocktail, e.g. combined in a mixture or kit of parts, to target *S. aureus,* such that the cocktail contains more than one therapeutically active agents administered to a subject, e.g. as a disease modifying or preventing combination therapy.

Alternatively, the antibody or immunogen described herein is administered in combination with one or more other therapeutic or prophylactic agents, including but not limited to standard treatment, e.g. antibiotics, steroid and non-steroid inhibitors of inflammation, and/or other antibody based therapy, e.g. employing anti-bacterial or anti-inflammatory agents.

A combination therapy is particularly employing a standard regimen, e.g. as used for treating MRSA infection. This may include antibiotics, e.g. tygecycline, linezolide, methicillin and/or vancomycin.

In a combination therapy, the antibody may be administered as a mixture, or concomitantly with one or more other therapeutic regimens, e.g. either before, simultaneously or after concomitant therapy.

Prophylactic administration of immunogens in some cases may employ a vaccine comprising the immunogen described herein, i.e. a monovalent vaccine. Yet, a multivalent vaccine comprising different immunogens to induce an immune response against the same or different target pathogens may be used.

The biological properties of the antibody, the immunogen or the respective pharmaceutical preparations described herein may be characterized *ex vivo* in cell, tissue, and whole organism experiments. As is known in the art, drugs are often tested *in vivo* in animals, including but not limited to mice, rats, rabbits, dogs, cats, pigs, and monkeys, in order to measure a drug's efficacy for treatment against a disease or disease model, or to measure a drug's pharmacokinetics, pharmacodynamics, toxicity, and other properties. The animals may be referred to as disease models. Therapeutics are often tested in mice, including but not limited to nude mice, SCID mice, xenograft mice, and transgenic mice (including knockins and knockouts). Such experimentation may provide meaningful data for determination of the potential of the antibody to be used as a therapeutic or as a prophylactic with the appropriate half-life, effector function, (cross-) neutralizing activity and/or immune response upon active or passive immunotherapy. Any organism, preferably mammals, may be used for testing. For example because of their genetic similarity to humans, primates, monkeys can be suitable therapeutic models, and thus may be used to test the efficacy, toxicity, pharmacokinetics, pharmacodynamics, half-life, or other property of the subject agent or composition. Tests in humans are ultimately required for approval as drugs, and thus of course these experiments are contemplated. Thus, the antibody, immunogen and respective pharmaceutical compositions described herein may be tested in humans to determine their therapeutic or prophylactic efficacy, toxicity, immunogenicity, pharmacokinetics, and/or other clinical properties.

The invention also provides the subject antibody of the invention for diagnostic purposes, e.g. for use in methods of detecting and quantitatively determining the concentration of a toxin or antibody as immunoreagent or target in a biological fluid sample.

Further described herein are methods for detecting the level of toxins or *S. aureus* infection in a biological sample, such as a body fluid, comprising the step of contacting the sample with an antibody described herein. The antibody described herein may be employed in any known assay method, such as competitive binding assays, direct and indirect sandwich assays, immunoprecipitation assays and enzyme-linked immunosorbent assays (ELISA).

A body fluid as used as described herein includes biological samples of a subject, such as tissue extract, urine, blood, serum, stool and phlegm.

In one embodiment the method comprises contacting a solid support with an excess of a certain type of antibody fragment which specifically forms a complex with a target, such as at least one of the toxins targeted by the antibody described herein, conditions permitting the antibody to attach to the surface of the solid support. The resulting solid support to which the antibody is attached is then contacted with a biological fluid sample so that the target in the biological fluid binds to the antibody and forms a target-antibody complex. The complex can be labeled with a detectable marker. Alternatively, either the target or the antibody can be labeled before the formation the complex. For example, a detectable marker (label) can be conjugated to the antibody. The complex then can be detected and quantitatively determined thereby detecting and quantitatively determining the concentration of the target in the biological fluid sample.

For particular applications the antibody described herein is conjugated to a label or reporter molecule, selected from the group consisting of organic molecules, enzyme labels, radioactive labels, colored labels, fluorescent labels, chromogenic labels, luminescent labels, haptens, digoxigenin, biotin, metal complexes, metals, colloidal gold and mixtures thereof. Antibodies conjugated to labels or reporter molecules may be used, for instance, in assay systems or diagnostic methods, e.g. to diagnose *S. aureus* infection or disease conditions associated therewith.

The antibody described herein may be conjugated to other molecules which allow the simple detection of said conjugate in, for instance, binding assays (e.g. ELISA) and binding studies.

Further described herein is a kit comprising an antibody, which may include, in addition to one or more antibodies, various diagnostic or therapeutic agents. A kit may also include instructions for use in a diagnostic or therapeutic method. Such instructions can be, for example, provided on a device included in the kit, e.g. tools or a device to prepare a biological sample for diagnostic purposes, such as separating a cell and/or protein containing fraction before determining the respective toxin(s) to diagnose a disease. Advantageously, such a kit includes an antibody and a diagnostic agent or reagent that can be used in one or more of the various diagnostic methods described herein. In another preferred embodiment, the kit includes an antibody, e.g. in the lyophilized form, in combination with pharmaceutically acceptable carrier(s) that can be mixed before use to form an injectable composition for near term administration.

The antibody designated #AB-29 specifically is characterized by amino acid sequences as indicated in Figure 1, and further characterized by the HC and LC sequences listed in Figure 2, wherein the HC sequence is identified as SEQ ID 147, and the LC sequence is identified as SEQ ID 148. In particular, the VH CDR sequences are identified as CDR1 SEQ ID 2, CDR2 SEQ ID 4, and CDR3 SEQ ID 6, and the VH FR sequences are identified as FR1 SEQ ID 1, FR2 SEQ ID 3, FR3 SEQ ID 5, and FR4 SEQ ID 7. The VL CDR sequences are identified as CDR4 SEQ ID 9, CDR5 SEQ ID 11, and CDR6 SEQ ID 13, and the VL FR sequences are identified as FR1 SEQ ID 8, FR2 SEQ ID 10, FR3 SEQ ID 12, and FR4 SEQ ID 14.

The antibody designated #AB-30 specifically is characterized by amino acid sequences as indicated in Figure 1, and further characterized by the HC and LC sequences listed in Figure 2, wherein the HC sequence is identified as SEQ ID 149, and the LC sequence is identified as SEQ ID 150. In particular, the VH CDR sequences are identified as CDR1 SEQ ID 26, CDR2 SEQ ID 28, and CDR3 SEQ ID 30, and the VH FR sequences are identified as FR1 SEQ ID 25, FR2 SEQ ID 27, FR3 SEQ ID 29, and FR4 SEQ ID 31. The VL CDR sequences are identified as CDR4 SEQ ID 32, CDR5 SEQ ID 33, and CDR6 SEQ ID 35, and the VL FR sequences are identified as FR1 SEQ ID 18, FR2 SEQ ID 20, FR3 SEQ ID 34, and FR4 SEQ ID 14.

The antibody variants designated #AB-30-3, and #AB-30-8, are functionally active variants of #AB-30 and characterized by amino acid sequences as indicated in Figure 1, specifically the respective VH or HC sequences, and further the VL or LC sequences, including the FR and CDR sequences as described in Figure 1, and further characterized by the HC and LC sequences listed in Figure 2.

The antibody designated #AB-31 specifically is characterized by amino acid sequences as indicated in Figure 1, and further characterized by the HC and LC sequences listed in Figure 2, wherein the HC sequence is identified as SEQ ID 155, and the LC sequence is identified as SEQ ID 156. In particular, the VH CDR sequences are identified as CDR1 SEQ ID 47, CDR2 SEQ ID 49, and CDR3 SEQ ID 51, and the VH FR sequences are identified as FR1 SEQ ID 46, FR2 SEQ ID 48, FR3 SEQ ID 50, and FR4 SEQ ID 52. The VL CDR sequences are identified as CDR4 SEQ ID 53, CDR5 SEQ ID 21, and CDR6 SEQ ID 54, and the VL FR sequences are identified as FR1 SEQ ID 18, FR2 SEQ ID 20, FR3 SEQ ID 22, and FR4 SEQ ID 14.

The antibody designated #AB-32 specifically is characterized by amino acid sequences as indicated in Figure 1, and further characterized by the HC and LC sequences listed in Figure 2, wherein the HC sequence is identified as SEQ ID 157, and the LC sequence is identified as SEQ ID 158. In particular, the VH CDR sequences are identified as CDR1 SEQ ID 71, CDR2 SEQ ID 72, and CDR3 SEQ ID 73, and the VH FR sequences are identified as FR1 SEQ ID 1, FR2 SEQ ID 3, FR3 SEQ ID 5, and FR4 SEQ ID 74. The VL CDR sequences are identified as CDR4 SEQ ID 75, CDR5 SEQ ID 41, and CDR6 SEQ ID 76, and the VL FR sequences are identified as FR1 SEQ ID 18, FR2 SEQ ID 20, FR3 SEQ ID 34, and FR4 SEQ ID 14.

The antibody variants designated #AB-32-6, and #AB-32-9, are functionally active variants of #AB-32 and characterized by amino acid sequences as indicated in Figure 1, specifically the respective VH or HC sequences, and further the VL or LC sequences, including the FR and CDR sequences as described in Figure 1, and further characterized by the HC and LC sequences listed in Figure 2.

The antibody designated #AB-33 specifically is characterized by amino acid sequences as indicated in Figure 1, and further characterized by the HC and LC sequences listed in Figure 2, wherein the HC sequence is identified as SEQ ID 163, and the LC sequence is identified as SEQ ID 164. In particular, the VH CDR sequences are identified as CDR1 SEQ ID 87, CDR2 SEQ ID 88, and CDR3 SEQ ID 89, and the VH FR sequences are identified as FR1 SEQ ID 25, FR2 SEQ ID 27, FR3 SEQ ID 29, and FR4 SEQ ID 90. The VL CDR sequences are identified as CDR4 SEQ ID 92, CDR5 SEQ ID 94, and CDR6 SEQ ID 96, and the VL FR sequences are identified as FR1 SEQ ID 91, FR2 SEQ ID 93, FR3 SEQ ID 95, and FR4 SEQ ID 14.

The antibody designated #AB-34 specifically is characterized by amino acid sequences as indicated in Figure 1, and further characterized by the HC and LC sequences listed in Figure 2, wherein the HC sequence is identified as SEQ ID 165, and the LC sequence is identified as SEQ ID 166. In particular, the VH CDR sequences are identified as CDR1 SEQ ID 104, CDR2 SEQ ID 105, and CDR3 SEQ ID 106, and the VH FR sequences are identified as FR1 SEQ ID 125, FR2 SEQ ID 27, FR3 SEQ ID 29, and FR4 SEQ ID 107. The VL CDR sequences are identified as CDR4 SEQ ID 92, CDR5 SEQ ID 94, and CDR6 SEQ ID 108, and the VL FR sequences are identified as FR1 SEQ ID 91, FR2 SEQ ID 93, FR3 SEQ ID 95, and FR4 SEQ ID 14.

The antibody variants designated #AB-34-14, #AB-34-6, and #AB-34-15 are functionally active variants of #AB-34 and characterized by amino acid sequences as indicated in Figure 1, specifically the respective VH or HC sequences, and further the VL or LC sequences, including the FR and CDR sequences as described in Figure 1, and further characterized by the HC and LC sequences listed in Figure 2.

Further variants of any of the antibodies identified in Figure 1 or 2, are contemplated, e.g. CDR variants and/or variants with mutations in any of the CDR or FR sequences, in particular variants based on the sequence of the parent antibody and containing one or more point mutations.

The foregoing description will be more fully understood with reference to the following examples. Such examples are, however, merely representative of methods of practicing one or more embodiments of the present invention and should not be read as limiting the scope of invention.

### EXAMPLES

### Example 1: Generation of LukGH mAbs binding with high affinity

### Generation of recombinant toxins:

Methods: The eight *S. aureus* toxins - LukH_TCH1516, LukH_MRSA252, LukH_MSHR1132, LukH_H19 LukG_TCH1516, LukG_MRSA252, LukG_MSHR1132 and LukG_H19 - were produced recombinantly in *E. coli* Tuner DE3, either individually or as LukGH complexes. Toxin genes for the mature proteins (determined using the SignalP 4.1 Server; http://www.cbs.dtu.dk/services/SignalP/) were codon optimized for *E. coli* expression and generated by gene synthesis based on published genome sequences of *Staphylococcus aureus* strains USA300_TCH1516, MRSA252, MSHR1132 and H19. The individual LukH and LukG proteins (for the USA300_TCH1516, MRSA252 and MSHR1132 variants) were expressed without tags in insoluble form, isolated from inclusion bodies, refolded and purified. The refolded LukH proteins were purified by size exclusion and cation exchange chromatography, while the denatured LukG proteins were purified by cation exchange chromatography, followed by refolding and the refolded proteins were further purified by anion exchange at pH 10.2 - 11.0. The four LukGH complexes (USA300_TCH1516, MRSA252, MSHR1132 and H19 variants) were generated by co-transfection of *E. coli* with two plasmids containing different antibiotic resistance markers, carrying either the LukH or the LukG gene. LukG was expressed as a fusion protein with NusA/His₆ at the N-terminus to allow metal affinity purification of the complex, while LukH was expressed in un-tagged form. The two proteins were found in the soluble fraction and were co-purified by immobilized metal affinity chromatography (IMAC). The NusA/His₆ tag was removed proteolytically with enterokinase giving the un-tagged, mature LukGH complex which was further purified by cation exchange chromatography. The proteins were assayed for purity by SDS-PAGE, monomeric state by size exclusion chromatography, and for functionality in *in vitro* toxin potency assays.

Co-expression stabilized the individual proteins. While the individual components were always expressed in the insoluble fraction of *E. coli,* the co-expressed LukGH was found in the soluble fraction.

SDS-PAGE indicated that the stoichiometry of LukG : LukH in the complex is 1:1. To determine the size of the complex in solution we employed dynamic light scattering (DLS) measurements using a DynoPro NanoStar (Wyatt) instrument equipped with a static light scattering detector. The molecular weight measured with the static light scattering detector (MW-S), is in excellent agreement with the calculated molecular weight of the heterodimer, 73 kDa.

For antibody selection, proteins were labeled with the amino reactive reagent Sulfo-NHS-LC biotin (Thermo-Scientific), according to the manufacturer's instructions, yielding biotin levels of 1 - 2.5 biotin/protein.

### Selection of monoclonal antibodies:

Toxin-specific antibodies were isolated from a full length human IgG1 antibody library (WO2009/036379A2; WO2010105256; WO2012009568) using an *in vitro* yeast selection system and associated methods. Toxin binding mAbs were enriched by incubating biotin labeled LukGH_TCH1516 with antibody expressing yeast cells at different concentrations followed by magnetic bead selection (Miltenyi, Biotec) and fluorescence-activated cell sorting (FACS, FACSAria II, BD Biosciences) employing streptavidin secondary reagents in several successive selection rounds. After the last round of enrichment yeast were sorted and plated onto agar plates and clonal isolates chosen for sequencing and IgG production. Optimization of antibodies for higher affinity were performed in successive cycles of selection rounds using lower concentrations of toxin baits with sub-libraries generated by light chain shuffling and targeted mutagenesis of CDR1 and CDR2 of heavy chains.

Antibodies were then produced by the selected yeast clones and purified by Protein A affinity chromatography.

Binding of mAbs to the different toxins was confirmed by interferometry measurements using a ForteBio Octet Red instrument (Pall Life Sciences). The biotinylated antigen or the antibody was immobilized on the sensor and the association and dissociation of the antibody Fab fragment or of the antigen, respectively (typically 70-270 nM), in solution, were measured. Fab Kd affinities measured by MSD method using a Sector Imager 2400 instrument (Meso Scale Discovery) Typically 20pM of biotinylated antigen was incubated with Fab at various concentations, for 16h at room temperature, and the unbound antigen captured on immobilized IgG. See also for example., Estep et al., "High throughput solution-based measurement of antibody-antigen affinity and epitope binning", MAbs, Vol. 5(2), pp. 270-278 (2013).

### Results:

The LukGH mAbs were discovered in successive rounds of selections with LukGH_TCH1516, as antigen from a library of full length human IgG1 (approx. diversity ∼ 10⁻¹¹) expressed on the surface of yeast. The antibodies from the final optimization round display high affinities towards LukGH_TCH1516 (< 5 pM as measured by Fab MSD Kd), and at least two orders of magnitude lower affinities towards LukG_TCH1516, as determined by MSD Fab Kd and ForteBio measurements, and no binding to LukH_TCH1516 (Figure 4). The antibodies are also cross-reactive between the LukGH variants studied, showing similar affinities to LukGH_TCH1516, LukGH_MRSA252, LukGH_MSHR1132 and LukGH_H19 as determined by ForteBio (Figure 4).

### Example 2: Analysis of human mAbs for their LukGH neutralizing activity.

The neutralizing activity of human mAbs against the USA300 LukGH toxin and the variant toxins of MRSA252 and MSHR1132 was assessed in a viability assay using either human PMNs isolated from human whole blood or differentiated ("neutrophil-like"). HL60 cells (ATCC CCL-240™) were differentiated with DMF (N,N-Dimethylformamide, 100mM) for 3-5 days, as described by Romero-Steiner, Clin Diagn Lab Immunol, 1997:415. Differentiation was determined by disappearance of CD71 and appearance of CD11b staining using Brilliant Violet 421 conjugated anti-CD11b (clone ICRF44, BioLegend, USA) and PE-conjugated anti-CD71 monoclonal antibodies (clone OKT9, eBioscience, USA), according to standard methods described in the literature (e.g. Collado-Escobar, Biochem J, 1994:553; Trayner, Leuk Res, 1998:537; Watanabe, J Leuk Biol, 1993:40). For viability assays, cells were re-suspended in RPMI 1640 (PAA Laboratories, Austria) supplemented with 10% FCS, L-Glutamine and Pen/Strep (=neutrophil medium). Monoclonal antibodies were serially diluted in neutrophil medium and mixed with toxins at a fixed concentration that decreased cell viability > 80% [0.69 - 4.11 nM, depending on cell type and LukGH variant used]. Viability assay was started after a 30 minutes pre-incubation step to allow antibody binding to LukGH. 25,000 cells were added per well, followed by 4 hours incubation at 37°C, in humidified atmosphere with 5% CO2. Cell viability was assessed by measuring cellular ATP levels (CellTiter-Glo® Luminescent Cell Viability Assay; Promega, USA) according to the manufacturer's instructions. % inhibition of toxin activity was calculated using the following formula: % inhibition = [(normal activity- inhibited activity) / (normal activity)] x 100. The control mAb (generated against an irrelevant antigen) was included in all assays.

### Results:

Toxin neutralization activity of LukGH mAbs was measured on neutrophil-like HL60 cells in the 0.03-208 nM mAb concentration range after preincubation with recombinant LukGH_TCH1516 at a fixed concentration (2.74 nM, 200 ng/ml) inducing more than > 80% cell lysis. The selected antibodies were proven to be highly potent in inhibiting LukGH cytotoxicity. The most efficacious mAbs reached a molar ratio of antibody to toxin at the half maximum lysis concentration (IC50) of <1. Examples (AB-31, AB-32-6, AB-32-9, AB-34, AB-34-14, AB-34-6 and AB-34-15 are shown in Fig. 5A).

To test for cross-reactivity to other LukGH sequence variants, selected antibodies were also tested for neutralization of the LukGH variants of strain MRSA252 (examples AB-29-2, AB-30-3, AB-31, AB-32-6, AB-33, AB-34, AB-34-15 are shown in Fig. 5B) and MSHR1132 (examples AB-29-2, AB-30-3, AB-31, AB-32-6, AB-33, AB-34, AB-34-15 in Fig. 5C.). As seen for LukGH_TCH1516, the most efficacious mAbs were highly potent in neutralizing the MSH1132 and MRSA252 sequence variants.

### Example 6. Epitope mapping/binding of antibodies using the crystal structure of LukGH:AB-32-9 complex

The epitope residues of the AB-32-9 antibody in the LukGH molecule were identified from the crystal structure of LukGH in complex with the Fab fragment of AB-32-9. The epitope is defined as the toxin residues at the Fab-toxin interface which are in contact with the specific binder, e.g. the Fab residue, i.e. the distance between any of their non-hydrogen atoms is smaller or equal to 4.0 Å, as measured in Pymol (PyMOL Molecular Graphics System, Version 1.5.0.4 Schrödinger, LLC).

The LukGH epitope depicted in Figure 6, is found in the rim domain of LukG (sphere representation), and is binding to residues from both the light chain (black cartoon) and heavy chain (grey, cartoon) of the variable domain of the AB-32-9 Fab. The LukG contact residues (Figure 6, black spheres) determined from the crystal structure are aminoacids: Asn71, Tyr73, Trp74, Asn206, Leu207, Trp208, Lys210, Asp211, Trp262, Phe267. All these amino acids are fully conserved between the LukGH variants.

### Example 7. In silico analysis of variant amino acids using the crystal structure of the LukGH: Fab AB-32-9 complex

The crystal structures of the AB-32-9 Fab fragment in complex with LukGH was used to calculate binding energies for all contact positions as well as for *in silico* mutagenesis of the Fab contact residues to predict variants with improved binding towards LukGH.

The structure was prepared using YASARA (Krieger E, Koraimann G, Vriend G. Increasing the precision of comparative models with YASARA NOVA--a self-parameterizing force field. Proteins. 2002;47(3):393-402), the ions and water molecules were stripped, the hydrogens were added and optimized, using steepest descent minimization. The contributions of each residue to the overall binding energy were calculated using Rosetta score 12 function (Kaufmann KW, Lemmon GH, Deluca SL, Sheehan JH, Meiler J. Practically useful:what the Rosetta protein modeling suite can do for you. Biochemistry. 2010 Apr 13;49(14):2987-98) without further optimization. Selected contact positions were mutated to all amino acids except Cys and Pro and the binding energy changes for each mutation are given in Table 2 below.

The *in silico* mutagenesis indicated that changing several AB-32-9 contact residues could lead to improved binding to LukGH, i.e. N7F, N7Y, S8I, Y9R in VL CDR4, A1G in VL CDR2, S1E, S1F, S1H, S1I, S1K, S1L, S1M, S1R, S1V, S1W, S1Y in VH CDR1, S5R, S5W, S7D, S7M, S7N, S7R, Y9D, Y9H in VH CDR2, R4D, R4H, M6F, M6H, H7E, H7K, H7Q, H7R in VH CDR3. There are also a relatively high number of mutations that are not affecting binding to LukGH (ΔΔG values <0.5), so these variants are expected to show similar binding profiles as AB-32-9 (Table 2).

## Claims

1. An antibody comprising at least one binding site that specifically binds to a LukGH heterodimer, which antibody comprises at least an antibody heavy chain variable region (VH), and an antibody light chain variable region (VL), having the heavy chain (HC) and light chain (LC) sequences of an antibody, which is selected from the group consisting of
a) an antibody comprising
a. the HC amino acid sequence of SEQ ID 161; and
b. the LC amino acid sequence of SEQ ID 162;
b) an antibody comprising
a. the HC amino acid sequence of SEQ ID 147; and
b. the LC amino acid sequence of SEQ ID 148;
c) an antibody comprising
a. the HC amino acid sequence of SEQ ID 149; and
b. the LC amino acid sequence of SEQ ID 150;
d) an antibody comprising
a. the HC amino acid sequence of SEQ ID 151; and
b. the LC amino acid sequence of SEQ ID 152;
e) an antibody comprising
a. the HC amino acid sequence of SEQ ID 153; and
b. the LC amino acid sequence of SEQ ID 154;
f) an antibody comprising
a. the HC amino acid sequence of SEQ ID 155; and
b. the LC amino acid sequence of SEQ ID 156;
g) an antibody comprising
a. the HC amino acid sequence of SEQ ID 157; and
b. the LC amino acid sequence of SEQ ID 158;
h) an antibody comprising
a. the HC amino acid sequence of SEQ ID 159; and
b. the LC amino acid sequence of SEQ ID 160;
i) an antibody comprising
a. the HC amino acid sequence of SEQ ID 163; and
b. the LC amino acid sequence of SEQ ID 164;
j) an antibody comprising
a. the HC amino acid sequence of SEQ ID 165; and
b. the LC amino acid sequence of SEQ ID 166;
k) an antibody comprising
a. the HC amino acid sequence of SEQ ID 167; and
b. the LC amino acid sequence of SEQ ID 168;
l) an antibody comprising
a. the HC amino acid sequence of SEQ ID 169; and
b. the LC amino acid sequence of SEQ ID 170;
and
m) an antibody comprising
a. the HC amino acid sequence of SEQ ID 171; and
b. the LC amino acid sequence of SEQ ID 172.

2. The antibody of claim 1, which is a full-length monoclonal antibody, an antibody fragment thereof comprising the binding site, or a fusion protein comprising the binding site.

3. The antibody of claim 1 or 2, for use in treating a subject at risk of or suffering from a S. *aureus* infection comprising administering to the subject an effective amount of the antibody to limit the infection in the subject, to ameliorate a disease condition resulting from said infection or to inhibit *S. aureus* disease pathogenesis, such as pneumonia, sepsis, bacteremia, wound infection, abscesses, surgical site infection, endophthalmitis, furunculosis, carbunculosis, endocarditis, peritonitis, osteomyelitis or joint infection.

4. A pharmaceutical preparation comprising the antibody of claim 1 or 2, comprising a parenteral or mucosal formulation containing a pharmaceutically acceptable carrier or excipient.

5. Use of the antibody of claim 1 or 2, in an in vitro diagnostic method to detect any *S. aureus* infections, including high toxin producing MRSA infections, such as necrotizing pneumonia, and toxin production in furunculosis and carbunculosis.

6. A diagnostic preparation comprising the antibody of claim 1 or 2, containing the antibody with a label and/or a further diagnostic reagent with a label.

7. Isolated nucleic acid molecules encoding an antibody of claim 1 or 2.

## Patentansprüche

1. Antikörper, umfassend mindestens eine Bindungsstelle, die spezifisch an ein LukGH-Heterodimer bindet, wobei der Antikörper mindestens eine variable Region der schweren Kette (VH) des Antikörpers, und eine variable Region der leichten Kette (VL) des Antikörpers umfasst, enthaltend die schwere Kette (HC) und leichte Kette (LC) Sequenzen eines Antikörpers, welcher ausgewählt ist aus der Gruppe bestehend aus
a) einem Antikörper, umfassend
a. die HC-Aminosäuresequenz von SEQ ID 161; und
b. die LC-Aminosäuresequenz von SEQ ID 162;
b) einem Antikörper, umfassend
a. die HC-Aminosäuresequenz von SEQ ID 147; und
b. die LC-Aminosäuresequenz von SEQ ID 148;
c) einem Antikörper, umfassend
a. die HC-Aminosäuresequenz von SEQ ID 149; und
b. die LC-Aminosäuresequenz von SEQ ID 150;
d) einem Antikörper, umfassend
a. die HC-Aminosäuresequenz von SEQ ID 151; und
b. die LC-Aminosäuresequenz von SEQ ID 152;
e) einem Antikörper, umfassend
a. die HC-Aminosäuresequenz von SEQ ID 153; und
b. die LC-Aminosäuresequenz von SEQ ID 154;
f) einem Antikörper, umfassend
a. die HC-Aminosäuresequenz von SEQ ID 155; und
b. die LC-Aminosäuresequenz von SEQ ID 156;
g) einem Antikörper, umfassend
a. die HC-Aminosäuresequenz von SEQ ID 157; und
b. die LC-Aminosäuresequenz von SEQ ID 158;
h) einem Antikörper, umfassend
a. die HC-Aminosäuresequenz von SEQ ID 159; und
b. die LC-Aminosäuresequenz von SEQ ID 160;
i) einem Antikörper, umfassend
a. die HC-Aminosäuresequenz von SEQ ID 163; und
b. die LC-Aminosäuresequenz von SEQ ID 164;
j) einem Antikörper, umfassend
a. die HC-Aminosäuresequenz von SEQ ID 165; und
b. die LC-Aminosäuresequenz von SEQ ID 166;
k) einem Antikörper, umfassend
a. die HC-Aminosäuresequenz von SEQ ID 167; und
b. die LC-Aminosäuresequenz von SEQ ID 168;
l) einem Antikörper, umfassend
a. die HC-Aminosäuresequenz von SEQ ID 169; und
b. die LC-Aminosäuresequenz von SEQ ID 170;
und
m) einem Antikörper, umfassend
a. die HC-Aminosäuresequenz von SEQ ID 171; und
b. die LC-Aminosäuresequenz von SEQ ID 172.

2. Antikörper nach Anspruch 1, welcher ein monoklonaler Antikörper mit voller Länge, ein Antikörperfragment davon, welches die Bindungsstelle umfasst, oder ein Fusionsprotein ist, welches die Bindungsstelle umfasst.

3. Antikörper nach Anspruch 1 oder 2 zur Verwendung bei der Behandlung eines Subjekts, bei dem das Risiko einer S.-*aureus*-Infektion besteht oder das an dieser leidet, umfassend die Verabreichung einer wirksamen Menge des Antikörpers an das Subjekt, um die Infektion bei dem Subjekt zu begrenzen und einen aus der Infektion resultierenden Krankheitszustand zu verbessern oder um die Entstehung einer *S-aureus*-Krankheit wie zum Beispiel Pneumonie, Sepsis, Bakteriämie, Wundinfektion, Abszesse, postoperative Infektion, Endophthalmitis, Furunkulose, Karbunkulose, Endokarditis, Peritonitis, Osteomyelitis oder Gelenkinfektion zu verhindern.

4. Pharmazeutisches Präparat, umfassend den Antikörper nach Anspruch 1 oder 2, der eine parenterale oder mukosale Formulierung umfasst, die einen pharmazeutisch annehmbaren Träger oder Hilfsstoff enthält.

5. Verwendung des Antikörpers nach Anspruch 1 oder 2 in einem In-vitro-Diagnoseverfahren zum Nachweis beliebiger S.-*aureus*-Infektionen, einschließlich stark toxinproduzierenden MRSA-Infektionen wie zum Beispiel nekrotisierender Pneumonie und Toxinproduktion bei Furunkulose und Karbunkulose.

6. Diagnostisches Präparat enthaltend den Antikörper nach Anspruch 1 oder 2, welche den Antikörper mit einer Markierung und/oder ein weiteres diagnostisches Reagens mit einer Markierung enthält.

7. Isolierte Nukleinsäuremoleküle, die einen Antikörper nach Anspruch 1 oder 2 codieren.

## Revendications

1. Anticorps comprenant au moins un site de liaison qui se lie spécifiquement à un hétérodimère LukGH, lequel anticorps comprend au moins une région variable de chaîne lourde d'anticorps (VH), et une région variable de chaîne légère d'anticorps (VL), ayant des séquences d'un chaîne lourde (HC) et d'un chaîne légère (LC) d'un anticorps, qui est choisi dans le groupe constitué par
a) un anticorps comprenant
a. la séquence d'acides aminés de HC de SEQ ID 161 ; et
b. la séquence d'acides aminés de LC de SEQ ID 162 ;
b) un anticorps comprenant
a. la séquence d'acides aminés de HC de SEQ ID 147 ; et
b. la séquence d'acides aminés de LC de SEQ ID 148 ;
c) un anticorps comprenant
a. la séquence d'acides aminés de HC de SEQ ID 149 ; et
b. la séquence d'acides aminés de LC de SEQ ID 150 ;
d) un anticorps comprenant
a. la séquence d'acides aminés de HC de SEQ ID 151 ; et
b. la séquence d'acides aminés de LC de SEQ ID 152 ;
e) un anticorps comprenant
a. la séquence d'acides aminés de HC de SEQ ID 153 ; et
b. la séquence d'acides aminés de LC de SEQ ID 154 ;
f) un anticorps comprenant
a. la séquence d'acides aminés de HC de SEQ ID 155 ; et
b. la séquence d'acides aminés de LC de SEQ ID 156 ;
g) un anticorps comprenant
a. la séquence d'acides aminés de HC de SEQ ID 157 ; et
b. la séquence d'acides aminés de LC de SEQ ID 158 ;
h) un anticorps comprenant
a. la séquence d'acides aminés de HC de SEQ ID 159 ; et
b. la séquence d'acides aminés de LC de SEQ ID 160 ;
i) un anticorps comprenant
a. la séquence d'acides aminés de HC de SEQ ID 163 ; et
b. la séquence d'acides aminés de LC de SEQ ID 164 ;
j) un anticorps comprenant
a. la séquence d'acides aminés de HC de SEQ ID 165 ; et
b. la séquence d'acides aminés de LC de SEQ ID 166 ;
k) un anticorps comprenant
a. la séquence d'acides aminés de HC de SEQ ID 167 ; et
b. la séquence d'acides aminés de LC de SEQ ID 168 ;
l) un anticorps comprenant
a. la séquence d'acides aminés de HC de SEQ ID 169 ; et
b. la séquence d'acides aminés de LC de SEQ ID 170 ;
et
m) un anticorps comprenant
a. la séquence d'acides aminés de HC de SEQ ID 171 ; et
b. la séquence d'acides aminés de LC de SEQ ID 172.

2. Anticorps selon la revendication 1, qui est un anticorps monoclonal pleine longueur, un fragment d'anticorps de celui-ci comprenant le site de liaison, ou une protéine de fusion comprenant le site de liaison.

3. Anticorps selon la revendication 1 ou 2, pour utilisation dans le traitement d'un sujet risquant de souffrir ou souffrant d'une infection à *S. aureus* comprenant l'administration au sujet d'une quantité efficace de l'anticorps pour limiter l'infection chez le sujet, pour améliorer un état pathologique résultant de ladite infection ou pour inhiber la pathogenèse d'une maladie à *S. aureus,* telle qu'une pneumonie, une septicémie, une bactériémie, une infection de plaie, des abcès, une infection de site opératoire, un endophtalmie, une furonculose, une carbunculose, une endocardite, une péritonite, une ostéomyélite ou une infection articulaire.

4. Préparation pharmaceutique comprenant l'anticorps selon la revendication 1 ou 2, comprenant une formulation parentérale ou mucosale contenant un vecteur ou excipient pharmaceutiquement acceptable.

5. Utilisation de l'anticorps selon la revendication 1 ou 2 dans une méthode de diagnostic in vitro pour détecter toutes les infections à S. aureus, dont les infections à SARM produisant des taux élevés de toxines, telle qu'une pneumonie nécrosante, et la production de toxines dans une furonculose ou une carbunculose.

6. Préparation de diagnostic comprenant l'anticorps selon la revendication 1 ou 2, contenant l'anticorps avec un marqueur et/ou un réactif de diagnostic supplémentaire avec un marqueur.

7. Molécules d'acide nucléique isolées codant un anticorps selon la revendication 1 ou 2.
